# EUROPÄISCHE PATENTSCHRIFT

(11) **EP 0 470 600 B1**
(45) Veröffentlichungstag und Bekanntmachung des Hinweises auf die Patenterteilung: **07.05.1997**
(21) Anmeldenummer: 91113282.7
(22) Anmeldetag: 07.08.1991
(51) Int. Cl.: C07D 239/42, C07D 239/46, C07D 239/48, A01N 43/54

(54) **Naphthylalkylaminopyrimidin-Derivate, Verfahren zu ihrer Herstellung und diese enthaltende Schädlingsbekämpfungsmittel**
Naphthylalkylaminopyrimidine derivatives, process for their preparation and pesticides containing them
Dérivés de naphthylalkylaminopyrimidine, procédé pour leur préparation et pesticides les contenant

(30) Priorität: 10.08.1990 CH 2603/90; 08.02.1991 CH 390/91
(43) Veröffentlichungstag der Anmeldung: 12.02.1992
(73) Patentinhaber: Novartis AG, 4058 Basel (CH)
(72) Erfinder: Kristiansen, Odd, Dr., CH-4313 Möhlin (CH); Zondler, Helmut, Dr., CH-4103 Bottmingen (CH); Müller, Urs, Dr., CH-4142 Münchenstein (CH)
(74) Vertreter: Zumstein, Fritz, Dr.

(56) Entgegenhaltungen:
- EP-A- 0 264 217
- EP-A- 0 326 329

## Beschreibung

Die vorliegende Erfindung betrifft Verbindungen der Formel I, Verfahren zu ihrer Herstellung und ihre Verwendung zur Schädlingsbekämpfung sowie Schädlingsbekämpfungsmittel, die diese Verbindungen neben einem geeigneten Trägermaterial als Wirkstoffe enthalten.

In den Verbindungen der Formel I 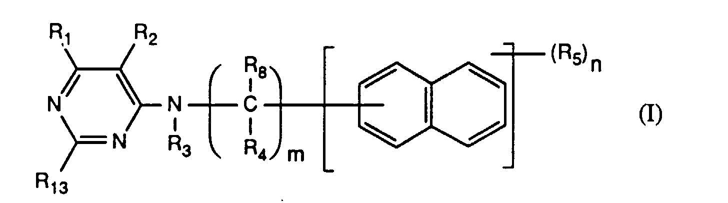 bedeuten:
R₁ Wasserstoff, C₁-C₅ Alkyl, das unsubstituiert oder durch Halogen oder C₁-C₃ Alkoxy oder S(O)ₚ-C₁-C₃-Alkyl substituiert ist; C₂-C₇ Alkenyl, das unsubstituiert oder durch Halogen substituiert ist; C₃-C₇ Cycloalkyl; Halogen; oder C₂-C₄-Alkinyl;
R₂ Wasserstoff, Hydroxy, C₁-C₅ Alkyl, das unsubstituiert oder durch Halogen oder C₁-C₃ Alkoxy substiutiert ist, C₁-C₄-Alkoxy, S(O)ₚ-C₁-C₄-Alkyl, Halogen, Nitro, Cyano, Amino, NHR₃, N(R₃)R₉ oder N=C(R₉)R₁₀;
R₃ Wasserstoff; C₁-C₅ Alkyl; Benzyl; -CO-R₆ oder -S-R₇;
R₄ und R₈ unabhängig voneinander Wasserstoff; C₁-C₅ Alkyl, das unsubstituiert oder durch Halogen oder C₁-C₃ Alkoxy substituiert ist; oder C₃-C₇ Cycloalkyl;
R₅ Halogen, C₁-C₃ Alkyl, C₁-C₃ Alkoxy, C₂-C₆-Alkoxyalkyl, C₁-C₃-Halogenalkyl, C₁-C₃Alkylthio, C₁-C₃-Halogenalkoxy, C₁-C₃-Halogenalkylthio, CN, NO₂ oder eine einmal am Naphthylring auftretende -X- Phenylgruppe, die unsubstituiert oder ein- bis dreifach gleich oder verschieden durch Halogen, C₁-C₃ Alkyl oder C₁-C₃ Alkoxy substituiert ist;
R₆ C₁-C₅ Alkyl; Phenyl, das unsubstituiert oder durch Halogen und/oder C₁-C₃ Alkyl substituiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
R₉ C₁-C₅-Alkyl,
R₁₀ Wasserstoff oder C₁-C₅-Alkyl,
R₁₃ Wasserstoff; C₁-C₄ Alkyl, das unsubstituiert oder durch Halogen oder C₁-C₃ Alkoxy substituiert ist; Cyclopropyl; Halogen; C₁-C₃ Alkoxy; C₁-C₃ Alkylthio oder -N(C₁-C₃ Alkyl)₂;
X Sauerstoff oder Schwefel;
m 1, 2 oder 3;
n 0, 1, 2 oder 3 und
p 0, 1 oder 2.

Bevorzugt sind Verbindungen der Formel I, worin bedeuten:
1.1
   R₁ Wasserstoff, C₁-C₅ Alkyl; oder durch S(O)ₚ-C₁-C₃-Alkyl substituiertes C₁-C₅-Alkyl; C₁-C₅ Haloalkyl mit 1 bis 5 Halogenatomen; C₂-C₆ Alkoxyalkyl; C₂-C₇ Alkenyl; C₂-C₇ Haloalkenyl mit 1 bis 2 Halogenatomen; C₃-C₆ Cycloalkyl; Halogen oder C₂-C₄-Alkinyl;
   R₂ Wasserstoff, C₁-C₅ Alkyl; C₁-C₅ Haloalkyl mit 1 bis 5 Halogenatomen; C₂-C₆ Alkoxyalkyl; Nitro, Cyano, S(O)ₚ-C₁-C₃-Alkyl, Amino, NHR₃, N(R₃)R₉, N=C(R₉)R₁₀ oder Halogen;
   R₃ Wasserstoff; C₁-C₅ Alkyl; Benzyl; -CO-R₆ oder -S-R₇;
   R₄ und R₈ unabhängig voneinander Wasserstoff; C₁-C₅ Alkyl; C₁-C₅ Haloalkyl mit 1 bis 3 Halogenatomen; C₂-C₆ Alkoxyalkyl oder C₃-C₆ Cycloalkyl;
   R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, CN, NO₂ oder eine am Naphthylring auftretende X-Phenylgruppe, die unsubstituiert oder eine - bis dreifach gleich oder verschieden durch Halogen, C₁-C₃ Alkyl oder C₁-C₃ Alkoxy substituiert ist;
   R₆ C₁-C₅ Alkyl oder Phenyl, das unsubstituiert oder durch Halogen und/oder C₁-C₃ Alkyl substiutiert ist;
   R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
   X Sauerstoff oder Schwefel
   R₉ C₁-C₅-Alkyl,
   R₁₀ Wasserstoff oder C₁-C₅-Alkyl,
   m 1, 2 oder 3,
   n 0, 1, 2 oder 3, und
   p 0, 1 oder 2.
1.2
   R₁ Wasserstoff, oder durch S(O)ₚ-C₁-C₃-Alkyl substituiertes C₁-C₅ Alkyl; C₁-C₅ Alkyl; C₁-C₃ Haloalkyl mit 1 bis 3 Halogenatomen; C₂-C₅ Alkoxyalkyl; C₂-C₅ Alkenyl; C₂-C₅ Haloalkenyl mit 1 bis 3 Halogenatomen; C₃-C₅ Cycloalkyl; Halogen oder C₂-C₄ Alkinyl;
   R₂ Wasserstoff, C₁-C₅ Alkyl; C₁-C₃ Haloalkyl mit 1 bis 3 Halogenatomen; C₂-C₅ Alkoxyalkyl; Nitro, Cyano, S(O)ₚ-C₁-C₃-Alkyl, Amino, NHR₃, N(R₃)R₉, N=C(R₉)R₁₀ oder Halogen;
   R₃ Wasserstoff; C₁-C₅ Alkyl; Benzyl; -CO-R₆ oder -S-R₇;
   R₄ und R₈ unabhängig voneinander Wasserstoff; C₁-C₅ Alkyl; C₁-C₅ Haloalkyl mit 1 bis 3 Halogenatomen; C₂-C₅ Alkoxyalkyl oder C₃-C₆ Cycloalkyl;
   R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, CN, NO₂ oder eine am Naphthylring auftretende X-Phenylgruppe, die unsubstituiert oder eine - bis dreifach gleich oder verschieden durch Halogen, C₁-C₃ Alkyl oder C₁-C₃ Alkoxy substituiert ist;
   R₆ C₁-C₅ Alkyl oder Phenyl, das unsubstituiert oder durch Halogen und/oder C₁-C₃ substiutiert ist;
   R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
   X Sauerstoff oder Schwefel
   R₉ C₁-C₅-Alkyl,
   R₁₀ Wasserstoff oder C₁-C₅-Alkyl,
   m 1, 2 oder 3;
   n 0, 1, 2 oder 3; und
   p 0, 1 oder 2.
1.3 Ferner Verbindungen der Formel I' 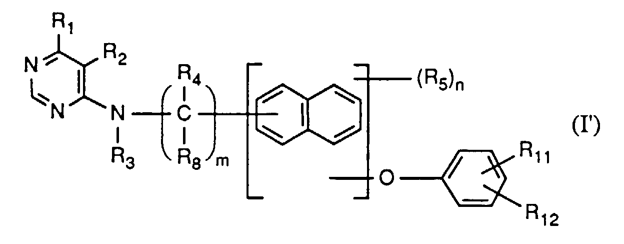 worin bedeuten:
   R₁ Wasserstoff, C₁-C₅ Alkyl, oder durch S(O)ₚ-C₁-C₃-Alkyl substituiertes C₁-C₅-Alkyl; C₁-C₃ Haloalkyl mit 1 bis 3 Halogenatomen; C₂-C₅ Alkoxyalkyl; C₂-C₅ Alkenyl; C₂-C₅ Haloalkenyl mit 1 bis 3 Halogenatomen; C₃-C₆ Cycloalkyl; Halogen oder C₂-C₄ Alkinyl;
   R₂ Wasserstoff, C₁-C₅ Alkyl; C₁-C₃ Haloalkyl mit 1 bis 3 Halogenatomen; C₃-C₅ Alkoxyalkyl; Nitro, Cyano, S(O)ₚ-C₁-C₃-Alkyl, Amino, NHR₃, N(R₃)R₉, N=C(R₉)R₁₀ oder Halogen;
   R₃ Wasserstoff; C₁-C₅ Alkyl; Benzyl; -CO-R₆ oder -S-R₇;
   R₄ und R₈ unabhängig voneinander Wasserstoff; C₁-C₅ Alkyl; C₁-C₅ Haloalkyl mit 1 bis 3 Halogenatomen; C₂-C₆ Alkoxyalkyl oder C₃-C₆ Cycloalkyl;
   R₅ Halogen; C₁-C₃ Alkyl; C₁-C₃ Alkoxy; C₁-C₃ Halogenalkyl, C₁-C₃ Halogenalkoxy,
   R₆ C₁-C₅ Alkyl; Phenyl, das unsubstituiert oder durch Halogen und/oder C₁-C₃ Alkyl substiutiert ist;
   R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
   R₉ C₁-C₅-Alkyl,
   R₁₀ Wasserstoff oder C₁-C₅-Alkyl,
   R₁₁ und R₁₂ unabhängig voneinander Wasserstoff, Halogen oder C₁-C₃-Alkyl,
   m 1, 2 oder 3;
   n 0, 1, 2; und
   p 0, 1 oder 2.
1.4 Ferner Verbindungen der Formel I'
   worin
   R₁ bis R₄ und R₈ die unter Formel I angegebenen Bedeutungen haben,
   R₅ Halogen, C₁-C₃ Alkyl, CF₃, C₂-C₅ Alkoxyalkyl oder C₁-C₃Alkoxy;
   R₁₁ und R₁₂ unabhängig voneinander Wasserstoff, Halogen oder C₁-C₃ Alkyl bedeuten;
   m 1, 2 oder 3; und
   n 0, 1 oder 2 darstellen.
1.5 Verbindungen der Formel I, worin bedeuten:
   R₁ Wasserstoff, C₁-C₅ Alkyl; C₁-C₅ Haloalkyl mit 1 bis 3 Halogenatomen; C₂-C₅ Alkoxyalkyl; C₂-C₅ Alkenyl; C₂-C₅ Halogenalkenyl oder Halogen;
   R₂ Wasserstoff, C₁-C₅ Alkyl; C₁-C₃ Halogenalkyl mit 1 bis 3 Halogenatomen; C₃-C₅ Alkoxyalkyl; Nitro, Cyano, S(O)ₚ-C₁-C₄-Alkyl, Amino, NHR₃, N(R₃)R₉, N=C(R₉)R₁₀ oder Halogen;
   R₃ Wasserstoff, C₁-C₃-Alkyl, Benzyl, -CO-R₆ oder SR₇;
   R₄ und R₈ unabhängig voneinander Wasserstoff; C₁-C₅ Alkyl; C₁-C₅ Halogenalkyl mit 1 bis 3 Halogenatomen; C₂-C₆ Alkoxyalkyl oder C₃-C₆ Cycloalkyl;
   R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, Cyano oder Nitro;
   R₆ C₁-C₅ Alkyl, Phenyl, durch Halogen und/oder C₁-C₃ Alkyl substiutiertes Phenyl;
   R₇ Phenyl, durch Halogen, Nitro oder Cyano ein- oder zweifach substituiertes Phenyl, Benzyl, durch Halogen, Nitro oder Cyano ein- oder zweifach im Ring substituiertes Benzyl, oder C₁-C₅ Alkyl oder durch Halogen oder Cyano substituiertes C₁-C₅-Alkyl;
   R₉ C₁-C₅-Alkyl;
   R₁₀ Wasserstoff oder C₁-C₃-Alkyl;
   R₁₃ Wasserstoff oder C₁-C₄-Alkyl;
   m 1, 2 oder 3;
   n 0, 1 oder 2; und
   p 0, 1 oder 2.
1.6 Verbindungen aus der Gruppe 1.5, worin bedeuten:
   R₁ C₁-C₅ Alkyl; CF₃, C₂-C₅ Alkoxyalkyl; C₂-C₄ Alkenyl; C₂-C₄ Monohaloalkenyl; oder Halogen;
   R₂ C₁-C₅ Alkyl oder Halogen;
   R₃ Wasserstoff oder C₁-C₃ Alkyl;
   R₄ Wasserstoff; C₁-C₃ Alkyl oder Cyclopropyl;
   R₅ Halogen; C₁-C₂ Alkyl; C₁-C₃ Alkoxy;
   R₈ Wasserstoff;
   n 0, 1, 2 oder 3; und
   m 1.
1.7 Verbindungen aus der Gruppe 1.6, worin bedeuten:
   R₁ C₁-C₄ Alkyl; CF₃; C₂-C₄ Alkoxyalkyl; C₂-C₄ Alkenyl; oder Halogen;
   R₂ C₁-C₄ Alkyl oder Halogen;
   R₃ Wasserstoff oder C₁-C₃ Alkyl;
   R₄ Wasserstoff oder C₁-C₃ Alkyl;
   R₅ Halogen; Methyl; Ethyl oder Methoxy;
   R₈ Wasserstoff;
   n 0, 1, 2 oder 3; und
   m 1.
1.8 Verbindungen aus der Gruppe 1.7 der Formel I'' 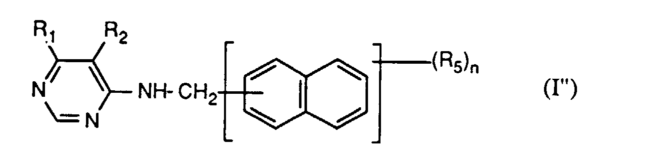 worin bedeuten:
   R₁ C₁-C₄ Alkyl, CF₃, C₂-C₄ Alkoxyalkyl oder Halogen;
   R₂ C₁-C₄ Alkyl oder Halogen;
   R₅ Chlor, Brom, Methyl, Ethyl, Methoxy und
   n 0, 1 oder 2.
1.9 Verbindungen der Formel I''' 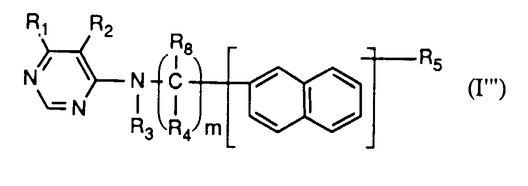 worin bedeuten:
   R₁ Wasserstoff, C₁-C₃ Alkyl, CF₃ oder Halogen;
   R₂ Wasserstoff, C₁-C₃ Alkyl, Halogen, NO₂ oder S(O)ₚ-C₁-C₃-Alkyl;
   R₃ Wasserstoff;
   R₄ Methyl, Ethyl, Isopropyl, n-Propyl oder Cyclopropyl;
   R₈ Wasserstoff, Methyl oder Ethyl;
   R₅ Wasserstoff, Halogen, C₁-C₃-Alkyl, OCHF₂, CF₃, NO₂ oder C₁-C₃-Alkoxy;
   m 1 oder 2; und
   p 0,1 oder 2.
1.10 Verbindungen der Gruppe 1.2, worin bedeuten:
   R₁ Wasserstoff, C₁-C₅ Alkyl; C₁-C₃ Halogenalkyl mit 1 bis 3 Halogenatomen; C₂-C₅ Alkoxyalkyl; C₂-C₅ Alkenyl; C₂-C₅ Halogenalkenyl mit 1 bis 3 Halogenatomen, C₃-C₆-Cycloalkyl oder Halogen;
   R₂ Wasserstoff, C₁-C₅ Alkyl; C₁-C₃ Halogenalkyl mit 1 bis 3 Halogenatomen; C₃-C₅ Alkoxyalkyl; Nitro, Cyano, S(O)ₚ-C₁-C₄-Alkyl, Amino, NHR₃, N(R₃)R₉, N=C(R₉)R₁₀ oder Halogen;
   R₃ Wasserstoff; C₁-C₅ Alkyl, Benzyl, COR₆ oder SR₇;
   R₄ und R₈ unabhängig voneinander Wasserstoff; C₁-C₅ Alkyl; C₁-C₅ Halogenalkyl mit 1 bis 3 Halogenatomen; C₂-C₆ Alkoxyalkyl oder C₃-C₆ Cycloalkyl;
   R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, Cyano oder Nitro oder eine am Naphthylring auftretende X-Phenylgruppe, die unsubstituiert oder ein- bis dreifach gleich oder verschieden durch Halogen, C₁-C₃-Alkyl oder C₁-C₃-Alkoxy substituiert ist;
   R₆ C₁-C₅ Alkyl, Phenyl, durch Halogen und/oder C₁-C₃ Alkyl substituiertes Phenyl;
   R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituierter Benzyl, oder C₁-C₅ Alkyl oder durch Halogen oder Cyano substituiertes C₁-C₅ Alkyl;
   R₉ C₁-C₅ Alkyl;
   R₁₀ Wasserstoff oder C₁-C₅ Alkyl;
   R₁₃ Wasserstoff;
   X Sauerstoff oder Schwefel
   m 2 oder 3,
   n 0, 1, 2 oder 3; und
   p 0,1 oder 2.
1.11 Verbindungen der Gruppe 1.10, worin bedeuten:
   R₁ Wasserstoff, C₁-C₃ Alkyl; C₁-C₃ Halogenalkyl mit 1 bis 3 Halogenatomen; C₂-C₅ Alkoxyalkyl; C₂-C₅ Alkenyl; C₃-C₆-Cycloalkyl oder Halogen;
   R₂ Wasserstoff, C₁-C₅ Alkyl; C₁-C₃ Halogenalkyl mit 1 bis 3 Halogenatomen; C₃-C₅ Alkoxyalkyl; Nitro, Cyano, S(O)ₚ-C₁-C₃-Alkyl, Amino, NHR₃, N(R₃)R₉, N=C(R₉)R₁₀ oder Halogen;
   R₃ Wasserstoff; C₁-C₃ Alkyl;
   R₄ und R₈ unabhängig voneinander Wasserstoff; C₁-C₅ Alkyl; CF₃; C₂-C₆ Alkoxyalkyl oder C₃-C₆ Cycloalkyl;
   R₅ Halogen; C₁-C₃ Alkyl; C₁-C₃ Alkoxy; C₂-C₆ Alkoxyalkyl, C₁-C₃ Halogenalkyl, C₁-C₃ Alkylthio, C₁-C₃ Halogenalkoxy, C₁-C₃ Halogenalkylthio, Cyano oder Nitro;
   R₆ C₁-C₅ Alkyl, Phenyl, durch Halogen und/oder C₁-C₃ Alkyl substituiertes Phenyl;
   R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituiertes Benzyl, oder C₁-C₅ Alkyl oder durch Halogen oder Cyano substituiertes C₁-C₅ Alkyl;
   R₉ C₁-C₅ Alkyl;
   R₁₀ Wasserstoff oder C₁-C₅ Alkyl;
   R₁₃ Wasserstoff,
   X Sauerstoff oder Schwefel
   m 2 oder 3,
   n 0, 1, 2 oder 3; und
   p 0, 1 oder 2.
1.12 Verbindungen der Gruppe 1.11, worin bedeuten:
   R₁ Wasserstoff, C₁-C₃ Alkyl; CF₃, C₂-C₅ Alkoxyalkyl; C₂-C₅ Alkenyl; C₃-C₆-Cycloalkyl oder Halogen;
   R₂ Wasserstoff, C₁-C₅ Alkyl; CF₃, C₃-C₅ Alkoxyalkyl; Nitro, Cyano, S(O)ₚ-C₁-C₄-Alkyl, Amino, N(R₃)R₉, N=C(R₉)R₁₀ oder Halogen;
   R₃ Wasserstoff;
   R₄ und R₈ unabhängig voneinander Wasserstoff; C₁-C₅ Alkyl; C₂-C₆ Alkoxyalkyl oder C₃-C₆ Cycloalkyl;
   R₅ Halogen; C₁-C₃ Alkyl; C₁-C₃ Alkoxy; C₂-C₆ Alkoxyalkyl, Halogenalkyl, C₁-C₃ Alkylthio, C₁-C₃ Halogenalkoxy, C₁-C₃ Halogenalkylthio, Nitro oder Cyano;
   R₆ C₁-C₅ Alkyl, Phenyl oder durch Halogen und/oder C₁-C₃ Alkyl substituiert ist;
   R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituiertes Benzyl, oder C₁-C₅ Alkyl oder durch Halogen oder Cyano substituiertes C₁-C₅ Alkyl;
   R₉ C₁-C₃ Alkyl;
   R₁₀ Wasserstoff oder C₁-C₃ Alkyl;
   R₁₃ Wasserstoff;
   X Sauerstoff oder Schwefel
   m 2;
   n 0, 1, 2 oder 3; und
   p 0, 1 oder 2.
1.13 Verbindungen der Gruppe 1.12, worin bedeuten:
   R₁ Wasserstoff, C₁-C₃ Alkyl; CF₃, C₂-C₅ Alkoxyalkyl; C₂-C₅ Alkenyl; C₃-C₆-Cycloalkyl oder Halogen;
   R₂ Wasserstoff, C₁-C₅ Alkyl; CF₃, C₃-C₅ Alkoxyalkyl; Nitro, Cyano, S(O)ₚ-C₁-C₃-Alkyl, Amino, N(R₃)R₉, N=C(R₉)R₁₀ oder Halogen;
   R₃ Wasserstoff;
   R₄ und R₈ unabhängig voneinander Wasserstoff; C₁-C₅ Alkyl; C₂-C₆ Alkoxyalkyl oder C₃-C₆ Cycloalkyl;
   R₅ Halogen; C₁-C₃ Alkyl; C₁-C₃ Alkoxy; C₂-C₆ Alkoxyalkyl, C₁-C₃ Halogenalkyl, C₁-C₃ Alkylthio, C₁-C₃ Halogenalkoxy, C₁-C₃ Halogenalkylthio, Nitro oder Cyano;
   R₆ C₁-C₅ Alkyl, Phenyl oder durch Halogen und/oder C₁-C₃ Alkyl substituiertes Phenyl;
   R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituiertes Benzyl, oder C₁-C₅ Alkyl oder durch Halogen oder Cyano substituiertes C₁-C₅ Alkyl;
   R₉ C₁-C₅ Alkyl;
   R₁₀ Wasserstoff oder C₁-C₃ Alkyl;
   R₁₃ Wasserstoff;
   X Sauerstoff oder Schwefel
   m 3,
   n 0, 1, 2 oder 3; und
   p 0, 1 oder 2.

Eine besondere Gruppe von Wirkstoffen der vorliegenden Erfindung stellen die Verbindungen der Formel Ia dar: 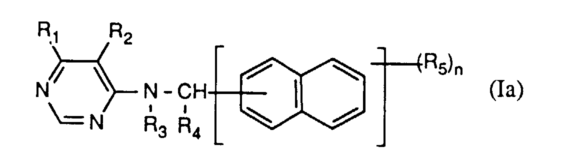 in welcher bedeuten:
R₁ C₁-C₅ Alkyl, das unsubstituiert oder durch Halogen oder C₁-C₃ Alkoxy substituiert ist; C₂-C₇ Alkenyl, das unsubstituiert oder durch Halogen substituiert ist; C₃-C₇ Cycloalkyl; oder Halogen;
R₂ C₁-C₅ Alkyl, das unsubstituiert oder durch Halogen oder C₁-C₃ Alkoxy substiutiert ist; Halogen; Nitro oder Cyano;
R₃ Wasserstoff; C₁-C₅ Alkyl; Benzyl; -CO-R₆ oder -S-R₇;
R₄ Wasserstoff; C₁-C₅ Alkyl, das unsubstituiert oder durch Halogen oder C₁-C₃ Alkoxy substituiert ist; oder C₃-C₇ Cycloalkyl;
R₅ Halogen; C₁-C₃ Alkyl; C₁-C₃ Alkoxy; C₁-C₃Alkylthio; oder eine einmal am Phenylring auftretende -X-Phenyl-Gruppe, die unsubstituiert oder ein- bis dreifach gleich oder verschieden durch Halogen, C₁-C₃ Alkyl oder C₁-C₃ Alkoxy substituiert ist;
R₆ C₁-C₅ Alkyl; Phenyl, das unsubstituiert oder durch Halogen und/oder C₁-C₃ Alkyl substituiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
X Sauerstoff oder Schwefel; und
n 0, 1, 2 oder 3.

Hinsichtlich ihrer biologischen Aktivität in der Bekämpfung von Schädlingen sind folgende Verbindungen als bevorzugt anzusehen:
(d,l)-4-[1'-(β-Naphthyl)-ethylamino]-5-chlor-6-ethylpyrimidin (Verb. 1.2);
(-)-4-[1'-(β-Naphthyl)-ethylamino]-5-chlor-6-ethylpyrimidin (Verb. 1.73);
(d,l)-4-[1'-(β-Naphthyl)-propylamino]-5-chlor-6-ethylpyrimidin (Verb. 1.3);
(d,l)-4-[1'-(β-Naphthyl)-ethylamino]-5-chlor-6-methylpyrimidin (Verb. 1.53);
(d,l)-4-[1'-(2-(6-Bromnaphthyl))-ethylamino]-5-chlor-6-ethylpyrimidin (Verb. 1.149);
(d,l)-4-[1'-(β-Naphthyl)-ethylamino]-5-brom-6-ethylpyrimidin (Verb. 1.84);
(d,l)-4-[1'-(β-Naphthyl)-ethylamino]-5-chlor-6-n-propylpyrimidin (Verb. 1.86);
(d,l)-4-[1'-(β-Naphthyl)-ethylamino]-5-jod-6-ethylpyrimidin (Verb. 1.100).

Unter Alkyl selbst oder unter Alkyl als Bestandteil eines anderen Substituenten, wie Haloalkyl, Alkoxy oder Alkylthio, ist je nach Anzahl der benannten Kohlenstoffatome beispielsweise Methyl, Ethyl, Propyl, Butyl, Pentyl, sowie ihre Isomeren, wie z.B. Isopropyl, Isobutyl, Isoamyl, tert.-Butyl oder sek.-Butyl zu verstehen.

C₂-C₇-Alkenyl stellt einen aliphatischen Rest mit einer Doppelbindung dar, z.B. Allyl, Vinyl, Methallyl, Crotyl, Butenyl, Pentenyl usw.

Halogen steht für Fluor, Chlor, Brom oder Jod.

Haloalkyl bezeichnet einfach bis perhalogenierte Reste, wie z.B. CHCl₂, CH₂F, CCl₃, CH₂Cl, CHF₂, CF₃, CH₂CH₂Br, C₂Cl₅, CH₂Br, CHBrCl usw., vorzugsweise CF₃ und CHF₂.

Cycloalkyl steht je nach Zahl der genannten Kohlenstoffatome z.B. für Cyclopropyl, Cyclobutyl, Cyclopentyl, Cyclohexyl oder Cycloheptyl.

4-Aminopyrimidinverbindungen sind bereits bekannt. z.B. aus der EP-A-264 217. Die erfindungsgemässen Verbindungen der Formel I unterscheiden sich in charakteristischer Weise von den bekannten Verbindungen durch die Kombination eines 4-Aminopyrimidinrings mit einem Naphthylalkyl-Rest, wodurch bei den neuen Verbindungen eine unerwartet hohe mikrobizide und insektizid/akarizide Wirksamkeit erreicht wird.

Die Verbindungen der Formel I sind bei Raumtemperatur stabile Oele, Harze oder Feststoffe. Sie lassen sich auf dem Agrarsektor oder verwandten Gebieten präventiv und kurativ zur Bekämpfung von pflanzenschädigenden Mikroorganismen, von Insekten und von Schädlingen der Ordnung Akarina einsetzen. Die erfindungsgemässen Wirkstoffe der Formel I zeichnen sich bei niedrigen Anwendungskonzentrationen nicht nur durch hervorragende Wirkung, sondern auch durch besonders gute Pflanzenverträglichkeit aus.

Die Verbindungen der Formel I können in Nachbarstellung zu R₄ ein asymmetrisches Kohlenstoffatom besitzen und können daher in enantiomeren bzw. diastereomeren Formen vorliegen. Im allgemeinen entsteht bei der Herstellung dieser Substanzen ein Gemisch von Enantiomeren bzw. Diastereomeren. Diese lassen sich auf übliche Weise, z.B. durch fraktionierte Kristallisation von Salzen mit optisch aktiven Säuren, in die reinen optischen Antipoden aufspalten. Solche Verbindungen können aber auch gezielt hergestellt werden durch diastereo- oder enantio-selektive Methoden. Die Enantiomeren können unterschiedliche biologische Wirkungen besitzen.

Die vorliegende Erfindung betrifft daher die racemischen Verbindungen und alle Isomeren der Formel I, ihre Herstellung und ihre Verwendung im Pflanzenschutz sowie die diese als Wirkstoffe enthaltenden Mittel.

Verbindungen der Formel I lassen sich nach verschiedenen Herstellungsvarianten gewinnen, indem man beispielsweise eine Verbindung der Formel II 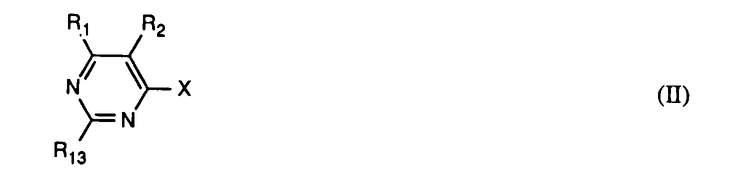 worin R₁, R₂ und R₁₃ die für Formel I angegebene Bedeutung haben und X einen leicht abspaltbaren Rest bedeutet, mit einer Verbindung der Formel III 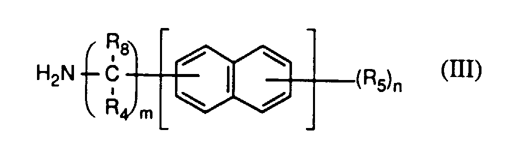 worin R₄, R₅, R₈, m und n die für Formel I gegebene Bedeutung haben, vorzugsweise in Gegenwart einer Base zur Reaktion bringt. Anschliessend kann der Substituent R₃ (Alkyl, Benzyl, Acyl oder -S-R₇) durch entsprechende N-Alkylierung, N-Benzylierung, N-Acylierung, N-Thioalkylierung oder N-Thioarylierung eingeführt werden.

Leicht abspaltbare Reste sind beispielsweise Halogen wie Chlor, Brom, Jod; C₁-C₆-Alkylthioreste wie Methylthio, Aethylthio, Propylthio; (Halo) Alkansulfonyloxygruppen wie Mesyloxy, Ethansulfonyloxy oder Trifluormethansulfonyloxy; Arylsulfonyloxygruppen wie Benzolsulfonyloxy oder Tosyloxy.

Als Basen zur Erleichterung der HX-Abspaltung eignen sich anorganische Vertreter wie Kalium- oder Natriumcarbonat, NaH und andere. Es eignen sich auch organische Basen wie Triäthylamin, Pyridin, N,N-Diäthylanilin, Triäthylendiamin und andere. Bevorzugt wegen seiner katalytischen Wirkung ist 4-(N,N-dimethylamino)pyridin.

Die Reaktionspartner können als solche ohne Zusatz eines Lösungsmittels, z.B. in der Schmelze, miteinander reagieren. In der Mehrzahl der Fälle ist jedoch der Zusatz eines Lösungsmittels oder mehrerer Lösungs- bzw. Verdünnungsmittel vorteilhaft. Als Beispiele seien aromatische, aliphatische und alicyclische Kohlenstoffe und Halogenkohlenwasserstoffe genannt, z.B. Benzol, Toluol, Xylol, Chlorbenzol, Brombenzol, Petroläther, Hexan, Cyclohexan, Methylenchlorid, Chloroform, Dichloräthan, Trichloräthylen; Aether wie Diäthyläther, Tetrahydrofuran (THF), Dioxan, tert.Butylmethyläther; Ketone wie Aceton, Methyläthylketon; Alkohole wie Methanol, Aethanol, Propanol, Butanol, Aethylenglycol, Glycerin; Amide wie Dimethylformamid (DMF), N,N-Dimethylacetamid; ferner Acetonitril, Dimethylsulfoxid (DMSO), dann auch eine im Ueberschuss zugesetzte Base wie Pyridin, N,N-Diäthylanilin, Triäthylamin und andere.

Die nachträgliche N-Alkylierung oder N-Benzylierung zur Einführung des Substituenten R₃ erfolgt auf übliche Art und Weise mit einem Alkyl- bzw. Benzylhalogenid, besonders dem entsprechenden Bromid, in Gegenwart einer starken Base. Eine N-Acylierung wird in üblicher Art mit einer C₁-C₆-Alkancarbonsäure bzw. mit einer Benzoesäure oder ihren Säurehalogeniden, insbesondere Säurechloriden oder -bromiden, in inerten trockenen Lösungsmitteln durchgeführt. Eine N-Thioalkylierung bzw. Thioarylierung lässt sich vorteilhaft mit dem entsprechenden Sulfenylchlorid in Gegenwart einer Base durchführen.

Sofern der Substituent R₃ C₁-C₅-Alkyl, Benzyl oder -S-R₇ bedeuten soll, kann nach einer anderen Verfahrensvariante die Verbindung der Formel II auch direkt mit einer Verbindung der Formel IV zur Reaktion gebracht werden: 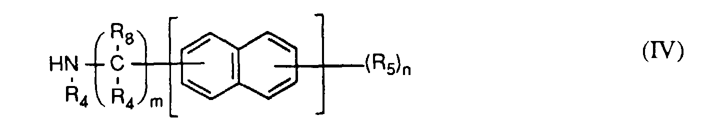 In diesem Falle ist die Gegenwart starker Basen unerlässlich.

Die Temperatur beträgt in den vorgenannten Verfahren 0° bis 180°C, bevorzugt 20° bis 130°C, und entspricht in vielen Fällen der Rückflusstemperatur des Lösungsmittels.

Folgende Pyrimidin-Verbindungen der Formel II' sind neu: 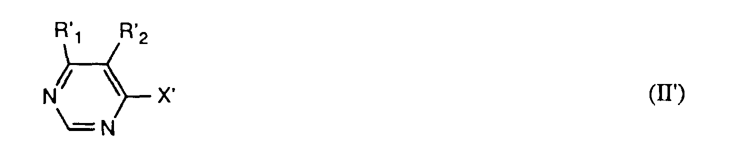 worin bedeuten
R'₁ CH₃, C₂H₅, n-C₃H₇, n-C₄H₉, CH(CH₃)₂ oder CF₃;
R'₂ Wasserstoff, Fluor, Chlor, Brom, Jod, Nitro oder Amino; und
R'₃ Hydroxy oder Chlor;
mit der Massgabe, dass nicht gleichzeitig sein dürfen:
1) R'₁ CH₃ oder C₂H₅; R'₂ Wasserstoff oder Chlor; und X' Hydroxy oder Chlor;
2) R'₁ CH₃; R'₂ Brom, Jod, Nitro oder Amino; und X' Hydroxy oder Chlor;
3) R'₁ n-C₃H₇ oder CH(CH₃)₂; R'₂ Wasserstoff; und X' Hydroxy;
4) R'₁ CF₃; R'₂ Wasserstoff; und X' Hydroxy oder Chlor;
5) R'₁ CF₃; R'₂ Chlor, Brom oder Jod; und X' Hydroxy;
6) R'₁ CH₃ oder n-C₄H₉; R'₂ Fluor; und X' Hydroxy.

Die neuen Verbindungen der Formel II' stellen einen Bestandteil der vorliegenden Erfindung dar.

Pyrimidine der Formel II sind entweder bereits bekannt oder werden nach allgemeinen Synthesemethoden hergestellt (vgl. D.J. Brown "The Pyrimidines" in Heterocyclic Compounds).

Nach D.J. Brown (ebd., Seite 10) benötigt die Nitrierung von Pyrimidinen wenigstens zwei Elektronendonatoren als Substituenten, weshalb die Nitrierung von 6-Alkyl-4-hydroxypyrimidinen unter den üblichen Nitrierbedingungen selbst in Gemischen aus 100 %iger Salpetersäure und 100 %iger Schwefelsäure nicht eintritt. Ueberraschenderweise lassen sich die 6-Alkyl-4-hydroxy-5-nitropyrimidine jedoch statt in 100 %iger Schwefelsäure in Oleum herstellen:

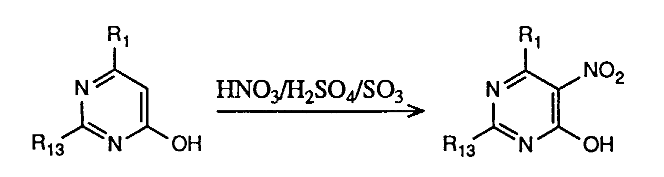

Dabei ist die Einhaltung möglichst niedriger Temperaturen erforderlich, um Nebenreaktionen zu unterdrücken. Als günstig erweisen sich 10° bis 70°C, vorzugsweise 20°-40°C. Als Oleum kommt H₂SO₄ mit Gehalten an SO₃ von 5 bis 70 % in Betracht, insbesondere solches mit 10 bis 40 % SO₃.

Analog den in der Literatur beschriebenen Methoden lassen sich leicht Chlor, Brom und Jod in 5-Stellung der 4-Hydroxy-pyrimidine einführen:

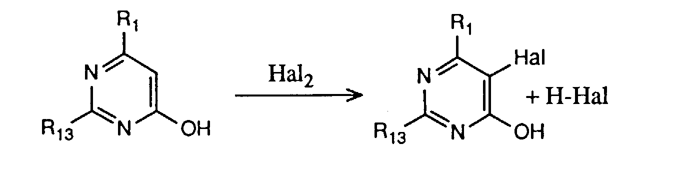

Man dosiert dabei die Halogene zu einer wässerigen Lösung oder Suspension der Pyrimidine und neutralisiert die entstehende Säure. Auch aliphatische Carbonsäuren, wie z.B. Essigsäure, eignen sich für die Halogenierungen als Lösungsmittel.

Die Ueberführung der 4-Hydroxypyrimidine in die 4-Chlorpyrimidine erfolgt nach den allgemein üblichen Methoden, vorzugsweise mit Phosphoroxychlorid:

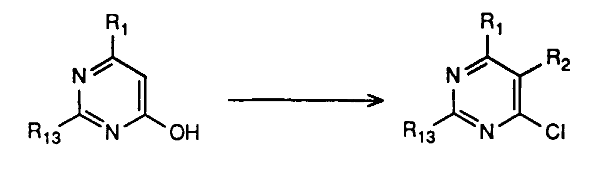

Hierbei kann mit einem Ueberschuss an Phosphoroxychlorid ohne Lösungsmittel gearbeitet werden, oder man verwendet inerte Lösungsmittel, wie z.B. Toluol oder Xylol. In vielen Fällen wirkt sich der Zusatz organischer Basen, wie z.B. Dimethyl- oder Diäthylanilin, günstig auf die Ausbeute aus. Man arbeitet in einem Temperaturbereich von 20° bis 140°C, vorzugsweise bei 40° bis 100°C.

Durch Reduktion der 5-Nitropyrimidine erhält man die 5-Aminopyrimidine:

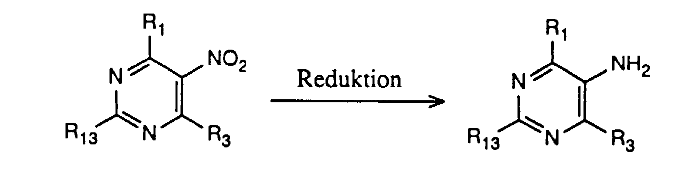

Hierbei können die allgemein üblichen Reduktionsmittel verwendet werden, wie z.B. Eisen- oder Zinkpulver in Lösungsmitteln, z.B. Essigsäure, oder metallkatalytisch aktivierter Wasserstoff.

Etliche Pyrimidine der Formel II können nach folgendem Reaktionsschema hergestellt werden.

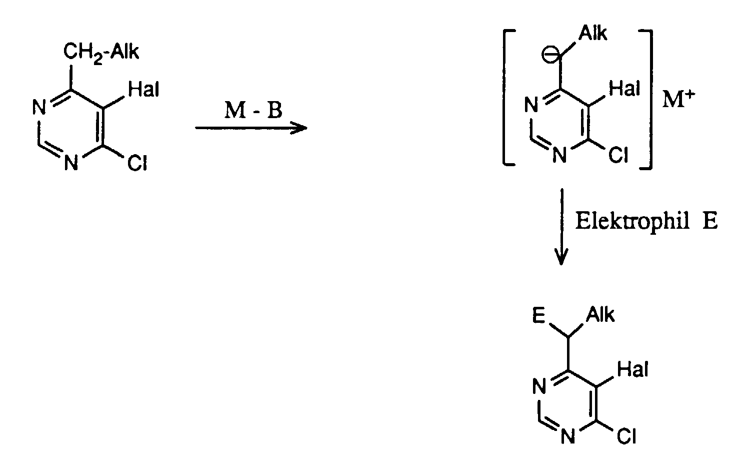

Als Basen (M-B) werden Alkali-/Erdalkali-organische Verbindungen, wie z.B. Alkyllithium, oder bevorzugt Lithiumdialkylaminoverbindungen, wie z.B. Lithiumdiisopropylamin, verwendet. Als Elektrophile können z.B. Halogenalkylverbindungen, wie Alkyljodide, Allylbromide oder Alkyl-SO₂-S-Alkyl und weitere allgemein übliche Elektrophile eingesetzt werden.

Die in den vorstehend beschriebenen Herstellungsverfahren aufgeführten Reste R₁ und R₂ besitzen die unter Formel I angegebenen Bedeutungen und Hal bedeutet Halogen.

Verbindungen der Formel (I), in denen R₂ = Halogen oder NO₂ ist, kann man auch so herstellen, dass man in 5-Stellung unsubstituierte Verbindungen (I) (R₂ = H) halogeniert oder nitriert.

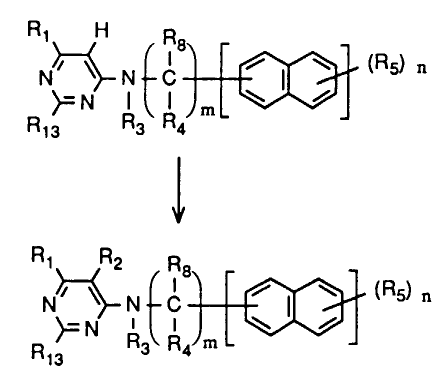

Die Halogenierungen oder Nitrierungen erfolgen unter den zuvor für die Herstellung der 4-Hydroxypyrimidine genannten Bedingungen.

Naphthalinderivate der Formel III bzw. IV sind gleichfalls aus der Literatur bekannt bzw. lassen sich nach bekannten Methoden z.B. wie folgt herstellen:
1) aus den entsprechenden Aldehyden oder Ketonen durch Umsetzung mit Aminen und Ameisensäure oder Formamid und Ameisensäure nach LEUCKART u. WALLACH 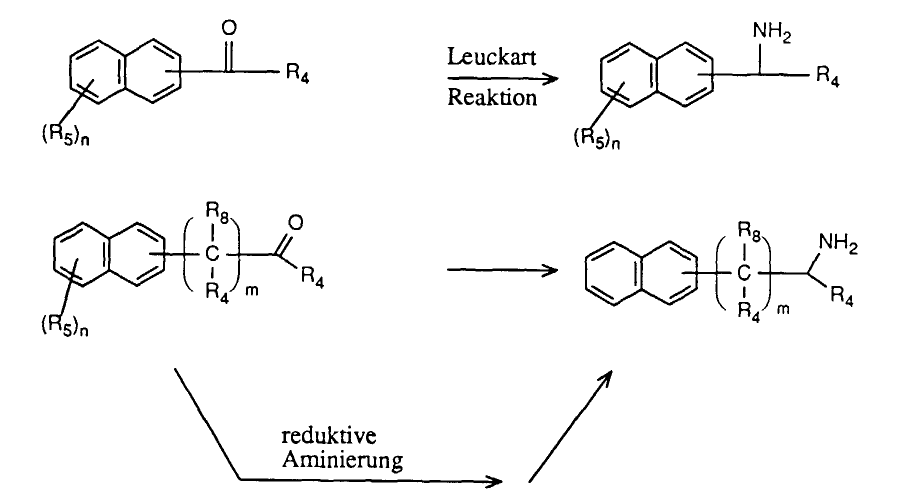 oder durch reduktive Aminierung der Aldehyde oder Ketone mit NH₂-R₃ und Wasserstoff in Gegenwart von Metallkatalysatoren; oder durch Reduktion der aus den Aldehyden oder Ketonen mit Hydroxylamin hergestellten Oxime. Die Reduktion der Oxime kann durch Wasserstoff in Gegenwart von Metallkatalysatoren oder durch Komplexhydride wie LiAlH₄ erfolgen: 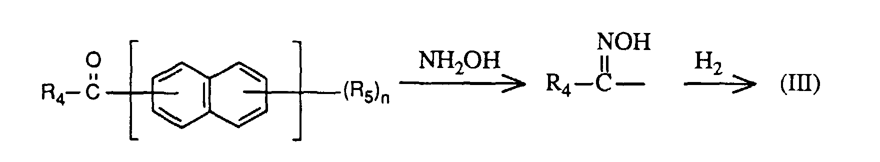
   Ebenso können die Naphthalinderivate der Formel III bzw. IV aus Aldehyden bzw. Ketonen durch Umsetzung mit Ammoniumformiat und anschliessende Hydrolyse gewonnen werden: 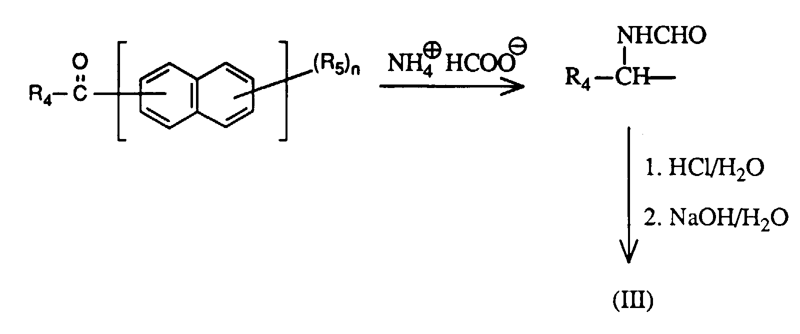
2) durch Reduktion von Nitrilen oder Amiden mittels Wasserstoff in Gegenwart von Metallkatalysatoren oder mittels Komplexhydride wie LiAlH₄: 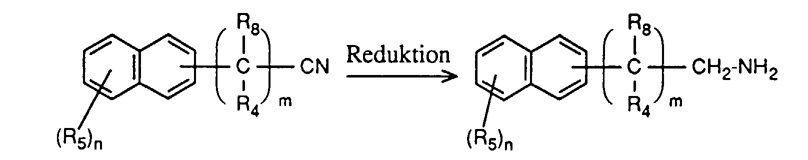 oder 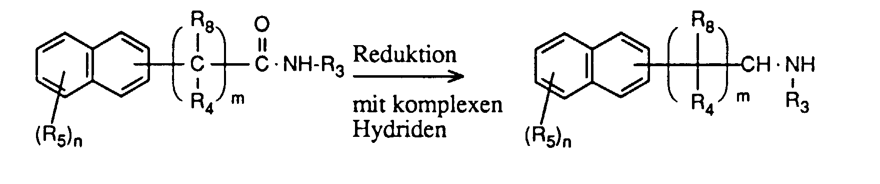
3) durch Reduktion von Nitroverbindungen mit Wasserstoff und Metallkatalysatoren: 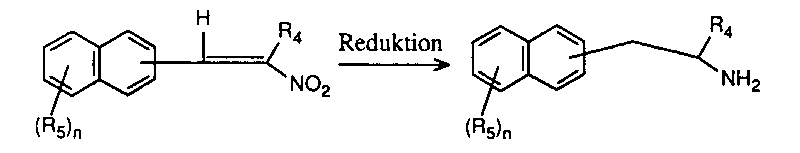 Die beschriebenen Herstellungsverfahren einschliesslich aller Teilschritte sind ein Bestandteil der vorliegenden Erfindung.

Die enantionmeren reinen Verbindungen der Formel I lassen sich wie folgt herstellen:
1. Durch Auftrennen der racemischen Form der Verbindungen der Formel I nach den allgemein bekannten Methoden der Racematspaltung. Dies kann durch Umsetzung der racemischen Verbindungen der Formel I mit einer enantiomerenreinen Carbonsäure oder Sulfosäure, wie Weinsäure, Camphersäure, Camphersulfonsäure etc. zu den Salzen und anschliessender fraktionierter Kristallisation der diastereomeren Salze, geschehen. Aus den reinen diastereomeren Salzen können dann die enantiomerreinen Verbindungen der Formel I mit Basen wieder freigesetzt werden;
2. durch Auftrennen der racemischen Form der Verbindungen der Formel I durch Chromatographie, speziell HPLC-Chromatographie an chiralem Trägermaterial, wie Acetylcellulose etc.;
3. durch Umsetzung der Pyrimidine der Formel II mit enantiomeren reinen Aminen der Formel III resp. der Formel IV. Enantiomer reine Amine der Formeln III oder IV lassen sich analog den oben beschriebenen Methoden oder nach allgemein bekannten Methoden der enantioselektiven Synthese herstellen.

Verbindungen der Formel I, in denen R₂ einen Thioalkylrest bedeutet, werden durch Austausch von R₂ = Halogen mit Mercaptanen unter basischen Bedingungen erhalten. Sie lassen sich durch Oxidation in die entsprechenden Sulfone und Sulfoxyde überführen.

Ueberraschenderweise wurde nun gefunden, dass Verbindungen der Formel I ein für praktische Bedürfnisse sehr günstiges biozides Spektrum zur Bekämpfung von Insekten, Schädlingen der Ordnung Akarina und von phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Verbindungen der Formel I sind z.B. gegen die den folgenden Klassen angehörenden phytopathogenen Pilze wirksam: Fungi imperfecti (z.B. Botrytis, Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. Venturia und Erysiphe, Podosphaera, Monilinia, Uncinula) und der Oomyceten (z.B. Phytophthora, Pythium, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Es wurde ferner gefunden, dass die erfindungsgemässen Verbindungen der Formel I wertvolle Wirkstoffe in der Bekämpfung von Schadinsekten und Akarina sind, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Sie sind gegen verschiedene Entwicklungsstadien dieser Arten wirksam. Dabei kann sich ihre Wirkung in unmittelbarer Abtötung der Schädlinge oder erst nach einiger Zeit, beispielsweise bei einer Häutung, oder in einer deutlich verminderten Eiablage und/oder Schlupfrate zeigen. Zu den oben erwähnten Schädlingen gehören:

Die Ordnung Lepidoptera (z.B. Chilo spp. und Heliothis spp.), die Ordnung Coleoptera (z.B. Anthonomus spp., Epilachna spp., Leptinotarsa decemlineata), die Ordnung Homoptera (z.B. Bemisia tabaci, Nephotettix spp., Nilaparvata spp.) und die Ordnung Acarina (z.B. Boophilus spp. und Tetranychus spp.). Diese Aufzählung ist nicht limitierend.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Eierfrüchte, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt Günstige Aufwandmengen liegen im allgemeinen bei 5 g bis 2 kg Aktivsubstanz (AS) je ha, bevorzugt 10 g bis 1 kg AS/ha, insbesondere bei 20 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden. Die agrochemischen Zubereitungen enthalten in der Regel 0,1 bis 99 %, insbesondere 0,1 bis 95 %, Wirkstoff der Formel I, 99,9 bis 1 %, insbesondere 99,8 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

### Herstellungsbeispiele

### Beispiel 1: Herstellung von d,l-4-[1'-(β-Naphthyl)-äthylamino]-5-chlor-6-äthyl-pyrimidin der Formel

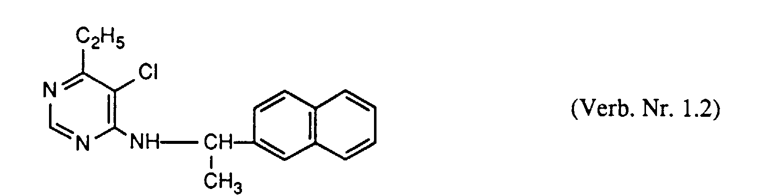

Zu einer Lösung von 12,39 g 4,5-Dichlor-6-äthyl-pyrimidin in 150 ml n-Butanol werden 11,98 g 1-(β-Naphthyl)-1-amino-äthan und 20 ml Triäthylamin gefügt. Das Gemisch wird 12 Std. unter Rückfluss gekocht. Nach dem Eindampfen der Reaktionslösung wird das Rohprodukt in 20 ml Chloroform gelöst, mit 6 ml konz. Chlorwasserstoffsäure versetzt und mit Wasser ausgeschüttelt. Nach dem Abtrennen und Trocknen der organischen Phase mit Natriumsulfat und Abdestillieren des Lösungsmittels, erhält man ein hellbraunes Oel. Das Oel wird mit Hexan verrieben, woraufhin das gewünschte Endprodukt auskristallisiert. Nach dem Abfiltrieren erhält man 11,6 g Substanz; Smp.: 80-81°C.

Das gewonnene Racemat kann durch fraktionierte Kristallisation mit z.B. optisch aktiver Weinsäure in ein (+)-Enantiomeres und sein biologisch wirksameres (-)-Enantiomeres aufgetrennt werden oder durch Auftrennen an einer chiralen mittels HPLC gewonnen werden.

### Beispiel 2: Herstellung von (d,l)-4-[1'-(2-(6-Methoxynaphthyl))-propylamino]-5-chlor-6-äthyl-pyrimidin

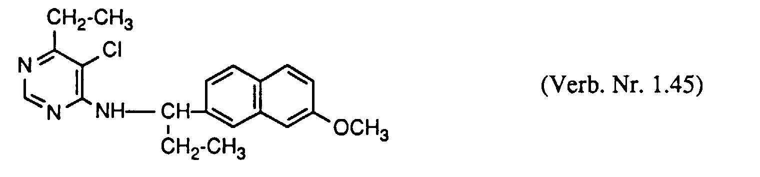

2,1 g 1-(2-(6-Methoxynaphthyl))-1-aminopropan, 2,1 g 4,5-Dichlor-6-äthylpyrimidin und 1,3 g Triäthylamin werden in 50 ml n-Butanol gegeben. Diese Lösung wird 12 Stunden unter Rückfluss erhitzt. Anschliessend wird das Lösungsmittel weitgehend abgedampft, der Rückstand mit Wasser versetzt und das Produkt mit Essigsäureethylester extrahiert. Die Lösung wird über Na₂SO₄ getrocknet und eingeengt. Der Rückstand wird durch eine kurze Säule an Kieselgel mit Essigsäureethylester/Hexan (1:1) chromatographiert. Ausbeute 2,4 g (Harz).

### Beispiel 2a: Herstellung von 2-(6-Methoxynaphthyl)-ethyl-ketoxim

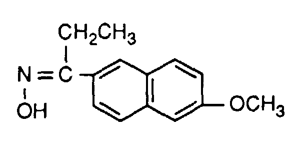

21,4 g 6-Methoxy-2-propionyl-naphthalin werden in 100 ml Ethanol gelöst. Zu dieser Lösung werden 7,7 g Hydroxylammoniumchlorid und 9,5 g Pyridin gegeben und anschliessend ca. 12 Stunden am Rückfluss gekocht. Nach dem Abkühlen wird das Lösungsmittel am Rotavapor abdestilliert, der Rückstand in Wasser aufgenommen und mit Essigsäureethylester extrahiert. Nach dem Waschen des Essigsäureethylesterextraktes mit Wasser und Sole wird dieser über Na₂SO₄ getrocknet, filtriert und am Rotavapor eingeengt. Der Rückstand wird aus Cyclohexan umkristallisiert. Ausbeute: 18,4 g, Smp. 161-162°C.

### Beispiel 2b: Herstellung von 1'-[2-(6-Methoxynaphthyl)]-propylamin

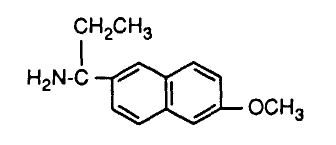

7,4 g 2-(6-Methoxynaphthyl)-äthyl-ketoxim werden in 100 ml Methanol und 6 g Ammoniak (flüssig) unter Zusatz von 0,7 g Raney-Nickel bei 60-70°C und 10⁷ Pa Druck mit Wasserstoff bis zum Stillstand hydriert. Der Katalysator wird über Hyflo abfiltiert und mit Methanol nachgewaschen. Das Methanol wird am Rotavapor unter vermindertem Druck abgedampft, der Rückstand in Methylenchlorid aufgenommen und mit Wasser gewaschen. Die über Na₂SO₄ getrocknete Lösung wird filtriert und das Lösungsmittel anschliessend abgedampft. Der Rückstand wird aus Diethylether/Hexan umkristallisiert. Ausbeute: 5,6 g; Smp. 61-62°C.

### Beispiel 3: Herstellung von (d,l)-4-[1'-(2-(1-Difluormethoxynaphthyl))-äthylamino]-5-chlor-6-äthyl-pyrimidin

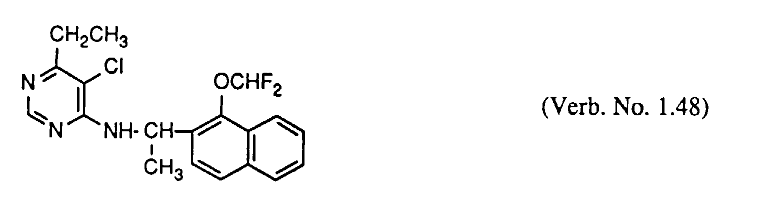

2,1 g Methyl-2-(1-difluormethoxynaphthyl)-methylamin, 1,6 g 4,5-Dichlor-6-ethylpyrimidin und 1,2 g Triethylamin werden zu 20 ml n-Butanol gegeben. Die Lösung wird ca. 12 Stunden bei 100°C gehalten. Anschliessend wird das Lösungsmittel am Rotavapor unter Vakuum weitgehend abgedampft, der Rückstand mit Wasser versetzt und mehrmals mit Essigsäureethylester extrahiert. Die Essigsäureethylesterlösung wird über Na₂SO₄ getrocknet und eingedampft. Das verbleibende Oel wird an Kieselgel mit Essigsäureethylester/Hexan (3:1) als Eluationsmittel chromatographiert, Ausbeute 1,4 g Oel.

### Beispiel 3a: Herstellung von 1-Difluormethoxy-2-acetylnaphthalin

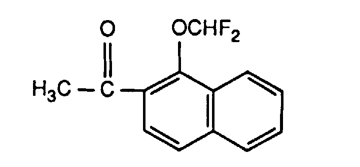

10 g 1-Hydroxy-2-acetylnaphthalin werden in 60 ml Dioxan gelöst und 30 ml 30%ige Natronlauge zugefügt. Bei einer Temperatur zwischen 70°C und 80°C wird Difluor-Chlormethan (Freon 22) solange eingeleitet bis keine Absorption mehr beobachtet wird. Die Dioxanphase wird anschliessend dekantiert, die NaOH-Phase durch dekantieren nachgewaschen mit Dioxan und die vereinigten Dioxanlösungen am Rotavapor unter Vakuum weitgehend eingeengt. Der Rückstand wird mit Wasser versetzt und mit Diethylether mehrmals extrahiert, die Diethyletherlösung über Na₂SO₄ getrocknet, filtriert und eingedampft. Der Rückstand wird aus Diethylether/Hexan umkristallisiert. Ausbeute 4 g, Smp. 80-81°C.

### Beispiel 3b: Herstellung von N-Formyl-1'-[2-(1-difluormethoxynaphthyl)]-ethylamin

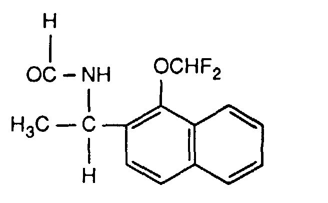

10,3 g 1-Difluormethoxy-2-acetylnaphthalin werden portionenweise zu 20 g Formamid, erhitzt auf 150°C, unter gleichzeitigem Zutropfen von 5 g Ameisensäure, gegeben. Anschliessend wird über Nacht (ca. 12 Stunden) bei dieser Temperatur weitergerührt. Nach dem Abkühlen wird mit Wasser versetzt und das Produkt mit Essigsäureethylester extrahiert. Nach mehrmaligem Nachwaschen der Essigsäureethylesterphase mit Wasser, wird diese über Na₂SO₄ getrocknet und am Rotavapor unter Vakuum eingeengt. Der ölige Rückstand wird an Kieselgel mit Essigsäureethylester (3:1) als Eluationsmittel chromatographiert. Ausbeute: 5,4 g Oel.

### Beispiel 3c: Herstellung von 1'-[2-(1-Difluormethoxynaphthyl)]-ethylamin

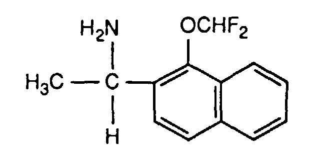

5,4 g von N-Formyl-1'(2-(1-Difluormethoxynaphthyl))-ethylamin werden mit 40 ml Ethanol/Wasser (1:1) versetzt, 1,8 g Kaliumhydroxidplätzchen zugegeben und anschliessend 6 Stunden am Rückfluss gekocht. Anschliessend wird Ethanol am Rotavapor weitgehend abgedampft und der wässrige Rückstand mehrmals mit Diethylether extrahiert. Die Diethyletherlösung wird über Na₂SO₄ getrocknet und am Rotavapor eingeengt. Der erhaltene ölige Rückstand, wird ohne Reinigung direkt weiterverwendet. Ausbeute: 4,1 g.

### Beispiel 4: Herstellung von 4-[1'-(β-Naphthyl)-ethylamino]-5-nitro-6-ethylpyrimidin

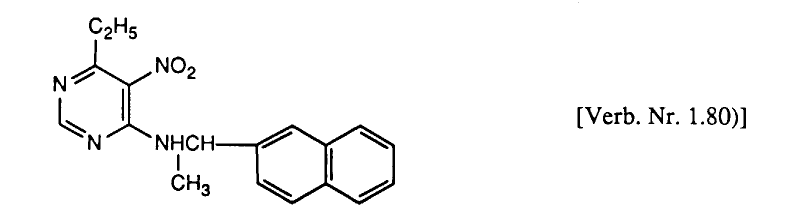

Man löst 1,87 g (0,01 Mol) 4-Chlor-5-nitro-6-ethylpyrimidin mit 1,52 g (0,015 Mol) Triethylamin in 15 ml Tetrahydrofuran und fügt tropfenweise unter Kühlung bei max. 50°C 1,97 g (0,0115 Mol) 1-(β-Naphthyl)-1-aminoethan zu. Nach einer Stunde wird das Gemisch mit Wasser und Essigsäureethylester extrahiert. Einengen des Extrakts ergibt 3,85 g Rohprodukt, das mittels Säulenchromatographie auf Kieselgel (Laufmittel 10 Teile Essigsäureethylester und 90 Teile Hexan) gereinigt wird. Die Ausbeute beträgt 2,75 g (85 % der Theorie) an farblosem Oel.

### Beispiel 4a: Herstellung von 4-Hydroxy-5-nitro-6-ethylpyrimidin

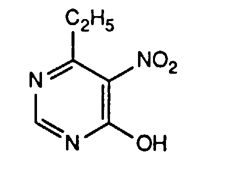

Zu 8 ml 100%iger Salpetersäure lässt man unter Kühlung bei maximal 10°C 8 ml 25%iges Oleum zutropfen. Danach werden in Portionen 12,4 g (0,10 Mol) 4-Hydroxy-6-ethylpyrimidin bei maximal 35°C eingetragen. Man hält über Nacht auf 40°C. Zur weiteren Umsetzung von noch vorhandenem Ausgangsmaterial werden bei maximal 40°C weitere 13 ml 25%iges Oleum und 11 ml 100%ige Salpetersäure zugetropft. Man lässt noch 12 Stunden bei 40°C ausreagieren, giesst sodann vorsichtig auf Eis, extrahiert vollständig mit Essigsäureethylester und wäscht die Extrakte mit Natriumhydrogencarbonatlösung. Beim Einengen des Extrakts am Rotationsverdampfer kristallisieren 11,8 g (69,7 % der Theorie) Reinsubstanz aus; Smp. 181-183°C.

### Beispiel 4b: Herstellung von 4-Chlor-5-nitro-6-ethylpyrimidin

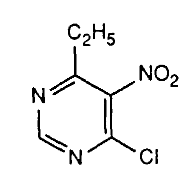

18,7 g (0,125 Mol) Diethylanilin werden in 18 ml Phosphorchlorid gelöst und dann 8,46 g (0,05 Mol) 4-Hydroxy-5-nitro-6-ethylpyrimidin eingerührt. Die Temperatur steigt dabei von 22° auf 47°C. Es entsteht eine Lösung, die noch zwei Stunden auf 60°C erwärmt wird. Sie wird anschliessend zur Hydrolyse mit Eiswasser und Essigsäureethylester 30 Minuten gerührt. Die organische Phase wird abgetrennt, mit den durch Nachextraktion erhaltenen Extrakten vereinigt und mit NaHCO₃-Lösung gewaschen. Nach Entfernung des Lösungsmittels verbleiben 9,1 g Rohprodukt, das durch Säulenchromatographie an Kieselgel (Laufmittel: Essigsäureethylester/Hexan 20:80 Teile) gereinigt wird. Man erhält 6,76 g (72,1 % d.Th.) Reinprodukt als Oel.

### Beispiel 5: Herstellung von 4-[1'-(β-Naphthyl)-ethylamino]-5-methylthio-6-ethylpyrimidin

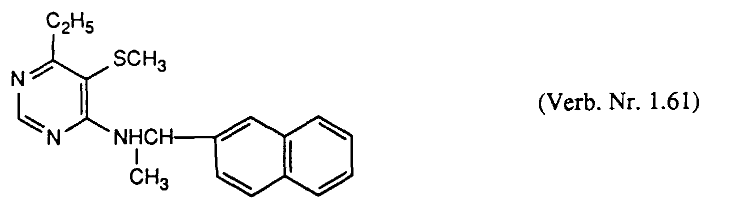

4,99 g (0,014 Mol) 4-[1'-(β-Naphthyl)-ethylamino]-5-brom-6-ethylpyrimidin werden in 18 ml N-Methylpyrrolidon gelöst und mit 3,2 g Natriummethanthiolat (CH₃SNa) 5 Stunden auf 60°C erwärmt. Man extrahiert sodann mit Wasser und Essigsäureethylester, wäscht das Extrakt und entfernt das Lösungsmittel am Rotationsverdampfer. Die chromatographische Reinigung auf Kieselgel (Laufmittel 3 Teile Essigsäureethylester und 7 Teile Hexan) ergibt neben dem Ausgangsprodukt 1,42 g Reinsubstanz als farbloses Oel. Analyse C₁₉H₂₁N₃S (M = 323,46).

| | berechnet | gefunden |
|---|---|---|
| % N | 12,99 | 12,96 |
| % S | 9,91 | 10,34 |

### Beispiel 6a: Herstellung von 4-Chlor-6-ethylpyrimidin

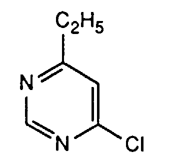

12,4 g (0,10 Mol) 4-Hydroxy-6-ethylpyrimidin, hergestellt nach EP 326 389, werden in 25 ml Phosphoroxychlorid eingetragen, wobei die Temperatur von selbst auf 70°C steigt. Es entsteht eine klare Lösung, die noch weitere 2 Stunden auf 70°C gehalten und nach dem Erkalten in Eiswasser eingerührt wird. Die Extraktion mit Ether ergibt 12,0 g flüssiges Rohprodukt, das im Kugelrohr bei 105°C und einem Druck von 26 mbar zu 11,0 g Reinsubstand destilliert wird. Analyse: C₆H₇ClN₂ (M = 142,59).

| | berechnet | gefunden |
|---|---|---|
| % C | 50,54 | 50,43 |
| % H | 4,95 | 4,89 |
| % N | 19,65 | 19,86 |
| % Cl | 24,86 | 24,79 |

### Beispiel 6b: Herstellung von 4-Hydroxy-5-brom-6-ethylpyrimidin

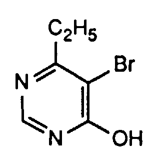

Man löst 24,8 g (0,20 Mol) 4-Hydroxy-6-ethylpyrimidin mit 16,8 g (0,20 Mol) Natriumhydrogencarbonat in 50 ml Wasser und tropft unter Kühlung langsam 32 g (0,20 Mol) Brom bei 8 bis 10°C zu. Dabei entwickelt sich Kohlensäure, und das Produkt fällt kristallin aus. Es wird durch Extraktion mit Essigsäureethylester abgetrennt und kristallisiert beim Einengen des Extrakts am Rotationsverdampfer aus. Man erhält 22,7 g (55,9 % der Theorie) einer ersten Fraktion mit Smp. 177- 179°C. Einengen der Mutterlauge ergibt weitere 3,5 g (8,6 %); Smp. 175-177°C.

### Beispiel 6c: Herstellung von 4-Chlor-5-brom-6-ethylpyrimidin

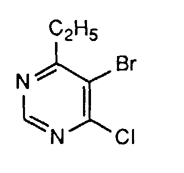

29,7 g (0,146 Mol) 4-Hydroxy-5-brom-6-ethylpyrimidin werden langsam in 27,3 g (0,178 Mol) Phosphoroxychlorid eingetragen. Die Suspension geht beim Erwärmen auf 70°C in Lösung; die Reaktion ist schwach exotherm. Man hält die Temperatur 1 1/2 Stunden auf 70°C, giesst die Lösung nach dem Erkalten auf Eiswasser und neutralisiert bei 0°C vorsichtig mit 30%iger Natronlauge auf pH 5-7. Die Extraktion mit Essigsäureethylester ergibt nach der Entfernung des Lösungsmittels 30,4 g Rohprodukt. Nach der Destillation erhält man 23,9 g (73,9 % der Theorie) Reinsubstanz vom Sdp. 112-114°C/24 mbar.

### Beispiel 6d: Herstellung von 4-Hydroxy-5-jod-6-ethylpyrimidin

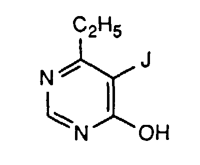

Man gibt 50,8 g Jod (0,20 Mol) in 850 ml Essigsäure und leitet bei 30-30°C 14,8 g (0,21 Mol) Chlor ein, wobei sich das Jod auflöst. Danach werden bei 20°C 49,7 g (0,4 Mol) 4-Hydroxy-6-ethylpyrimidin gelöst in 150 ml Essigsäure zugetropft, wobei sich das kristalline Endprodukt abscheidet Absaugen und Umkristallisation aus Essigsäureethylester erbibt 24,4 g Reinsaubstanz, Smp. 191-192°C.

### Beispiel 6e: Herstellung von 4-Chlor-5-jod-6-ethylpyrimidin

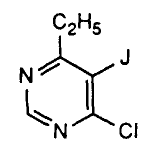

21,7 g (0,087 Mol) 4-Hydroxy-5-jod-6-ethylpyrimidin werden in 50 ml auf 60°C erwärmtes Phosphoroxychlorid eingerührt, wobei die leicht exotherme Reaktion abläuft. Man hält die Temperatur stets auf 60°C und lässt 1 Stunde lang ausreagieren. Danach wird auf Eiswasser gegossen und zur vollständigen Hydrolyse des überschüssigen Phoshoroxychlorids 30 Minuten gerührt. Die Extraktion mit Essigsäureethylester ergibt 23,5 g kristallines Rohprodukt. Die Umkristallisation aus einem Gemisch aus 1 Teil Essigsäureethylester und 10 Teilen Hexan ergibt 18,8 g (80,7 % der Theorie) Reinsubstanz; Smp. 56-57°C.

### Beispiel 6f: Herstellung von 4,5-Dichlor-6-isopropylpyrimidin

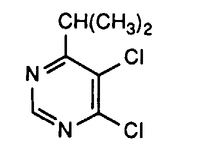

In eine Lösung von Diisopropylamin (4,4 ml) in Tetrahydrofuran (100 ml) wird unter Stickstoffatmosphäre bei 0°C n-Butyllithium (1,6 m Lösung in Hexan, 19,4 ml) tropfenweise zugegeben und 20 Minuten gerührt. Diese Lösung wird auf -78°C gekühlt und anschliessend werden 5 g 4,5-Dichlor-6-äthylpyrimidin, gelöst in 10 ml Tetrahydrofuran, zugetropft. Dieses Gemisch wird bei -78°C eine Stunde weitergerührt und anschliessend über 2 Stunden auf Raumtemperatur gebracht Nach Zugabe von Wasser (200 ml) wird mit Essigsäureäthylester extrahiert, die organische Phase über Natriumsulfat getrocknet und eingeengt. Nach Chromatographie an Kieselgel (Laufmittel Essigsäureäthylester/Hexan 1:10) erhält man 3,8 g (71 % der Theorie) 4,5-Dichlor-6-isopropylpyrimidin als gelbes Oel.
H-NMR: 8.79 (1H, s), 3,58 (1H,septett), 1.3 (6H, d)

Auf diese Art oder nach einer der weiter oben angegebenen Methoden lassen sich folgende Verbindungen herstellen.

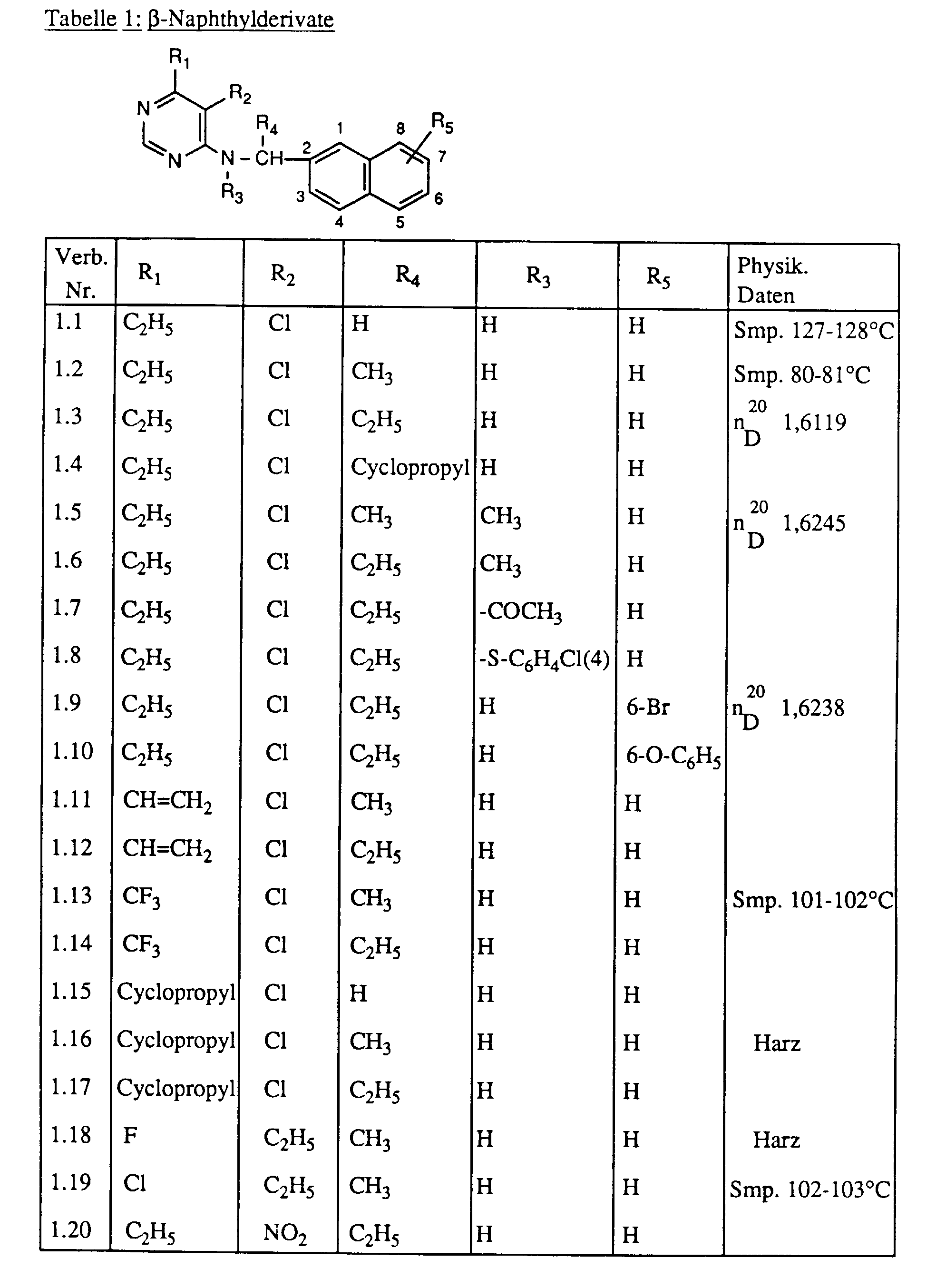

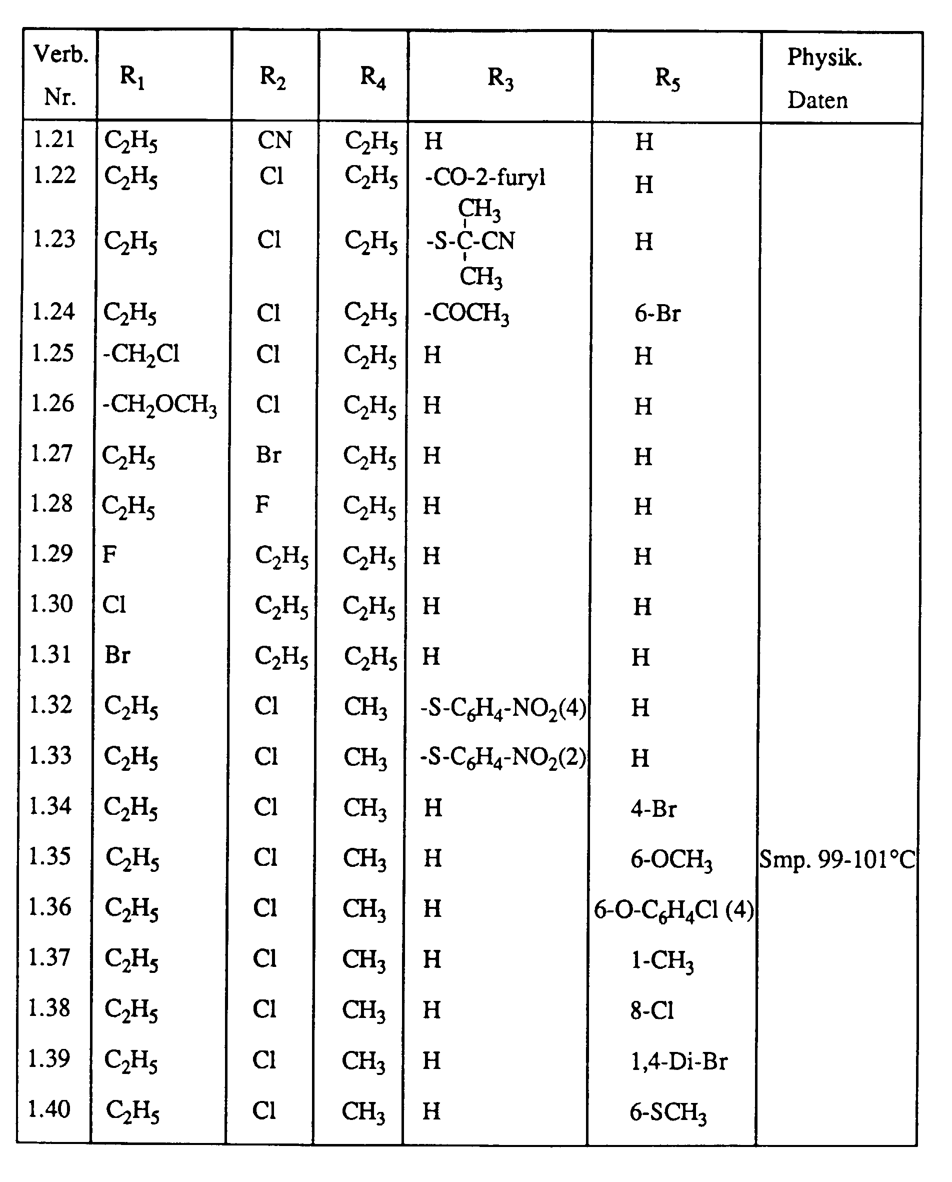

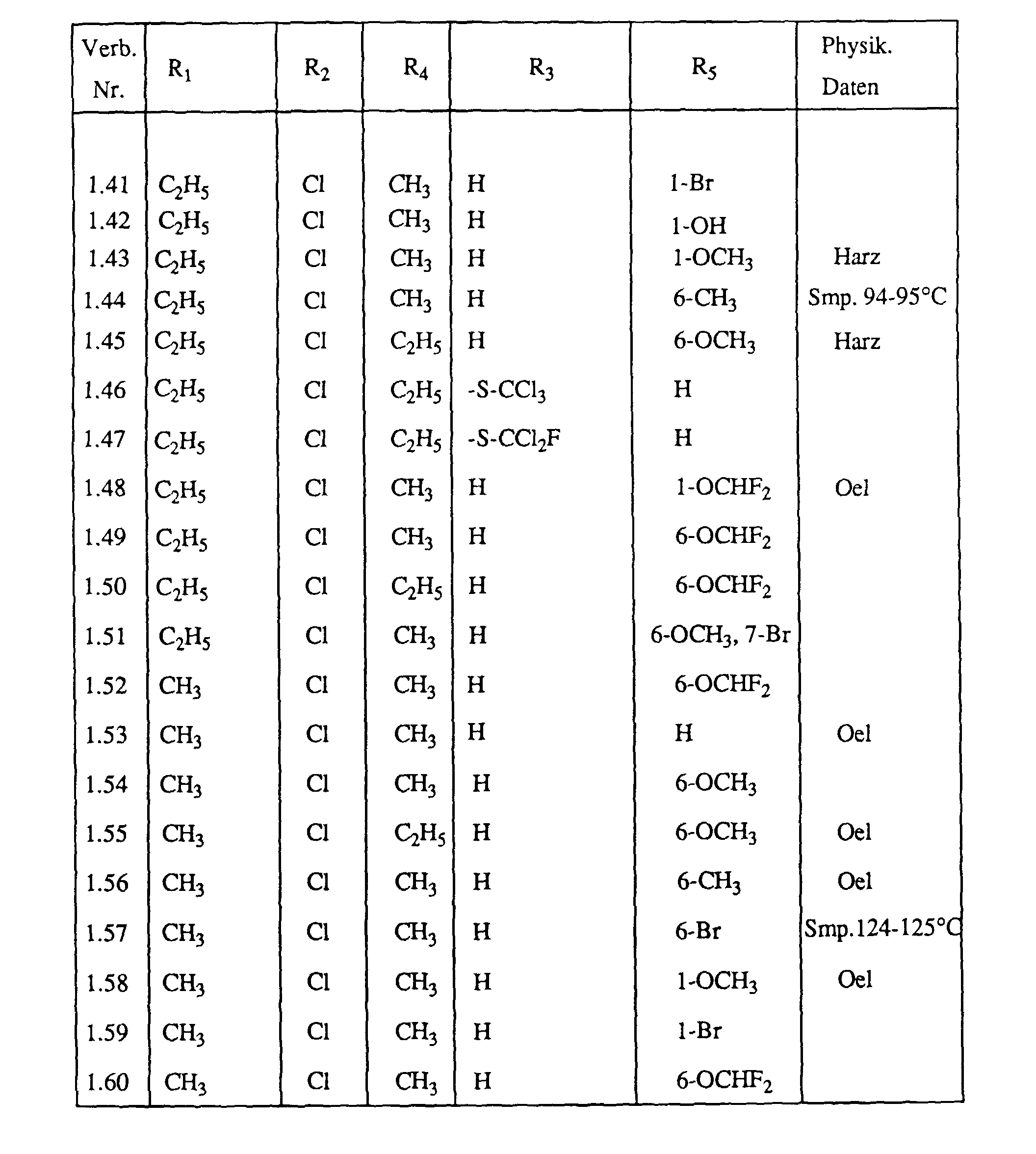

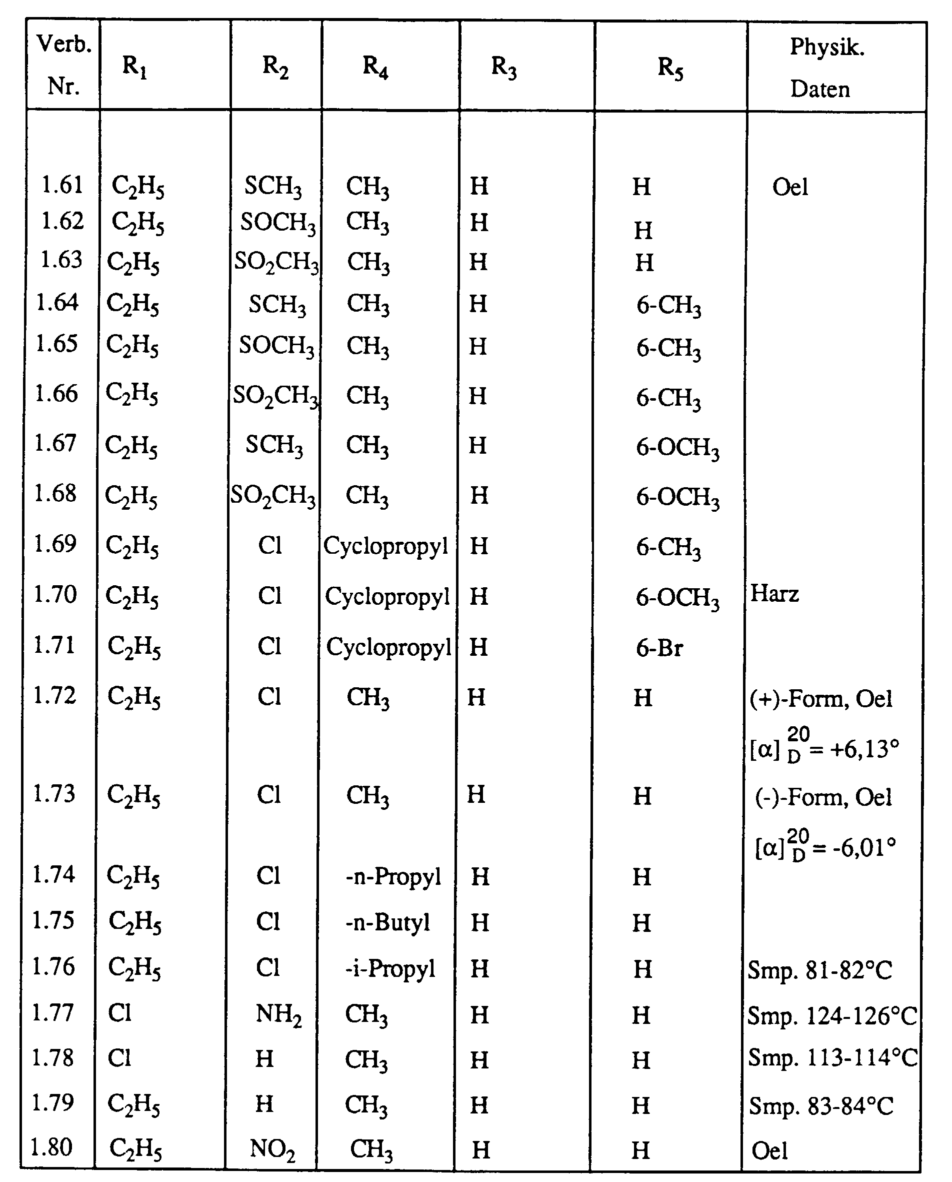

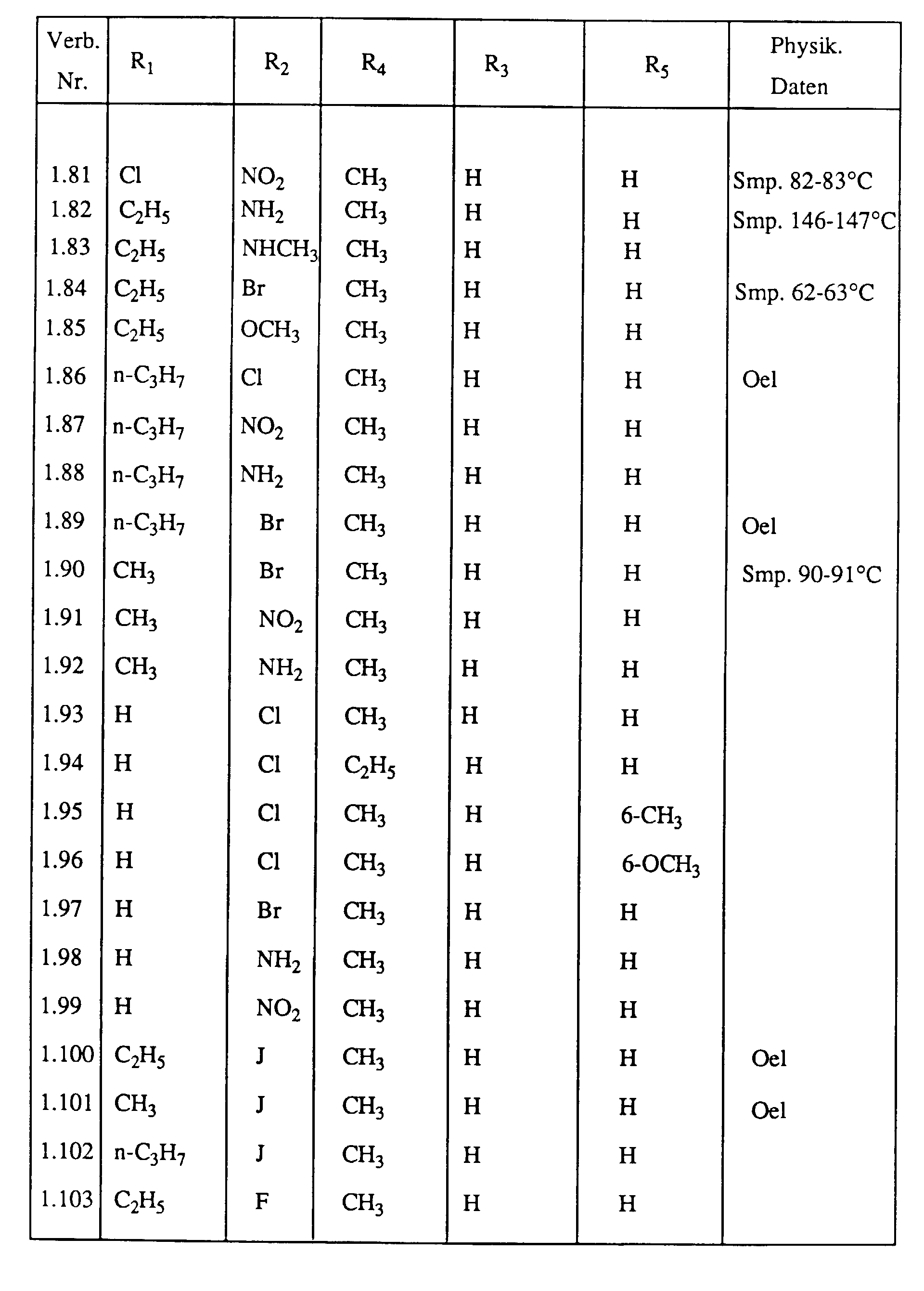

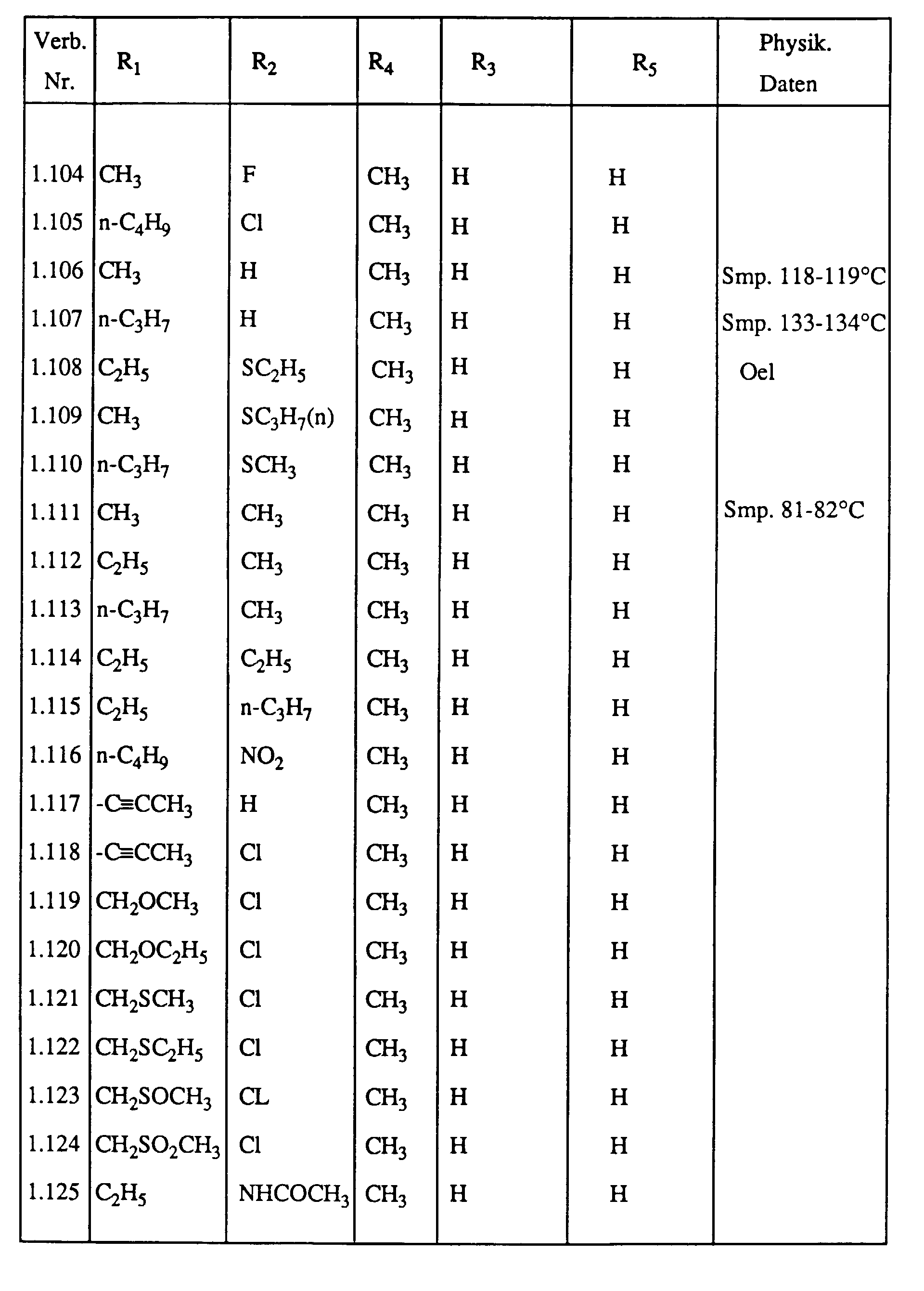

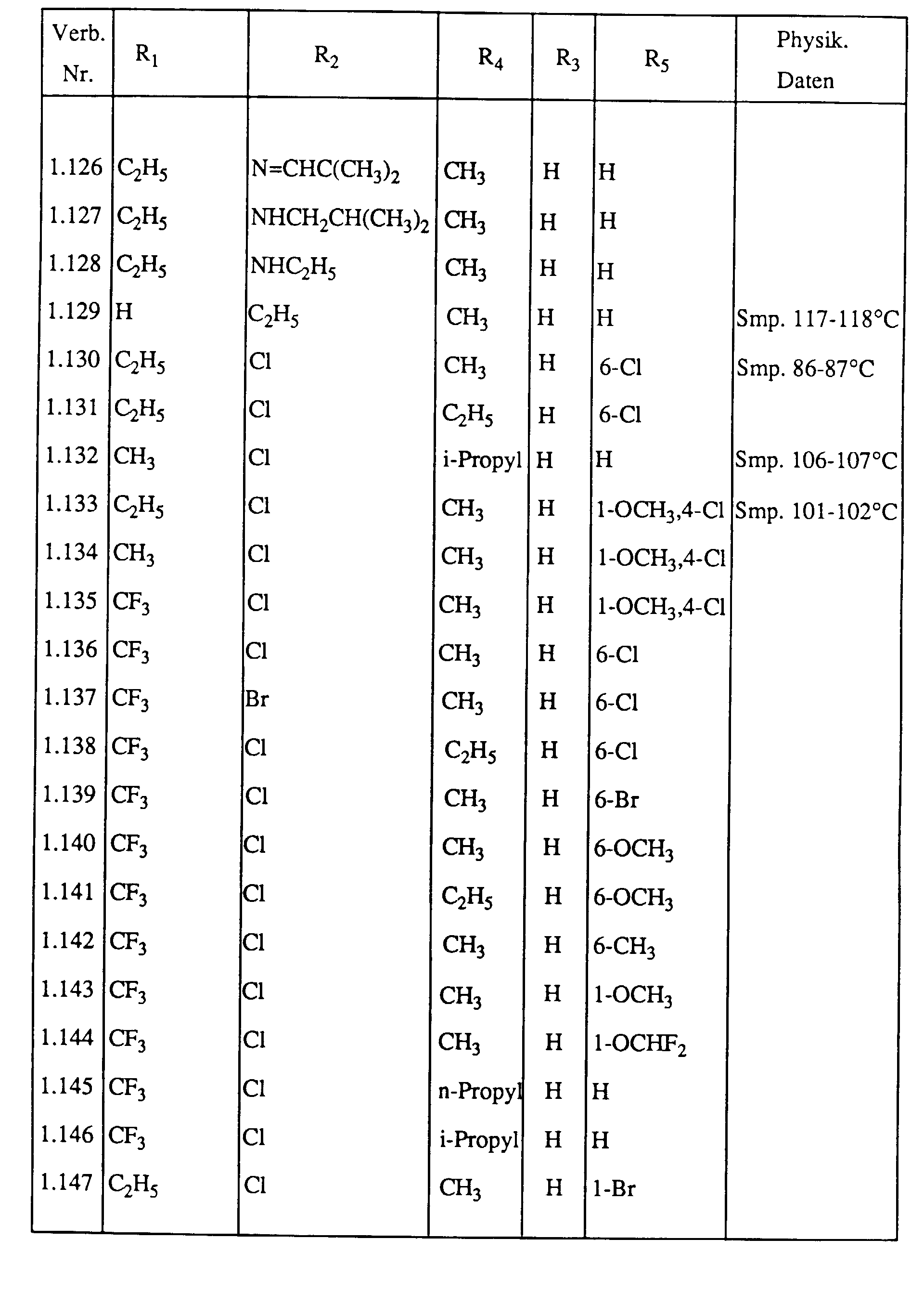

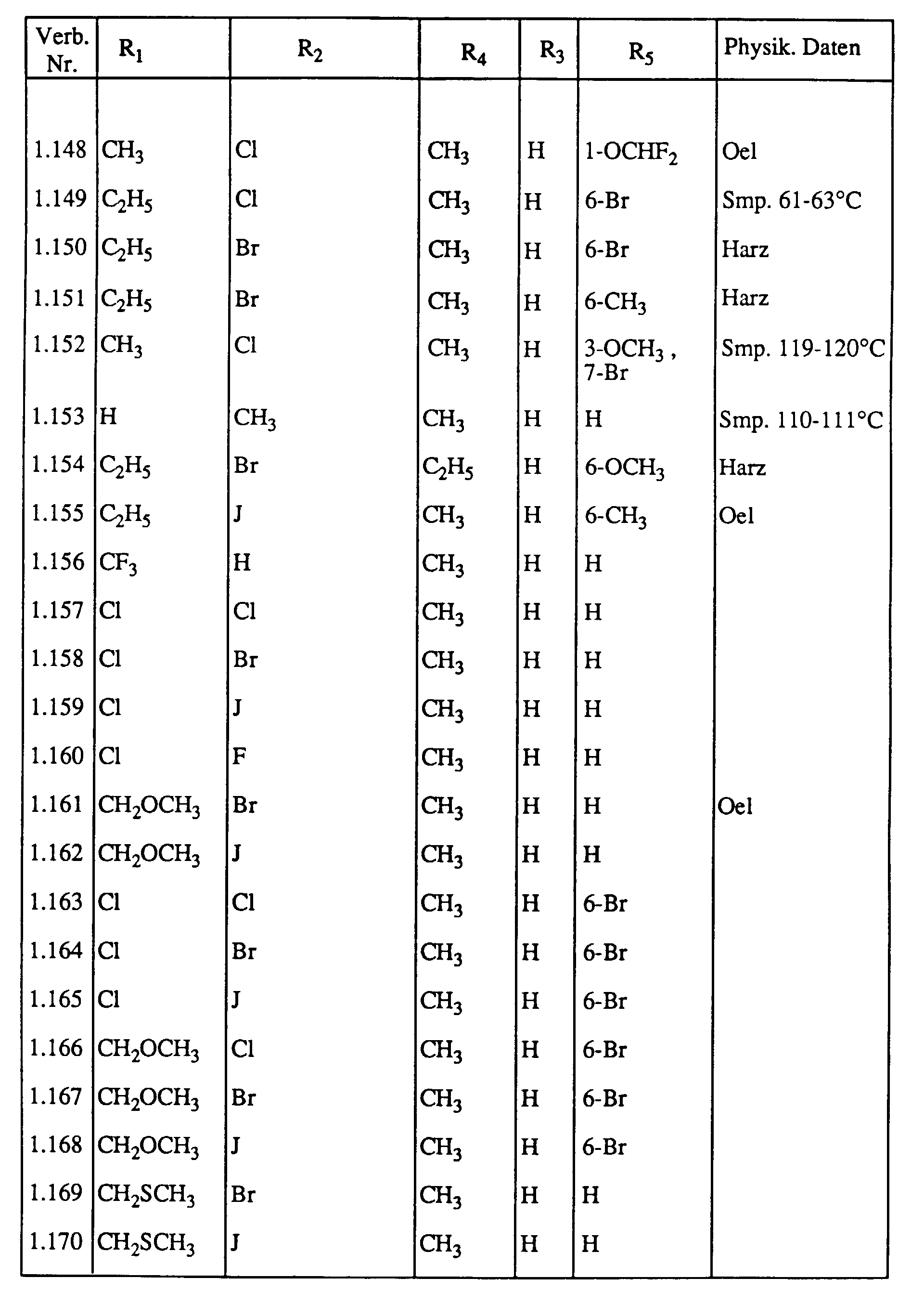

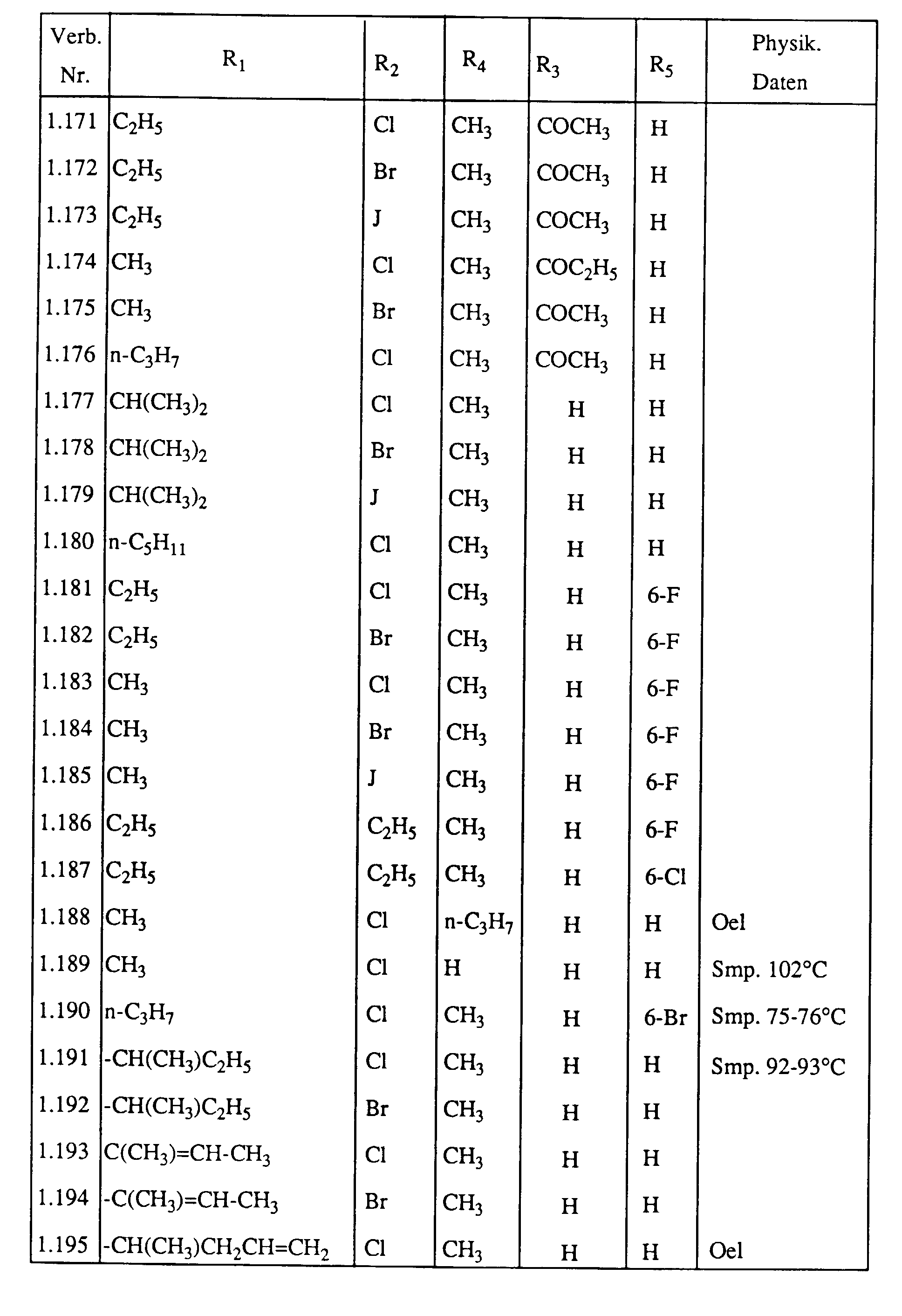

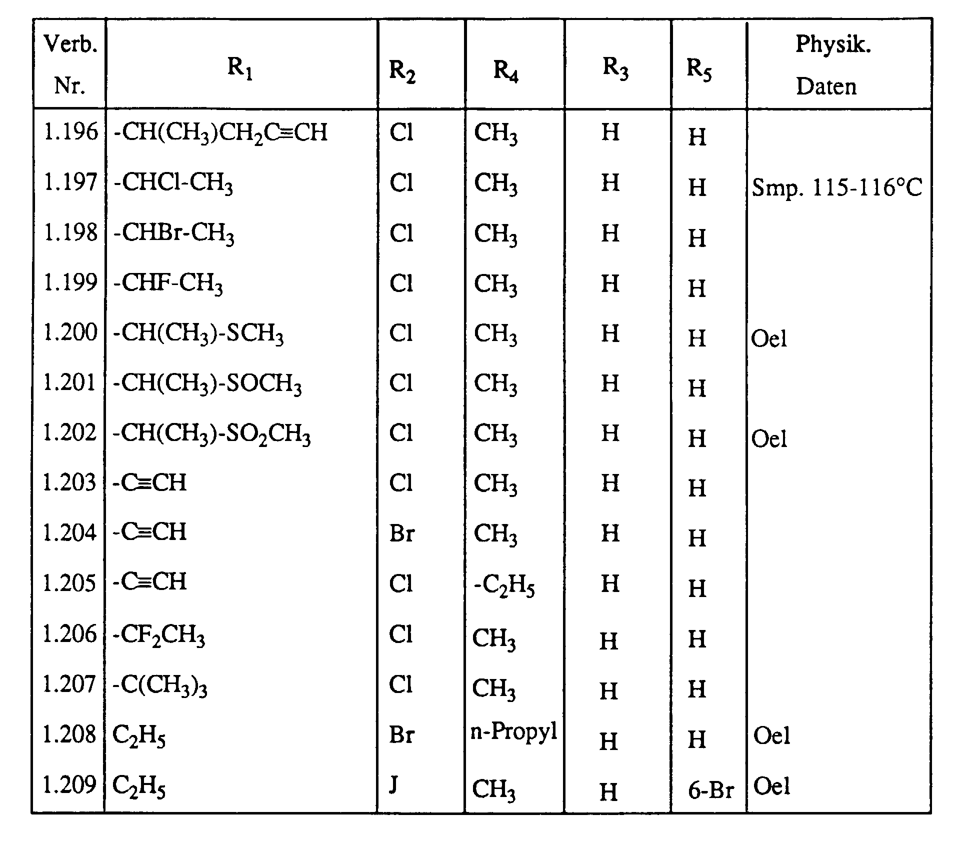

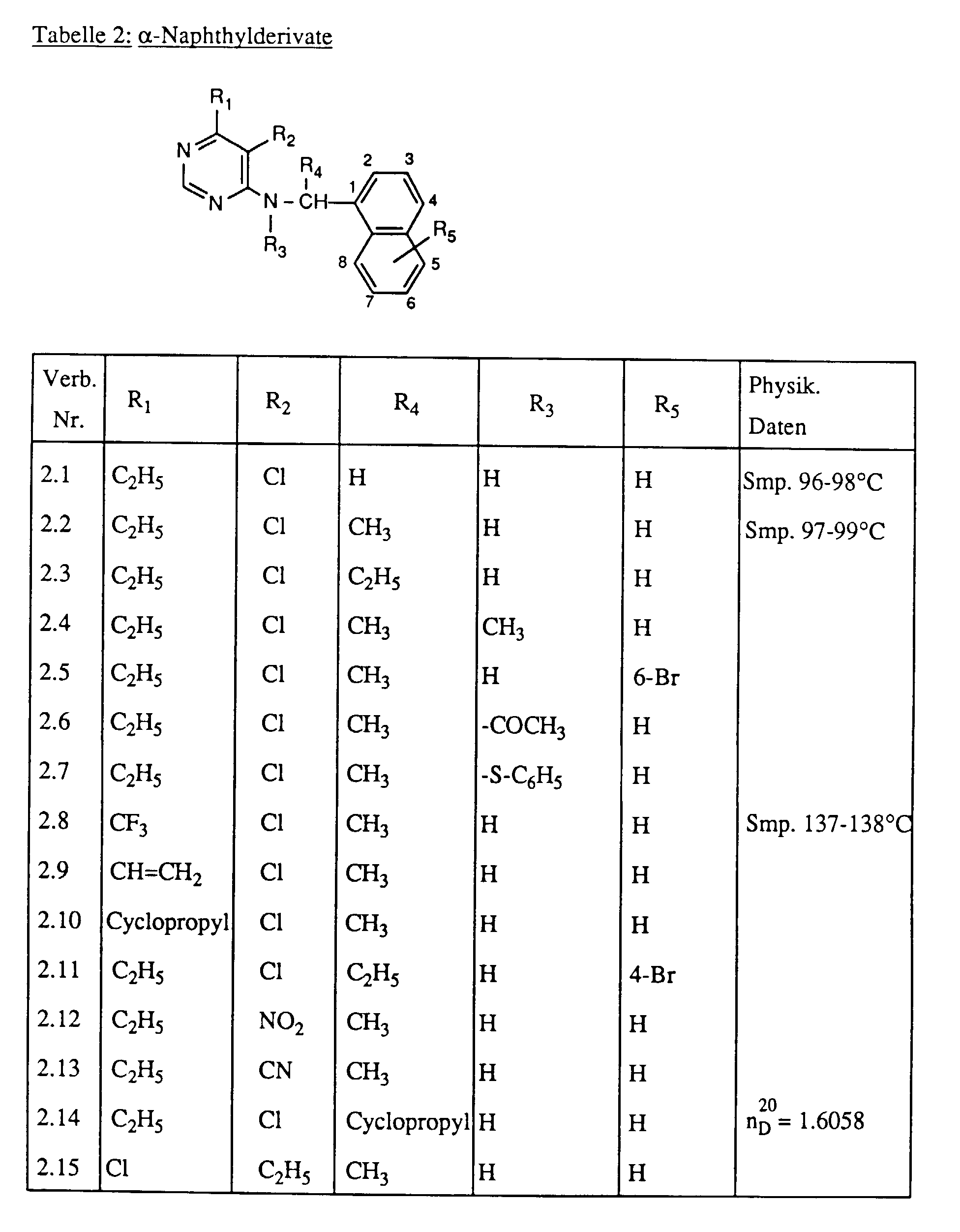

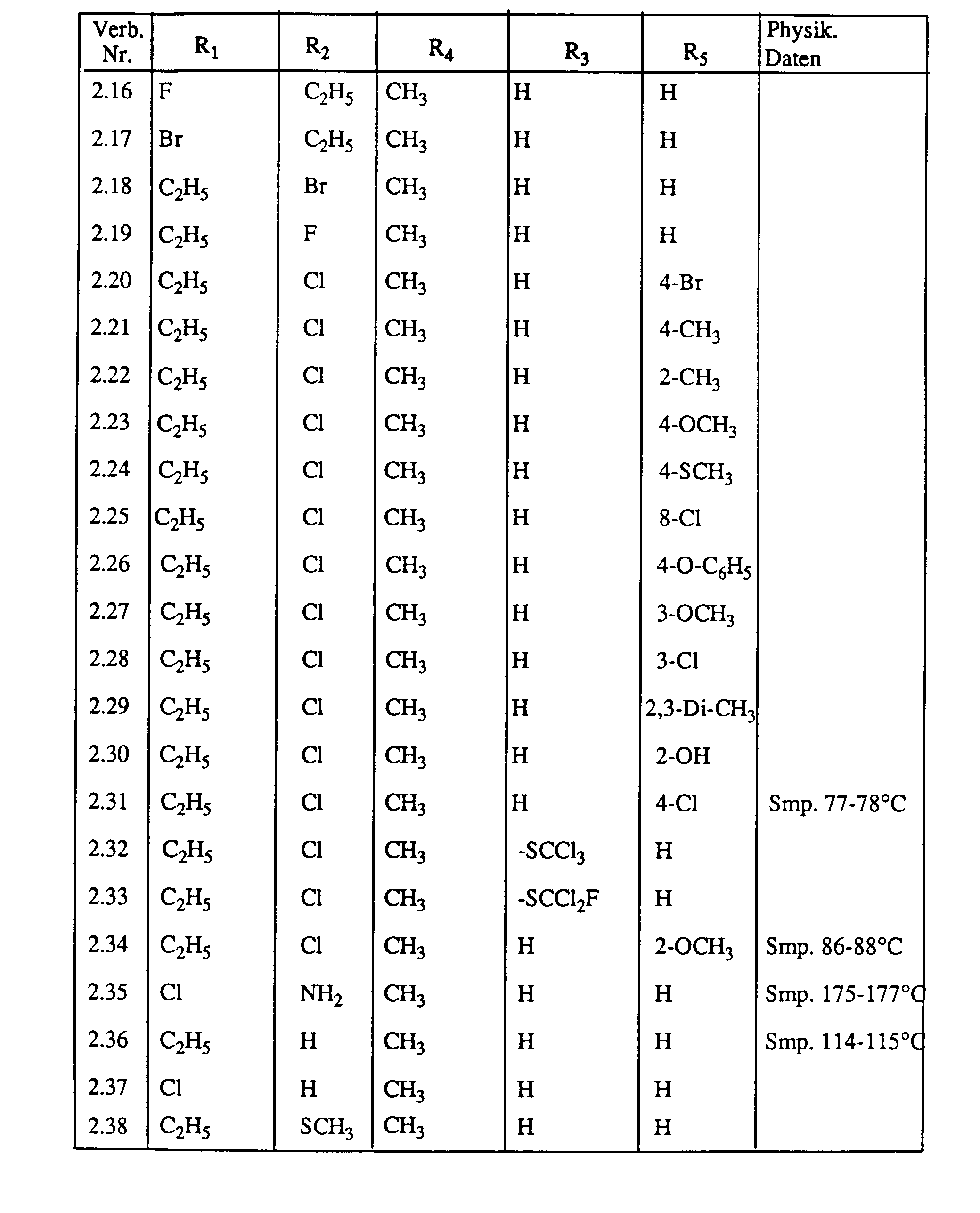

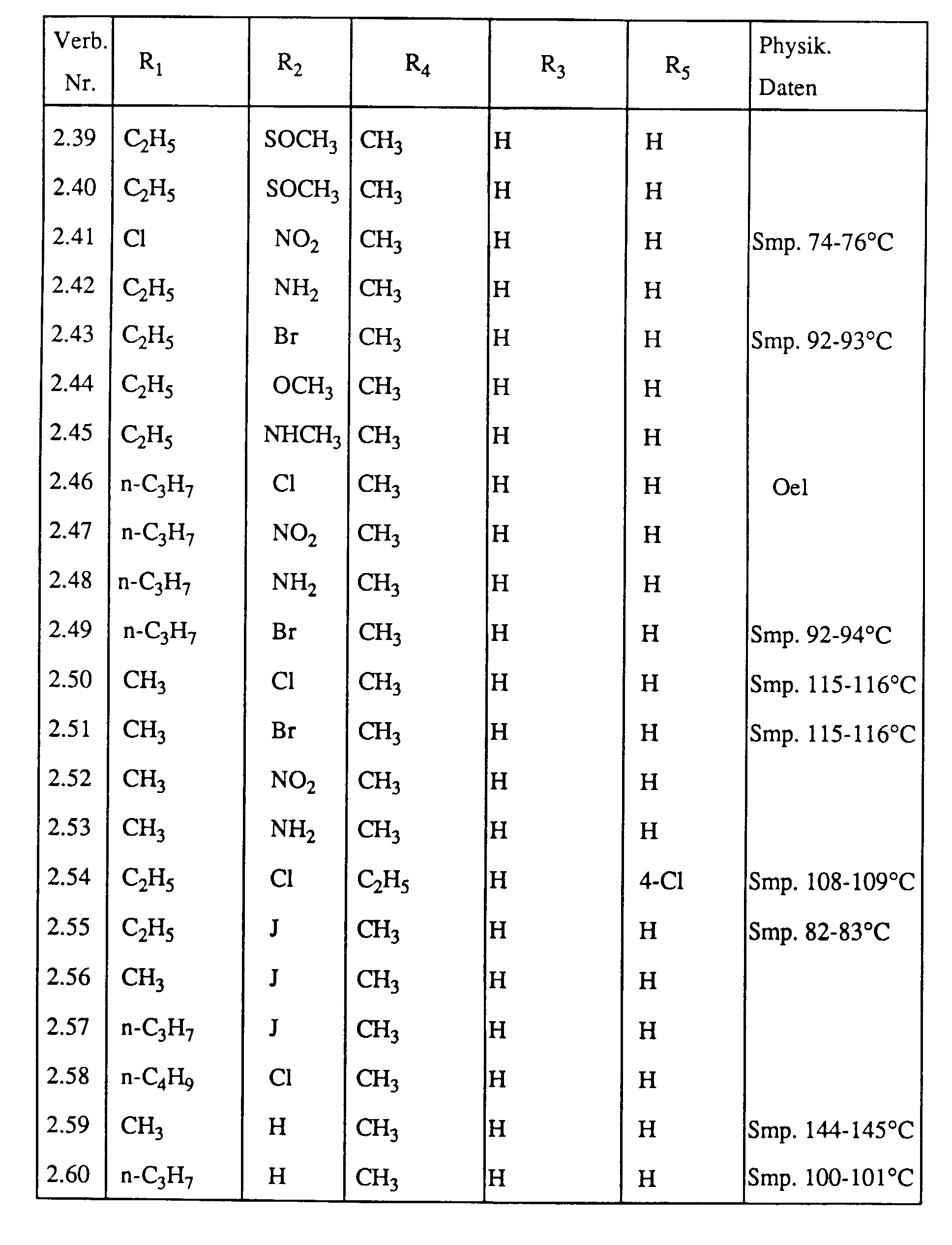

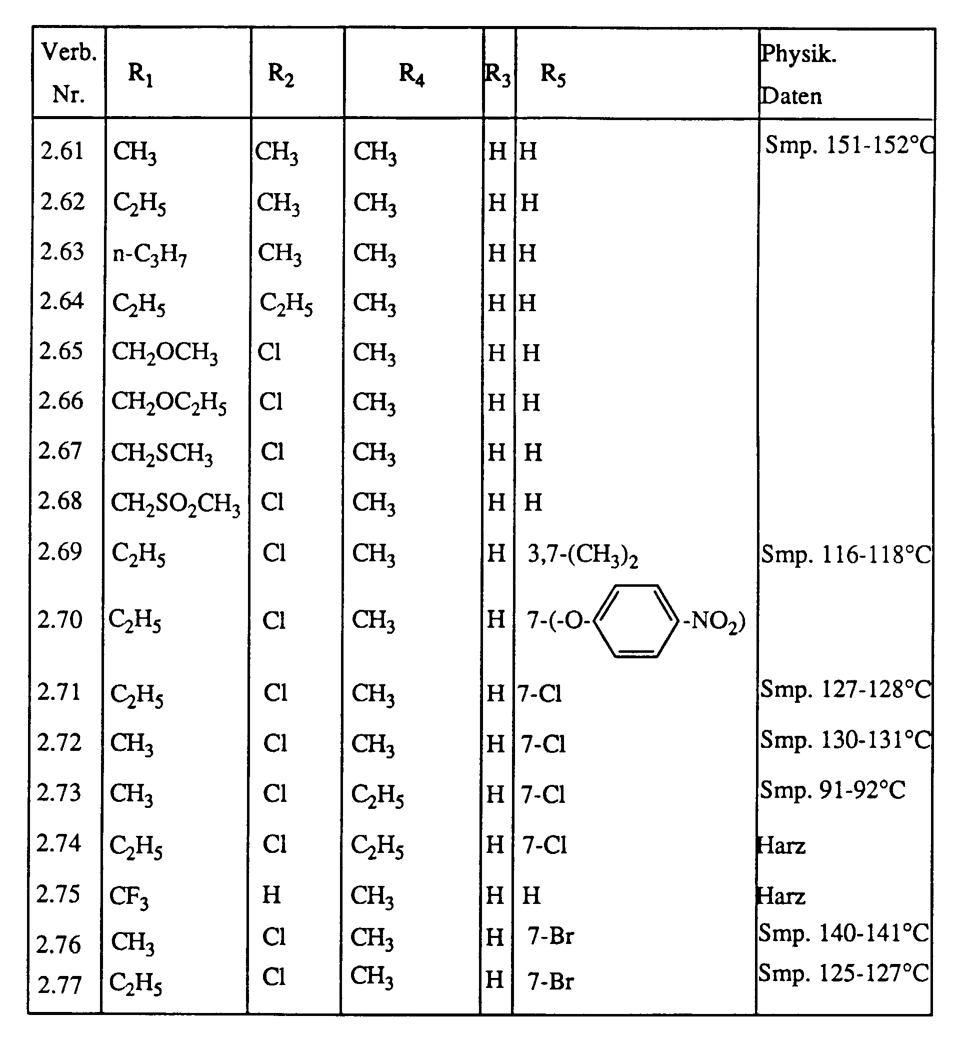

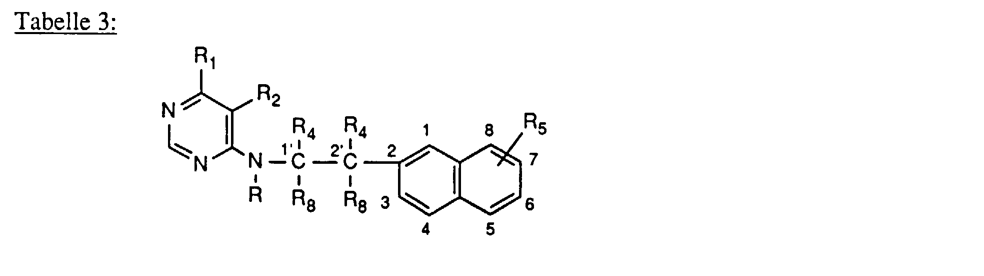

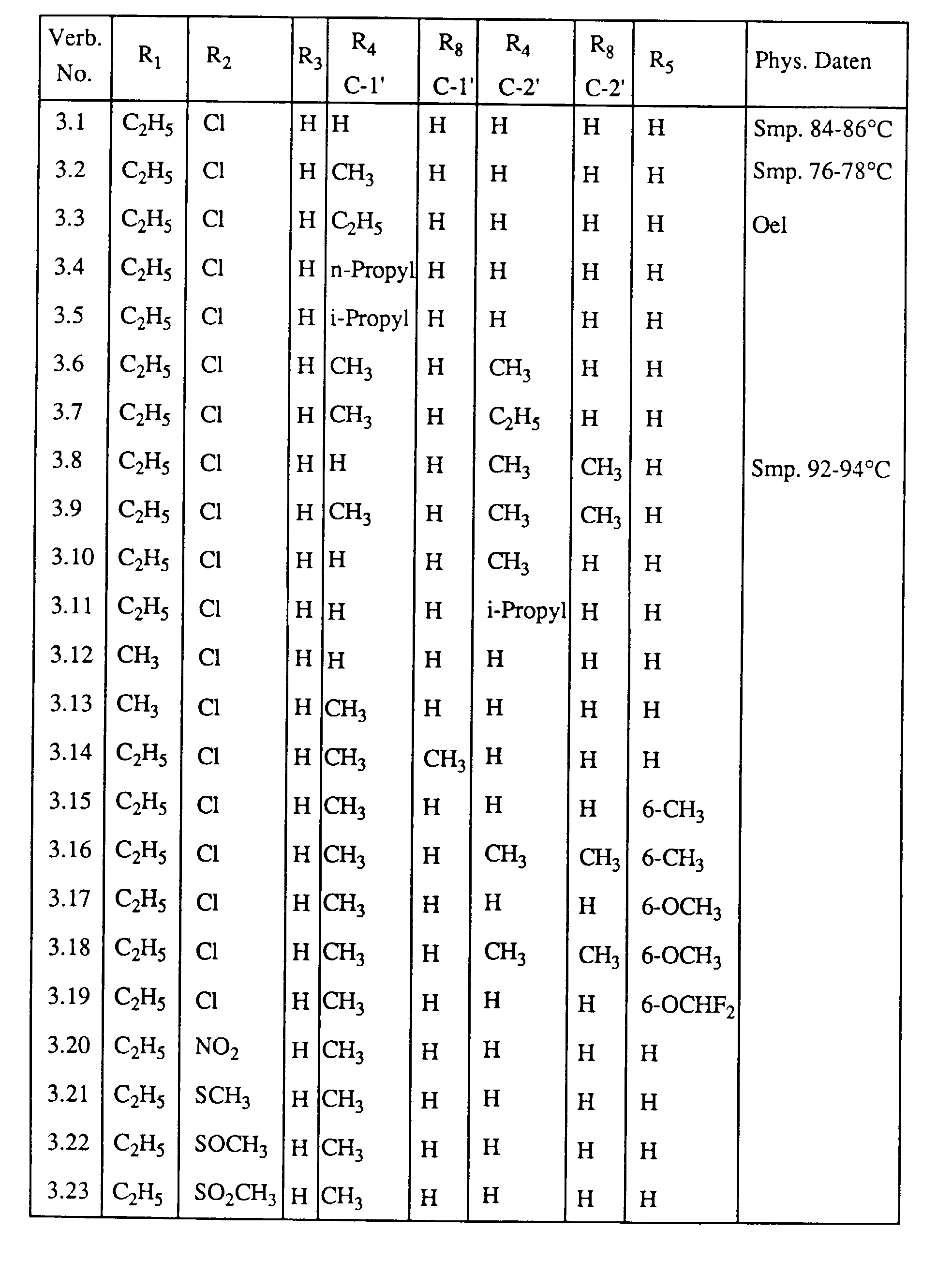

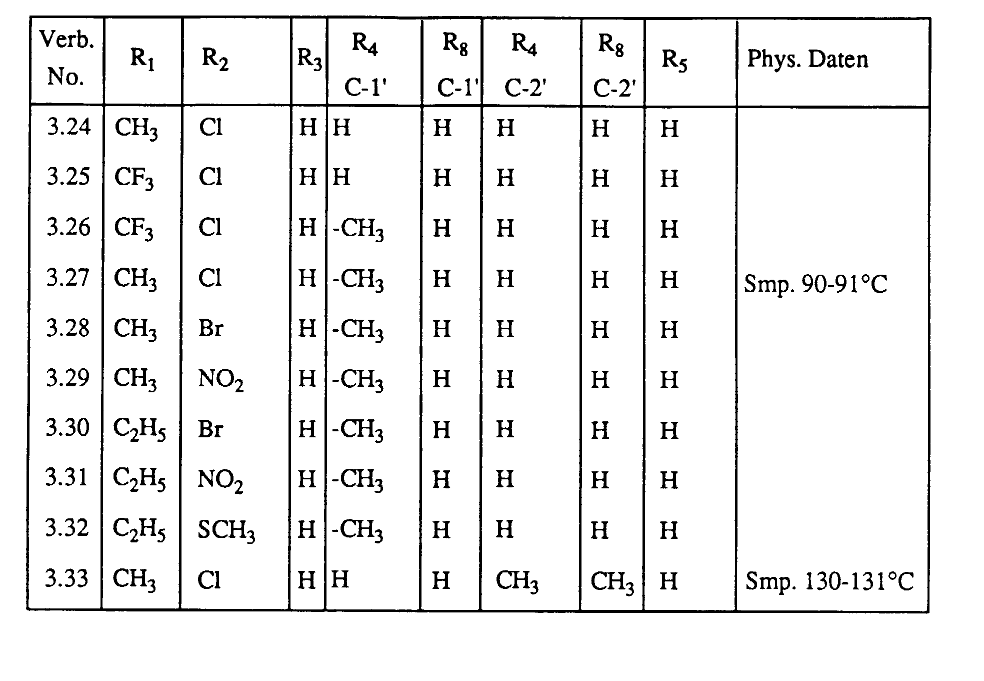

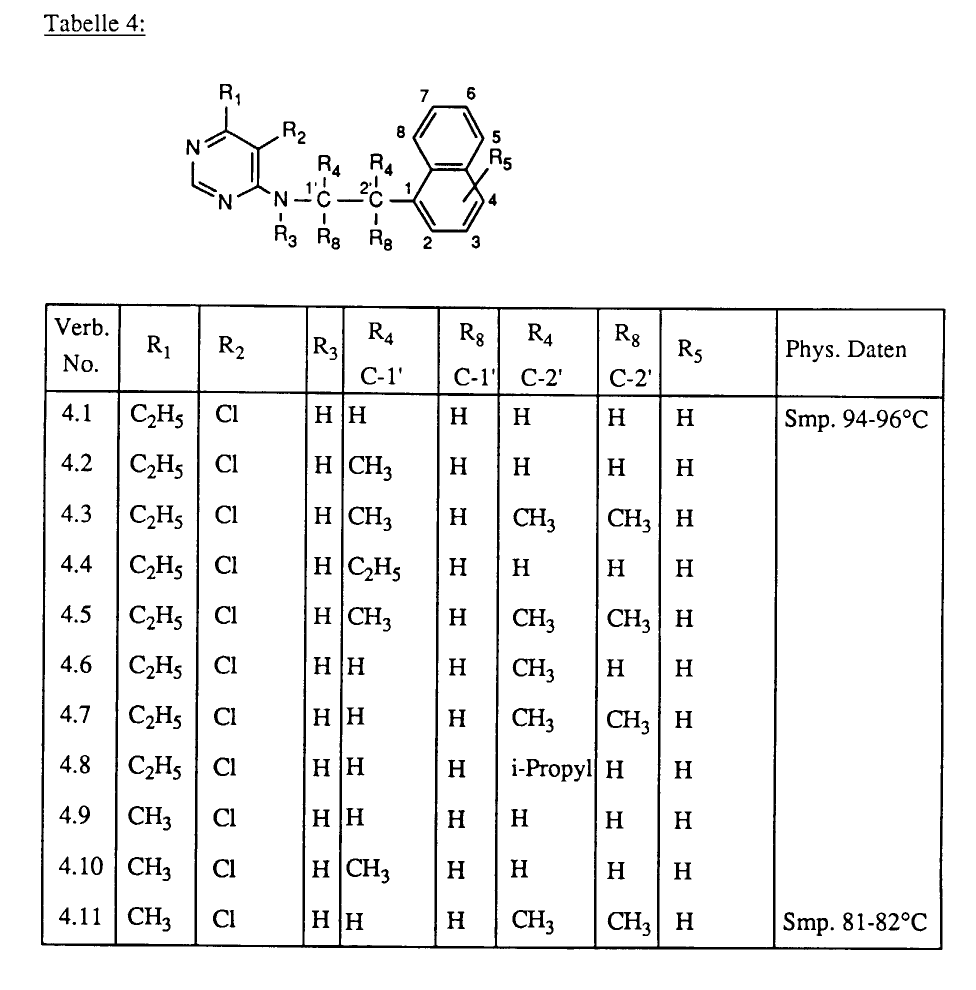

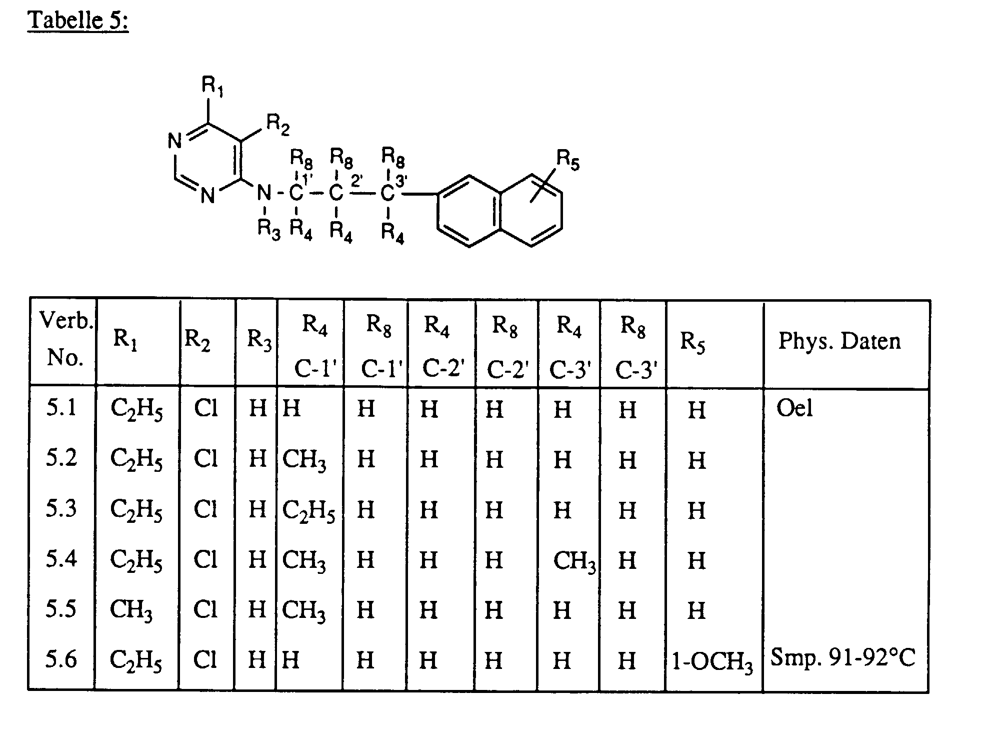

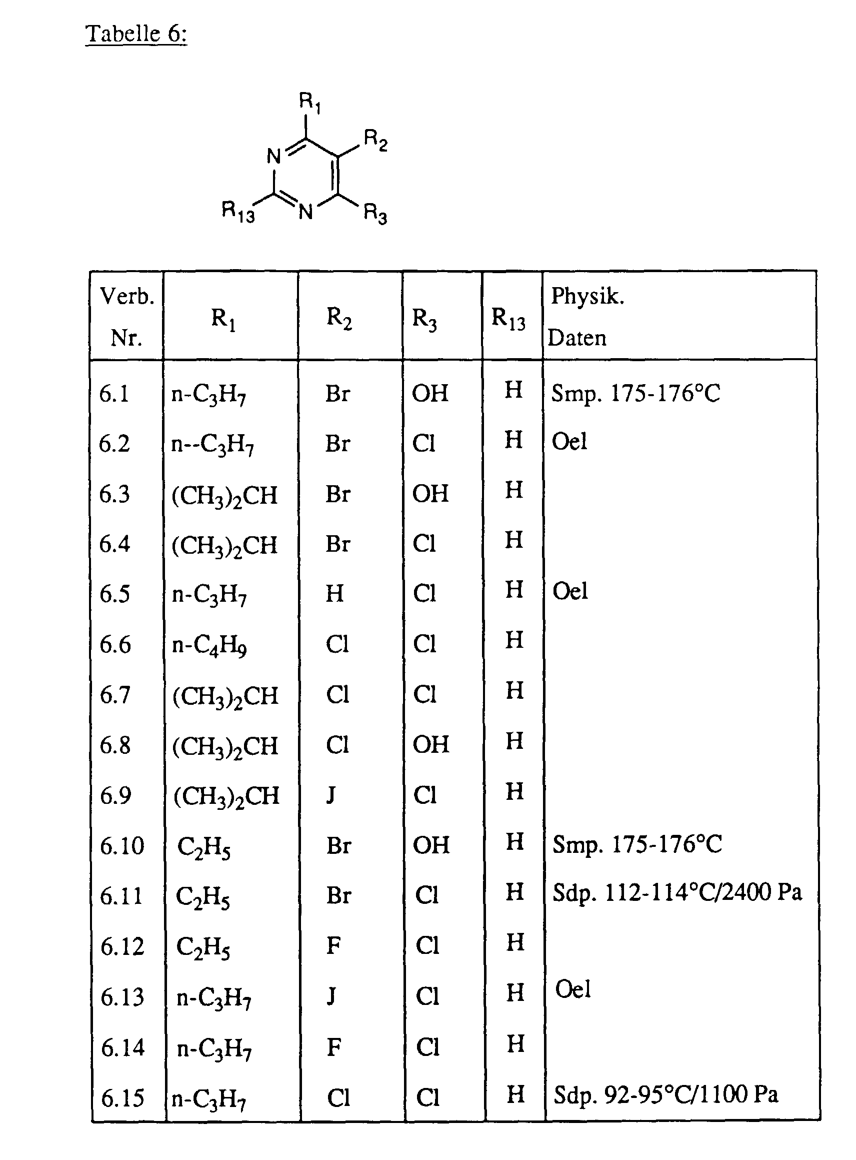

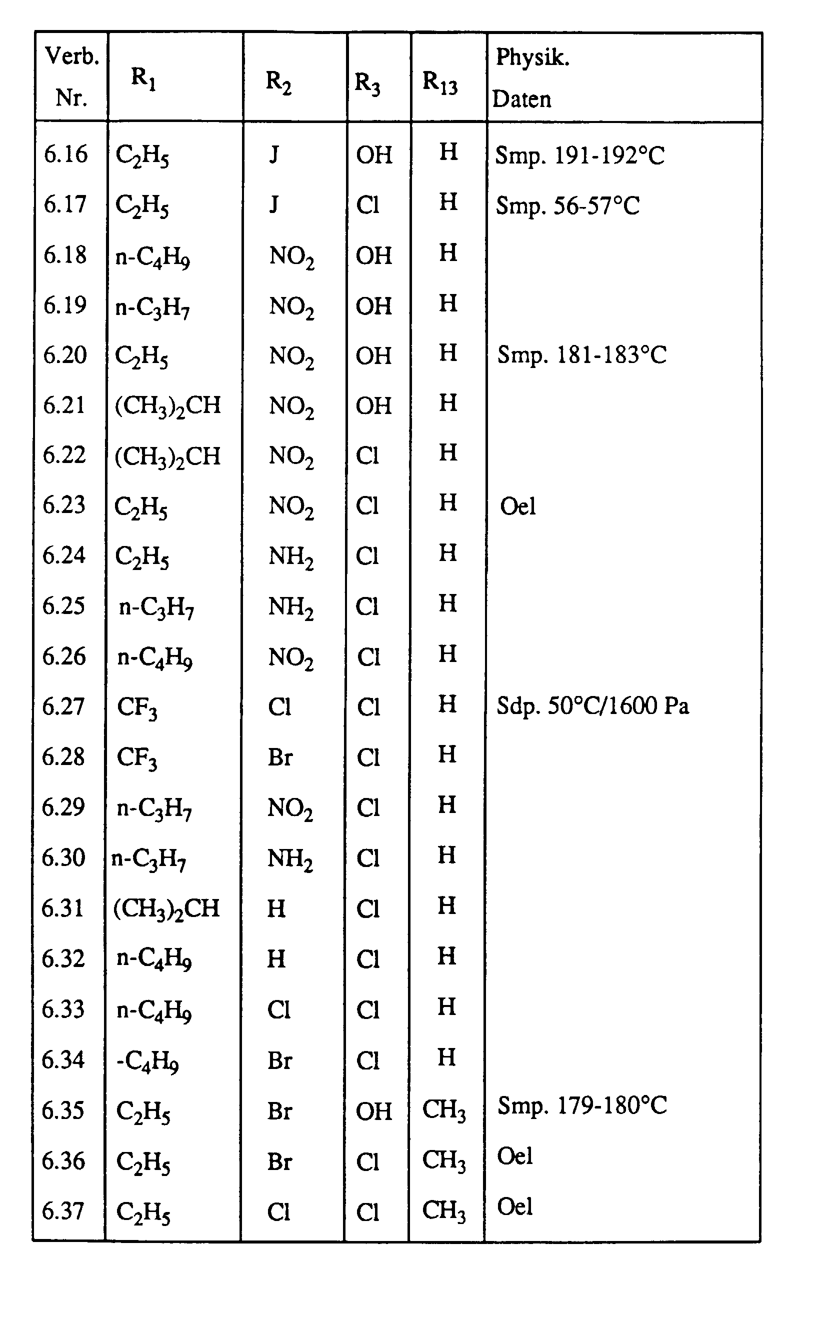

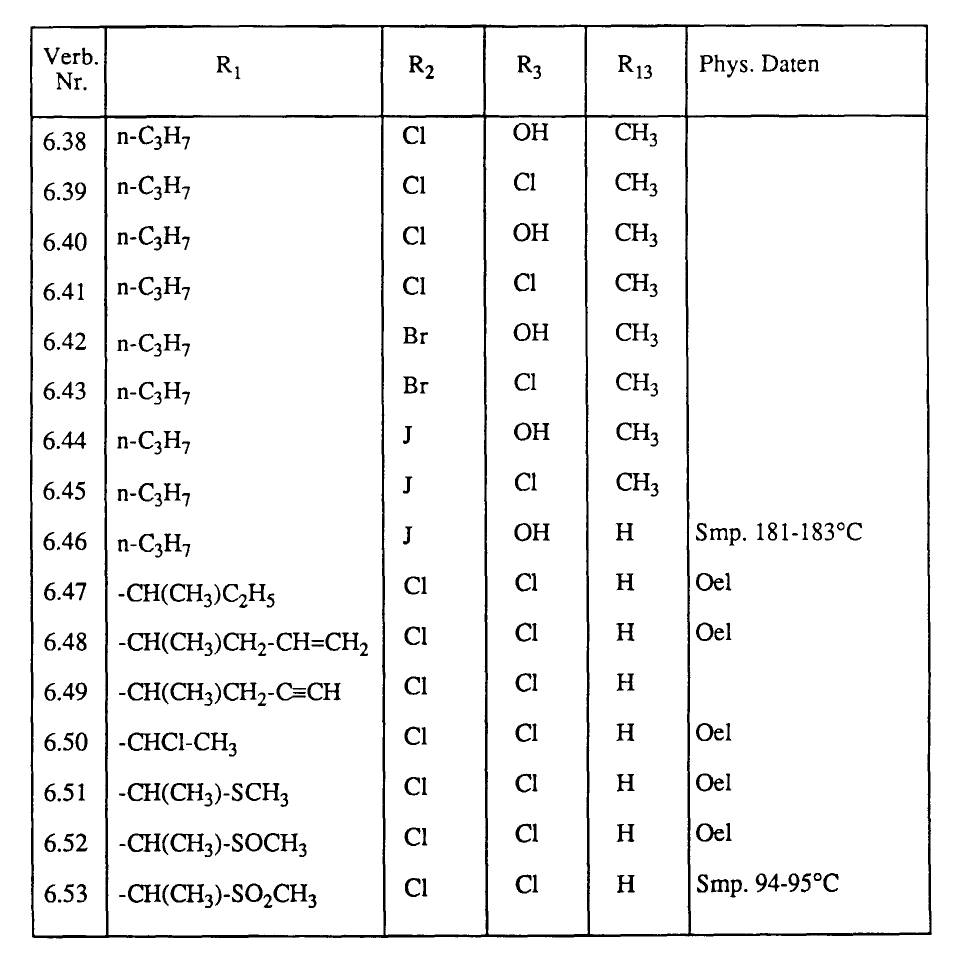

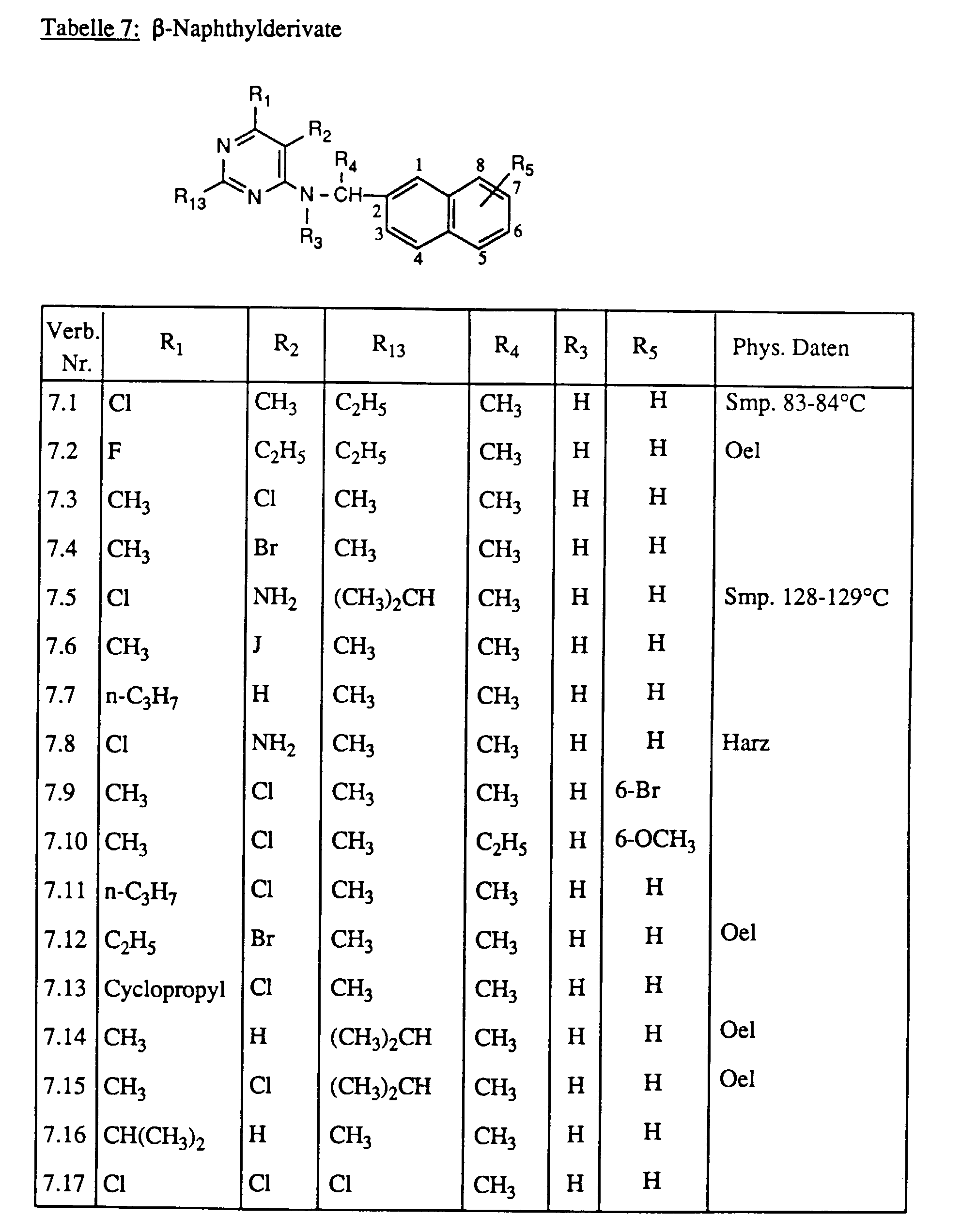

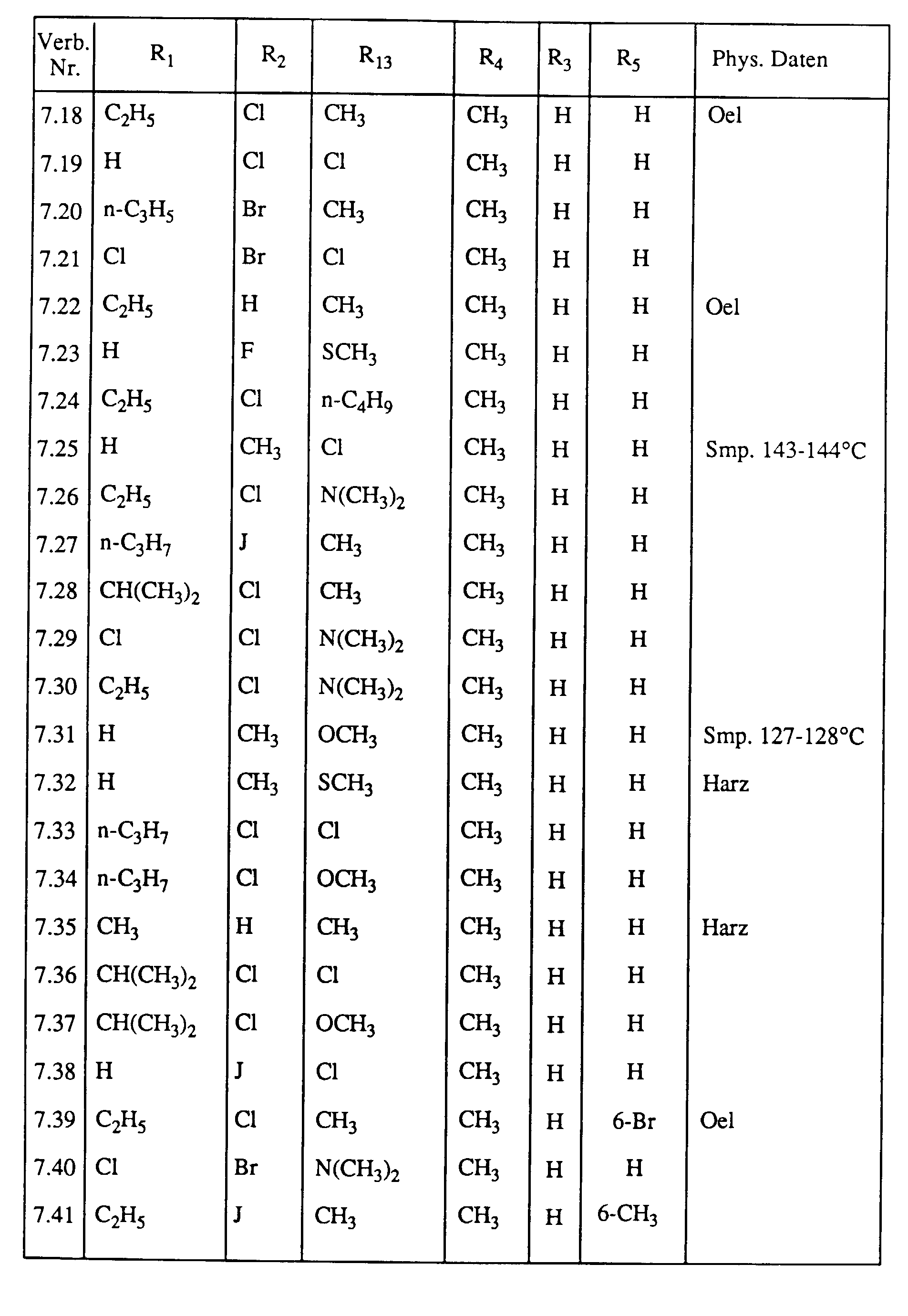

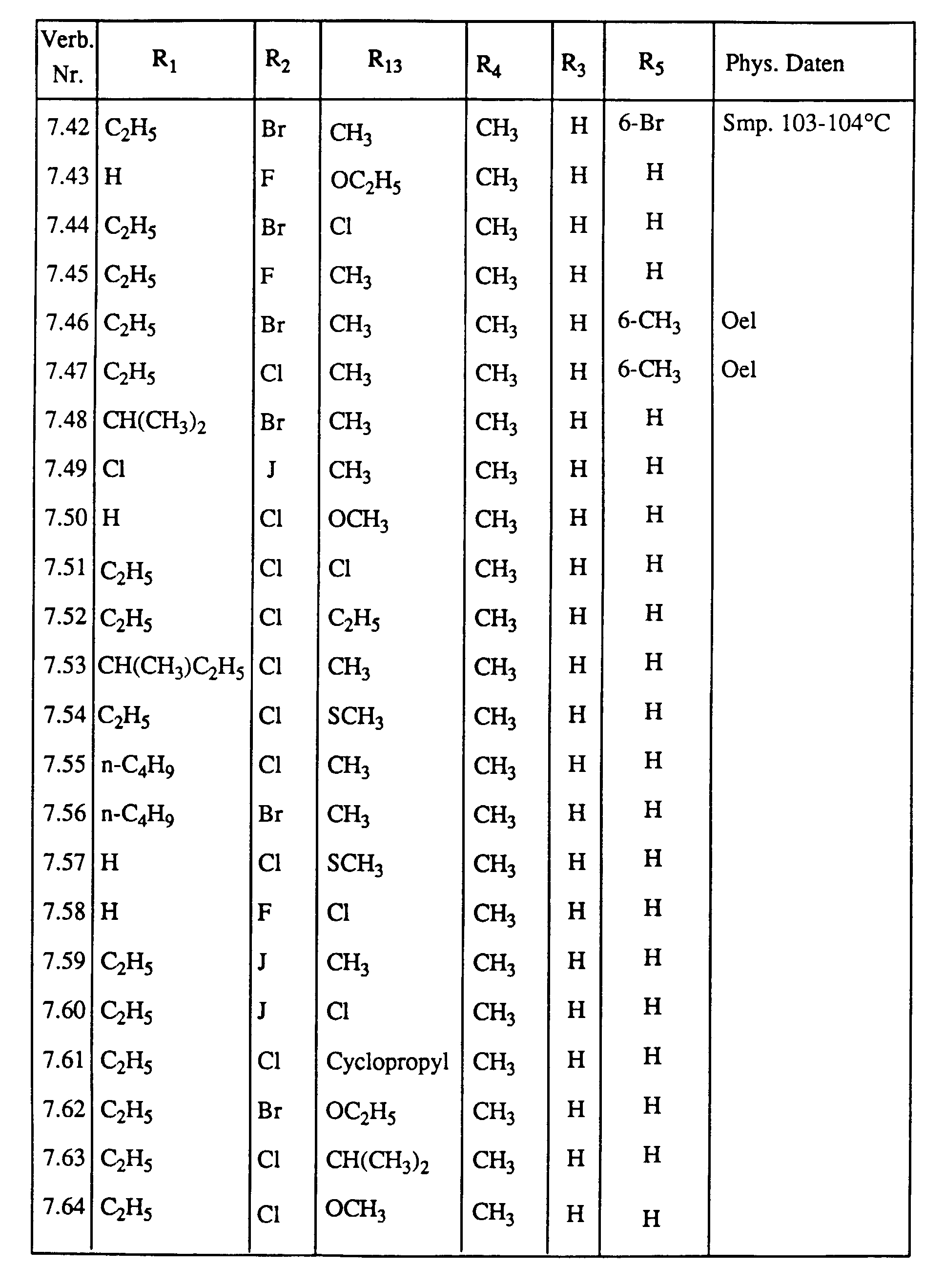

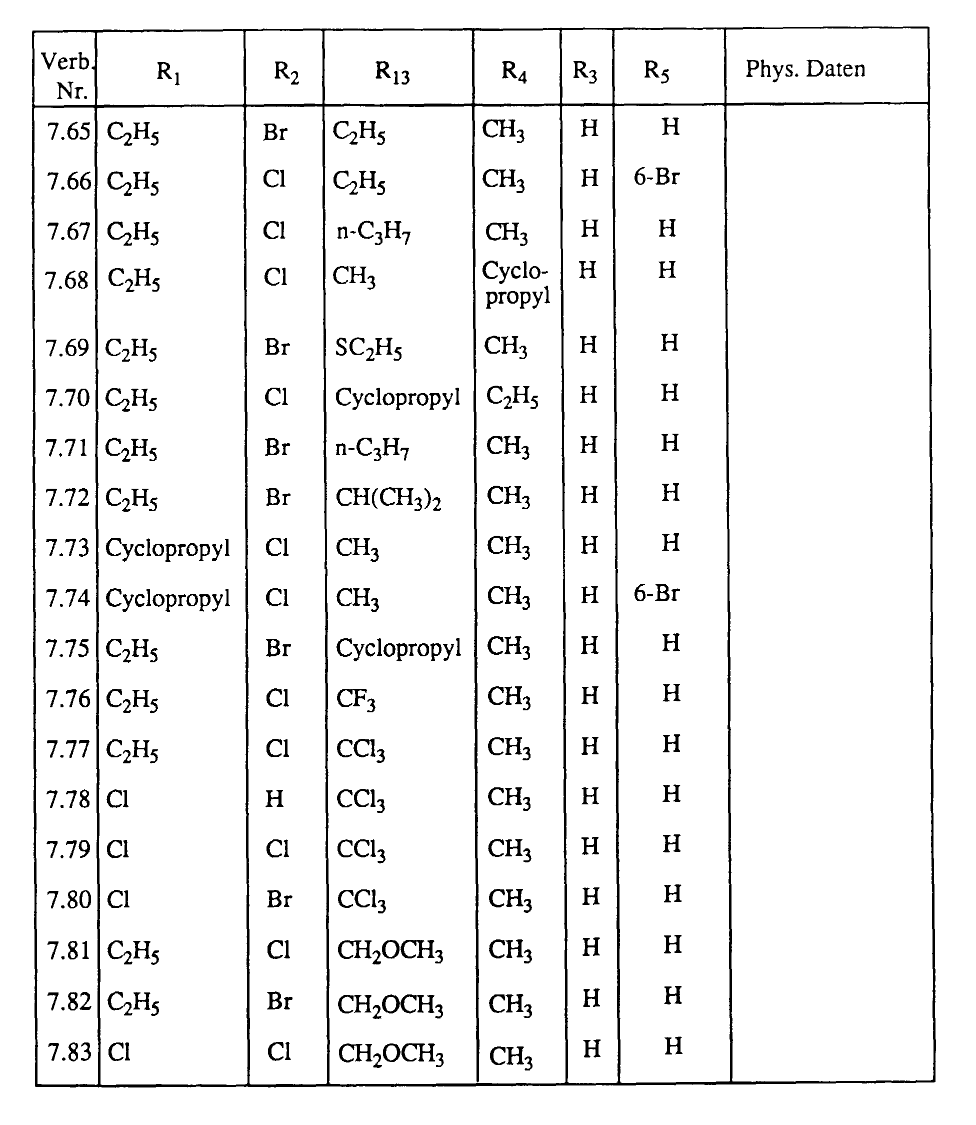

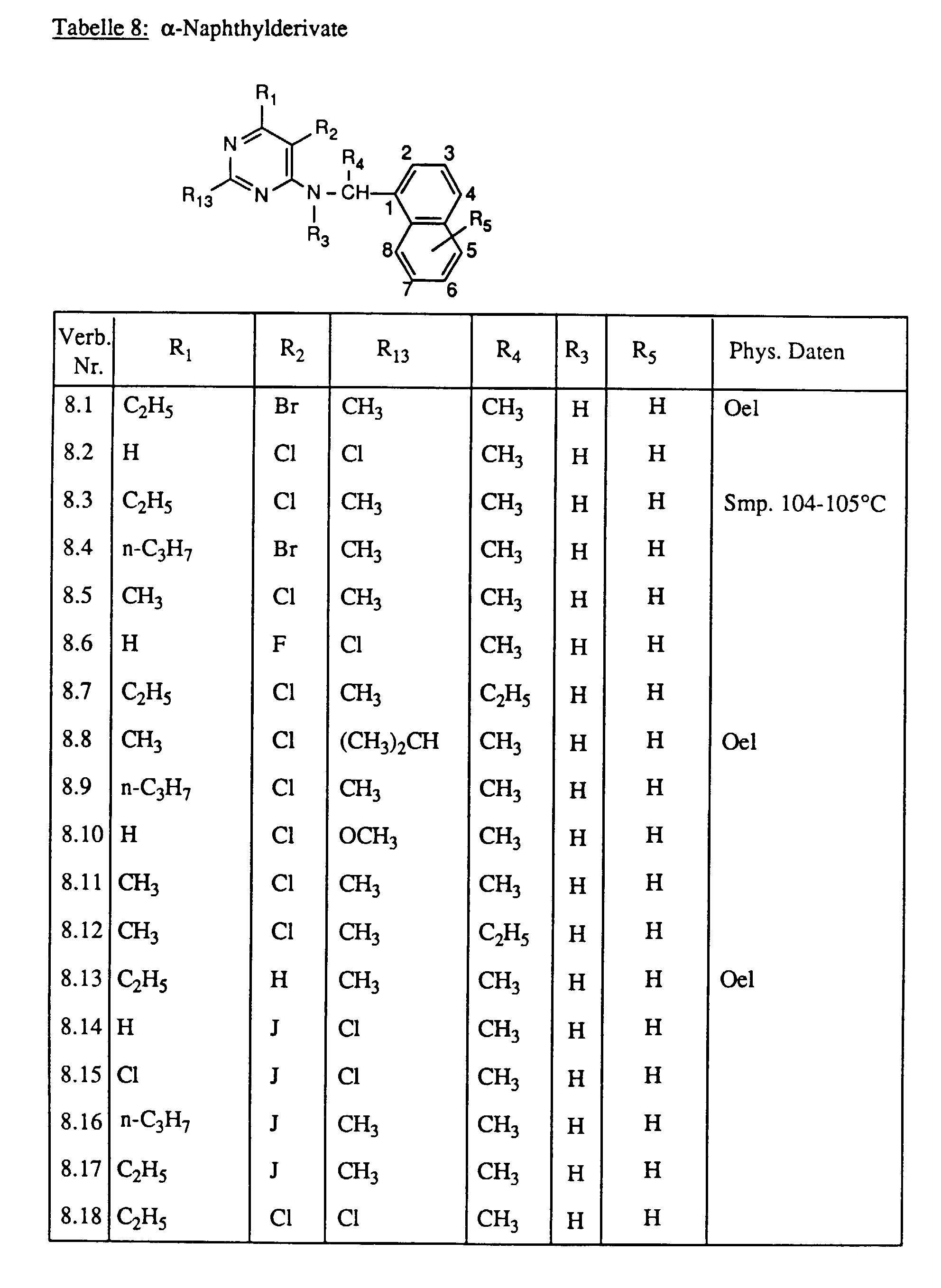

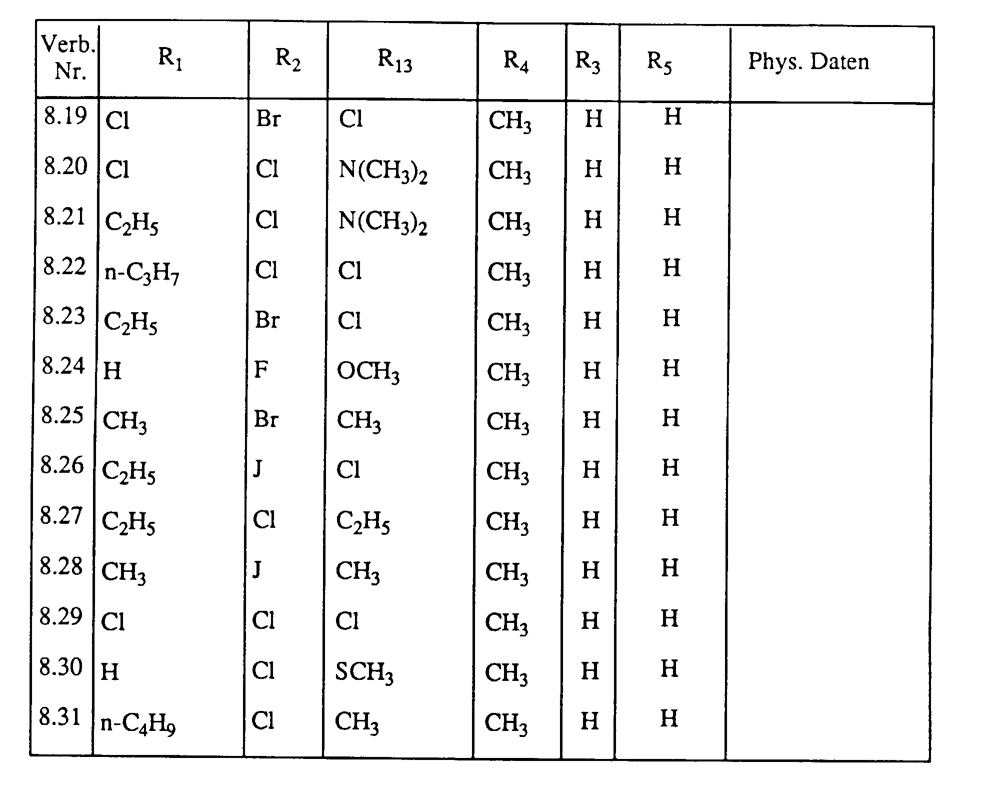

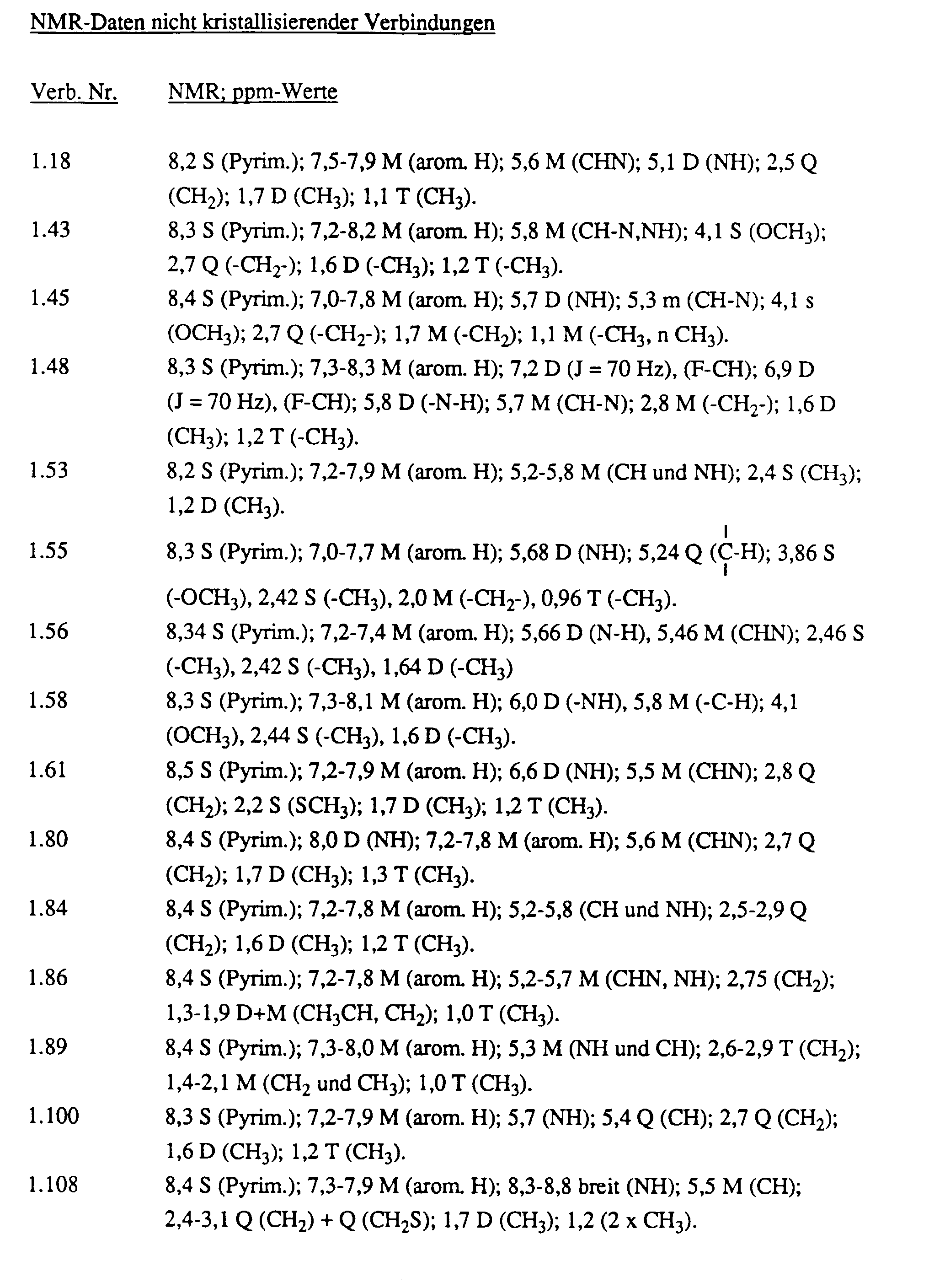

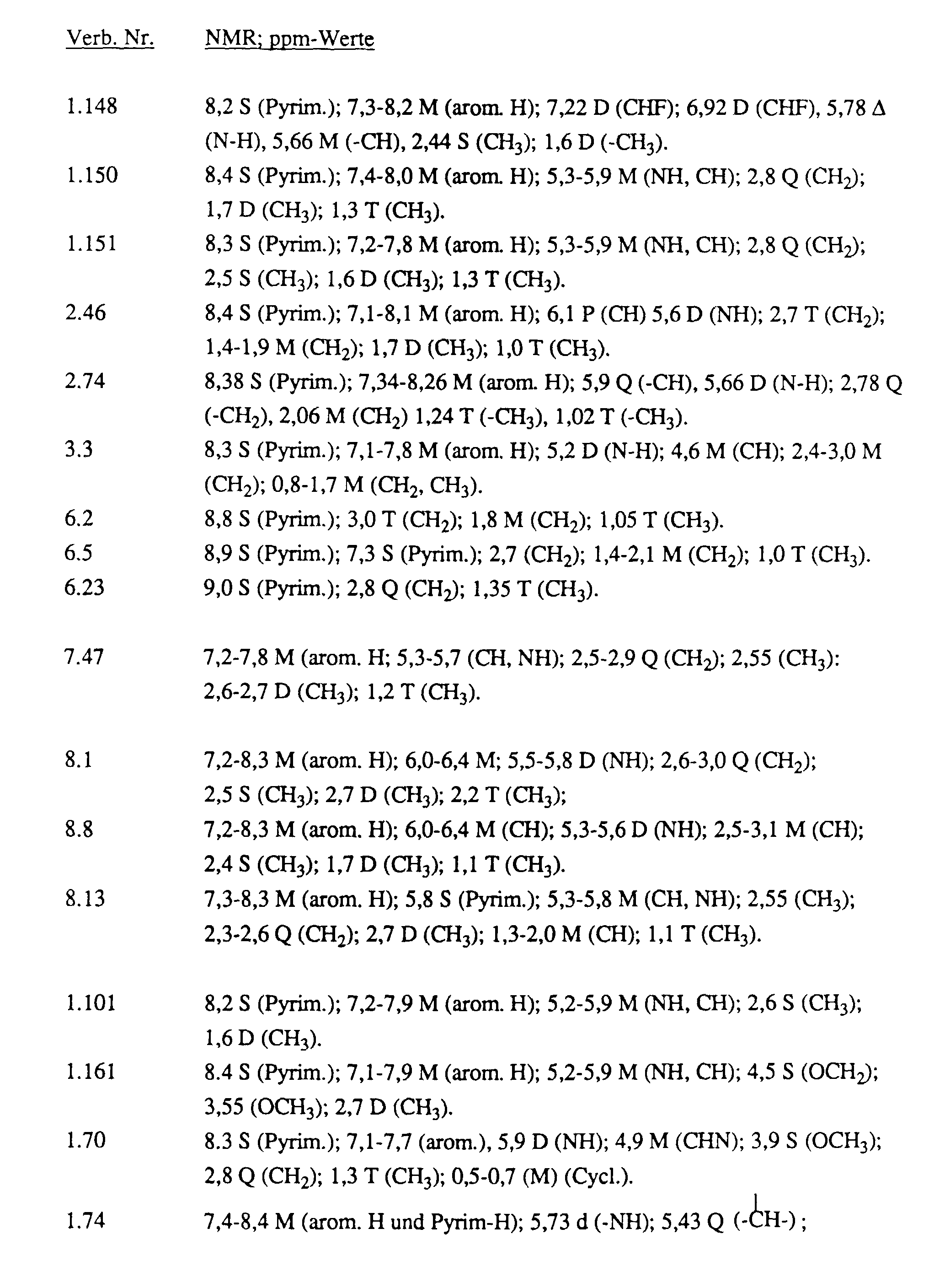

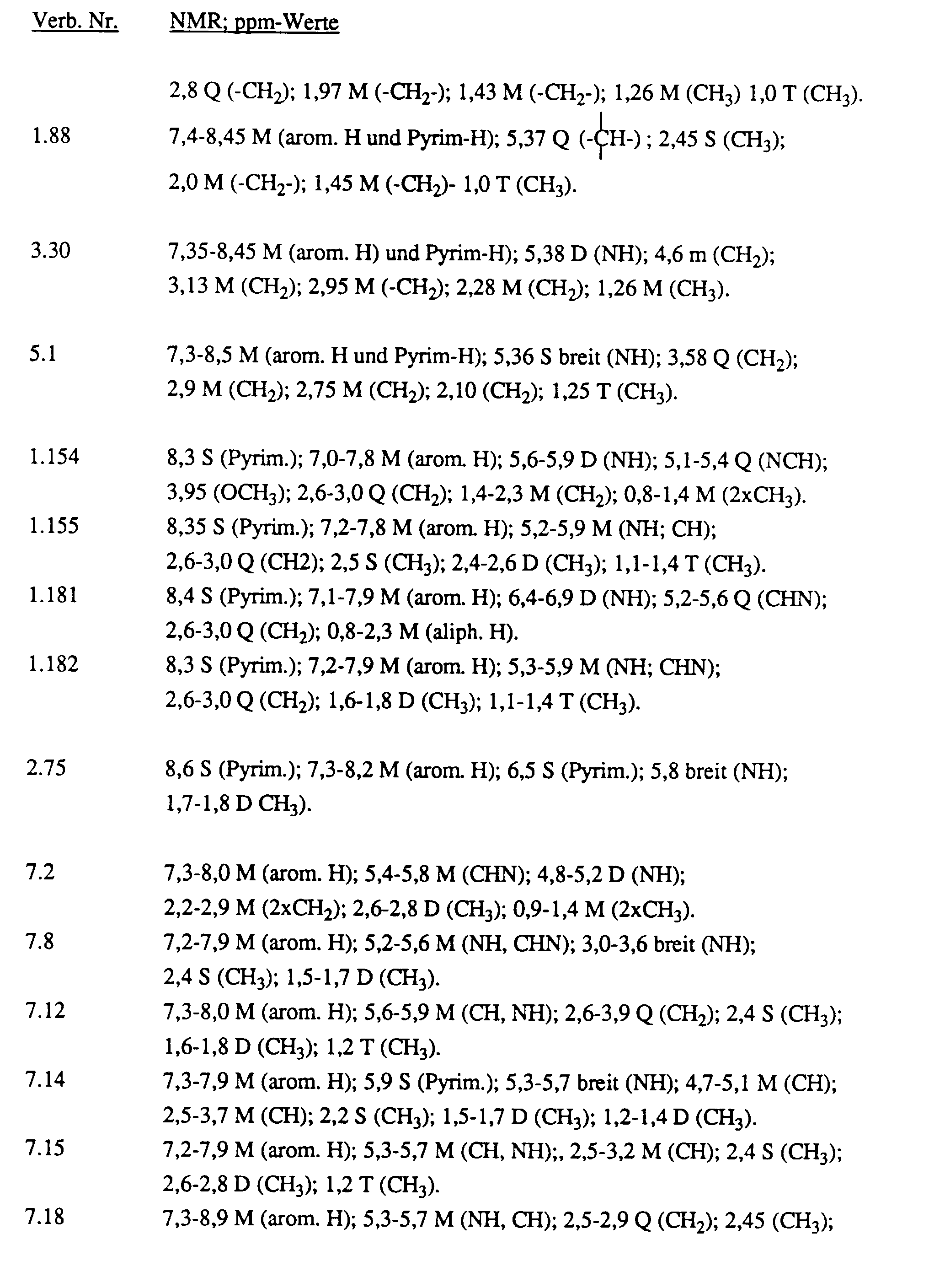

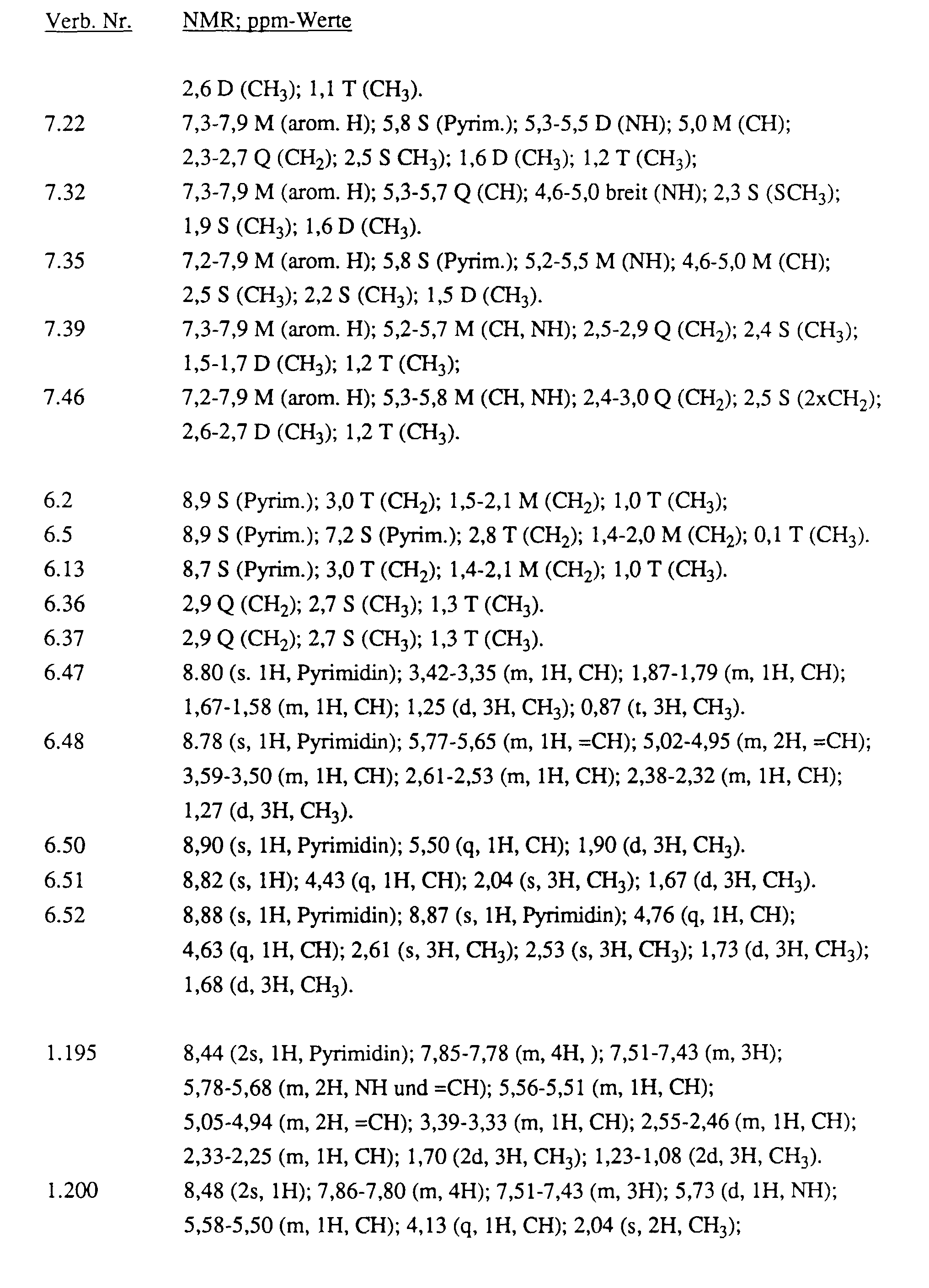

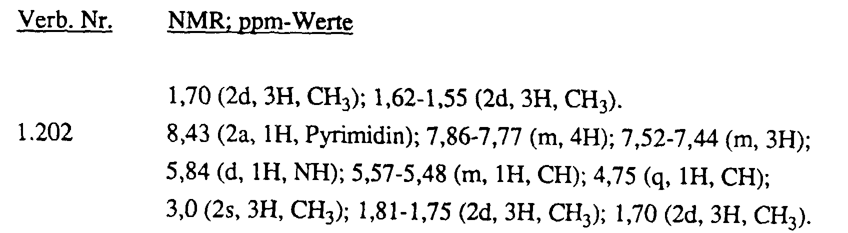

### Formulierungsbeispiele für Wirkstoffe der Formel I (% = Gewichtsprozent)

| 1. Emulsions-Konzentrate | a) | b) | c) |
|---|---|---|---|
| Wirkstoff gemäss Tabelle 1 bis 5, 7, 8 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykolether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff gemäss Tabelle 1 bis 5, 7, 8 | 80 % | 10 % | 5 % | 95 % |
| Propylenglykol-monomethyl-ether | 20 % | - | - | - |
| Polyethylenglykol MG400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 3. Granulate | a) | b) | c) | d) |
|---|---|---|---|---|
| Wirkstoff gemäss Tabelle 1 bis 5, 7, 8 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff gemäss Tabelle 1 bis 5, 7, 8 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| 5. Spritzpulver | a) | b) | c) |
|---|---|---|---|
| Wirkstoff gemäss Tabelle 1 bis 5, 7, 8 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff gemäss Tabelle 1 bis 5, 7, 8 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Auf diesem Konzentrat können durch Verdünnen mit Wasser Emulsionen jeder gewünschten Konzentration hergestellt werden.

### Biologische Beispiele: A. Mikrobizide Wirkungen

B-1: Pythium ultimum auf Beta vulgaris (Zuckerrübe, "Kleinwanzleben Monogerm") und auf Pythium ultimum auf Zea mays (Mais, "Sweet Corn")
   Testmethode: Myzel von Pythium ultimum wird mit Erde gemischt (500 ml Myzelsuspension auf 10 Liter Erde) und das Pilz-Erdgemisch in 250 ml Plastikschalen abgefüllt. Nach einer 4-tägigen Inkubation bei 10°C werden in jede Schale 10 Saatkörner der zu prüfenden Pflanze (Mais oder Zuckerrübe) gesteckt. Am nächsten Tag werden die so vorbereiteten Schalen mit je 50 ml (aus 25 % Spritzpulver und Wasser hergestellten) Spritzlösungen mit 0,002 % AS übergossen. Nach einer 7-tägigen Inkubationsphase bei 10°C und einer anschliessenden 4-tägigen Inkubationsphase bei 22°C wird die Wirkung der Testsubstanzen nach Zahl und Aussehen der aufgelaufenen Pflanzen bewertet. Verbindungen aus den Tabellen 1 bis 5, 7 und 8 zeigen gegen Pythium-Erreger gute Wirkung.
B-2: Wirkung gegen Puccinia graminis auf Weizen
   a) Residual protektive Wirkung:
      Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.
   b) Systemische Wirkung:
      Zu Weizenpflanzen wird 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt Die Beurteilung der Rostpustelnentwicklung erfolgt 12 Tage nach der Infektion.

   Verbindungen aus den Tabellen 1 bis 5, 7 und 8 zeigten gegen Puccinia-Pilze eine gute Wirkung. So reduzierten z.B. die Verbindungen 1.2 und 2.2 den Pilzbefall auf 0 bis 20 %. Unbehandelte jedoch infizierte Kontrollpflanzen wiesen dagegen einen Puccinia-Befall von 100 % auf.
B-3: Wirkung gegen Phytophthora infestans auf Tomatenpflanzen
   a) Residual protektive Wirkung:
      Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infizien. Die Beurteilung des Pilzbefalls erfolgte nach einer Inkubation der infizierten Pflanzen während 5 Tagen bei 90-100 % relativer Luftfeuchtigkeit und 20°C.
   b) Systemische Wirkung:
      Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.
      Verbindungen aus den Tabellen 1 bis 5, 7 und 8 zeigten gegen den Phytophthora-Pilz eine gute Schutzwirkung. So reduzierten z.B. die Verbindungen 1.2, 1,18 und 1.19 den Pilzbefall auf 0 bis 20 %. Unbehandelte jedoch infizierte Kontrollpflanzen wiesen dagegen einen 100 %igen Phytophthora-Befall auf.
B-4: Wirkung gegen Cercospora arachidicola auf Erdnusspflanzen
   10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt.
   Die Beurteilung der fungiziden Wirkung erfolgt 12 Tage nach der Infektion basierend auf Anzahl und Grösse der auftretenden Flecken.
   Im Vergleich zu unbehandelten, jedoch infizierten Kontrollpflanzen (Anzahl und Grösse der Flecken = 100 %), zeigten Erdnusspflanzen, die mit Wirkstoffen aus den Tabellen 1 bis 5, 7 und 8 behandelt wurden, einen reduzierten Cercospora-Befall (10-20 %), so z.B. die Verbindungungen 1.2; 1,3; 1.53; 1.73; 1.84; 1.86; 1.100; 1.149.
B-5: Wirkung gegen Plasmopara viticola auf Reben
   a) Residual-protektive Wirkung:
      Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.
   b) Kurative Wirkung:
      Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20°C werden die infizierten Pflanzen mit mit einer aus Spritzpulver der Wirksubstanz hergestellten Spritzbrühe (0,02 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.
      Verbindungen aus den Tabellen 1 bis 5, 7 und 8 zeigten gegen Plasmopara viticola eine gute Schutzwirkung (Befall unter 20 %), so z.B. 1.2 und 1.18. Unbehandelte jedoch infizierte Kontrollpflanzen wiesen dagegen einen 100 %igen Plasmopara-Befall auf.
B-6: Wirkung gegen Pyricularia oryzae auf Reispflanzen
   a) Residual-protektive Wirkung
      Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und ca. 22°C wird der Pilzbefall beurteilt.
   b) Systemische Wirkung
      Zu 2 Wochen alten Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Pflanzen mit einer Konidiensuspension des Pilzes infiziert. Die infizierten Gerstenpflanzen wurden in einem Gewächshaus bei ca. 22°C aufgestellt und der Pilzbefall nach 10 Tagen beurteilt. Nach einer Inkubation der infizierten Pflanzen während 5 Tagen bei 95-100 % relativer Luftfeuchtigkeit und ca. 24°C wird der Pilzbefall beurteilt.
      Reispflanzen, die mit einer Spritzbrühe auf Basis der Aktivsubstanzen der Tabellen 1 bis 5 behandelt wurden, zeigten im Vergleich zu unbehandelten Kontrollpflanzen (100 % Befall) nur geringen Pilzbefall. So reduzierten z.B. im Test (a) die Verbindungen Nr. 1.2; 1.3; 1.53; 1.73; 1.84; 1.86; 1.100; 1.149 und im Test (b) die Verbindungen 1.46 und 1.47 den Pilzbefall auf 0 bis 10%.
B-7: Residual-protektive Wirkung gegen Venturia inaequalis auf Apfeltrieben.
   Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.
   Verbindungen aus den Tabellen 1 bis 5 zeigen gute Schutzwirkung gegen Venturia.
B-8: Wirkung gegen Erysiphae graminis auf Gerste
   a) Residual-protektive Wirkung
      Ca. 8 cm hohe Gerstenpflanzen wurden mit einer aus Spritzpulver des Wirkstoffes hergestellten Spritzbrühe (0,02 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.
   b) Systemische Wirkung
      Zu ca. 8 cm hohen Gerstenpflanzen wurde eine aus Spritzpulver des Wirkstoffes hergestellte Spritzbrühe gegossen (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.
      Verbindungen der Formeln aus den Tabellen 1 bis 5, 7 und 8 zeigten eine gute Wirkung gegen Erysiphae-Pilze. Unbehandelte aber infizierte Kontrollpflanzen zeigten einen Erysiphae-Befall von 100 %. Unter anderen Verbindungen aus den Tabellen hemmten die Verbindungen Nr. 1.2; 1.3; 1.53; 1.73; 1.84; 1.86; 1.100; 1.149 den Pilzbefall auf 0 bis 5 %.

### Biologische Beispiele: B. Akarizide/Insektizide Wirkungen

B-9: Wirkung gegen Tetranychus urticae
   Junge Bohnenpflanzen werden mit einer Mischpopulation von Tetranychus urticae besiedelt und einen Tag später mit einer wässrigen Emulsions- Spritzbrühe, die 400 ppm des Wirkstoffes enthält, besprüht. Die Pflanzen werden anschliessend für 6 Tage bei 25 °C inkubiert und danach ausgewertet. Aus dem Vergleich der Anzahl toter Eier, Larven und Adulte auf den behandelten zu denjenigen auf den unbehandelten Pflanzen wird die prozentuale Reduktion der Population (% Wirkung) bestimmt.
   Die Verbindungen der Tabelle 1 zeigen eine gute Wirkung gegen Tetranychus urticae in diesem Test. Insbesondere die Verbindungen 1.2, 1.3, 1.9; 1.43; 1.53; 1.55; 1.58; 1.84; 1.149; 3.1; 3.3 zeigen eine Wirkung über 80%.
B-10: Wirkung gegen Nilaparvata lugens
   Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
   Die Verbindungen der Tabelle 1 zeigen eine gute Wirkung gegen Nilaparvata lugens in diesem Test. Insbesondere die Verbindungen 1.2, 1.3, 1.9 zeigen eine Wirkung über 80 %.
B-11: Wirkung gegen Nephotettix cincticeps
   Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
   Die Verbindungen der Tabelle 1 zeigen eine gute Wirkung gegen Nephotettix cincticeps in diesem Test. Insbesondere die Verbindung 1.2 zeigt eine Wirkung über 80 %.
B-12: Wirkung gegen Bemisia tabaci
   Buschbohnen-Pflanzen werden in Gazekäfige gestellt und mit Adulten von Bemisia tabaci (Weisse Fliege) besiedelt. Nach erfolgter Eiablage werden alle Adulten entfernt und 10 Tage später die Pflanzen mit den darauf befindliche Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf prozentuale Schlupfrate im Vergleich zu den unbehandelten Kontrollansätzen.
   Verbindungen gemäss Tabelle 1 zeigen in diesem Test gute Wirkung gegen Bemisia tabaci. Insbesondere die Verbindung 1.2 zeigt eine Wirkung über 80 %.
B-13: Ovo/larvizide Wirkung auf Heliothis virescens
   Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensrate der Raupen im Vergleich zu unbehandelten Kontrollansätzen ausgewertet (prozentuale Reduktion der Population).
   Verbindungen gemäss Tabelle 1 zeigen in diesem Test gute Wirkung gegen Heliothis virescens. Insbesondere die Verbindungen 1.2, 1.2, 1.9 zeigen eine Wirkung von über 80 %.
B-14: Wirkung auf Mischpopulation von Tetranychus cinnabarinus.
   Verdünnungsreihe.
   BUSCHBOHNEN im 2-Blattstadium werden mit einer Mischpopulation (Eier, Larven/Nymphen, Adulte) eines OP-toleranten TETRANYCHUS CINNABARINUS Stammes besiedelt. Die Produkte werden 24h nach der Infektion mit den Dosierungen 200, 100, 50 mgAS/l in der automatischen Spritzkabine auf die Pflanzen appliziert. Die Substanzen sind formuliert und werden mit Wasser auf die entsprechenden Dosierungen verdünnt. Ausgewertet wird der Versuch 2 und 7 Tage nach der Applikation auf prozentuelle Mortalität gegen
   - Eier
   - Larven/Nymphen
   - Adulte.

Verbindungen der Tabellen 1 bis 5 zeigen über 80 % Mortalität in Verdünnungen bis 50 mg AS/Liter. Die jeweiligen (-)-Enantionmeren zeigen auch Wirkung bei noch tieferen Dosierungen.

## Patentansprüche (Patentansprüche für folgende(n) Vertragsstaat(en): CH, LI, DE, DK, FR, GB, IT, NL)

1. Verbindungen der Formel I 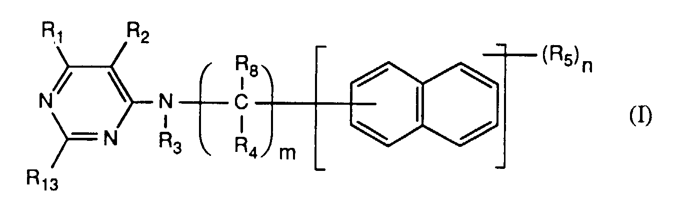 worin bedeuten:
R₁ Wasserstoff, C₁-C₅ Alkyl, das unsubstituiert oder durch Halogen oder C₁-C₃ Alkoxy oder S(O)ₚ-C₁-C₃-Alkyl substituiert ist; C₂-C₇ Alkenyl, das unsubstituiert oder durch Halogen substituiert ist; C₃-C₇ Cycloalkyl; Halogen; oder C₂-C₄-Alkinyl;
R₂ Wasserstoff, Hydroxy, C₁-C₅ Alkyl, das unsubstituiert oder durch Halogen oder C₁-C₃ Alkoxy substiutiert ist, C₁-C₄-Alkoxy, S(O)ₚ-C₁-C₄-Alkyl, Halogen, Nitro , Cyano, NHR₃, N(R₃)R₉ oder N=C(R₉)R₁₀;
R₃ Wasserstoff; C₁-C₅ Alkyl; Benzyl; -CO-R₆ oder -S-R₇;
R₄ und R₈ unabhängig voneinander Wasserstoff; C₁-C₅ Alkyl, das unsubstituiert oder durch Halogen oder C₁-C₃ Alkoxy substituiert ist; oder C₃-C₇ Cycloalkyl;
R₅ Halogen, C₁-C₃ Alkyl, C₁-C₃ Alkoxy, C₂-C₆-Alkoxyalkyl, Cᵢ-C₃-Halogenalkyl, C₁-C₃-Alkylthio, C₁-C₃-Halogenalkoxy, C₁-C₃-Halogenalkylthio, CN, NO₂ oder eine einmal am Naphthylring auftretende -X- Phenylgruppe, die unsubstituiert oder ein- bis dreifach gleich oder verschieden durch Halogen, C₁-C₃ Alkyl oder C₁-C₃ Alkoxy substituiert ist;
R₆ C₁-C₅ Alkyl; Phenyl, das unsubstituiert oder durch Halogen und/oder C₁-C₃ Alkyl substituiert ist;
R₇ Phenyl oder Benzyl, die unsubstituiert oder im Ring durch Halogen, Nitro, Cyano einbis zweifach gleich oder verschieden substituiert sind; oder C₁-C₅ Alkyl, das unsubstituiert oder durch Halogen oder Cyano substituiert ist;
R₉ C₁-C₅-Alkyl,
R₁₀ Wasserstoff oder C₁-C₅-Alkyl,
R₁₃ Wasserstoff; C₁-C₄ Alkyl, das unsubstituiert oder durch Halogen oder C₁-C₃ Alkoxy substituiert ist; Cyclopropyl; Halogen; C₁-C₃ Alkoxy; C₁-C₃ Alkylthio oder -N(C₁-C₃ Alkyl)₂;
X Sauerstoff oder Schwefel;
m 1, 2 oder 3;
n 0, 1, 2 oder 3 und
p 0, 1 oder 2.

2. Verbindungen der Formel I gemäss Anspruch 1, worin bedeuten:
R₁ Wasserstoff, C₁-C₅ Alkyl; oder durch S(O)ₚ-C₁-C₃-Alkyl substituiertes C₁-C₅-Alkyl; C₁-C₅ Haloalkyl mit 1 bis 5 Halogenatomen; C₂-C₆ Alkoxyalkyl; -C₂-C₇ Alkenyl; C₂-C₇ Haloalkenyl mit 1 bis 2 Halogenatomen; C₃-C₆ Cycloalkyl; Halogen oder C₂-C₄-Alkinyl;
R₂ Wasserstoff, C₁-C₅ Alkyl; C₁-C₅ Haloalkyl mit 1 bis 5 Halogenatomen; C₂-C₆ Alkoxyalkyl; Nitro, Cyano, S(O)ₚ-C₁-C₃-Alkyl, Amino, NHR₃, N(R₃)R₉, N=C(R₉)R₁₀ oder Halogen;
R₃ Wasserstoff; C₁-C₅ Alkyl; Benzyl; -CO-R₆ oder -S-R₇;
R₄ und R₈ unabhängig voneinander Wasserstoff; C₁-C₅ Alkyl; C₁-C₅ Haloalkyl mit 1 bis 3 Halogenatomen; C₂-C₆ Alkoxyalkyl oder C₃-C₆ Cycloalkyl;
R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, CN, NO₂ oder eine am Naphthylring auftretende X-Phenylgruppe, die unsubstituiert oder eine - bis dreifach gleich oder verschieden durch Halogen, C₁-C₃ Alkyl oder C₁-C₃ Alkoxy substituiert ist;
R₆ C₁-C₅ Alkyl oder Phenyl, das unsubstituiert oder durch Halogen und/oder C₁-C₃ Alkyl substiutiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
X Sauerstoff oder Schwefel
R₉ C₁-C₅-Alkyl,
R₁₀ Wasserstoff oder C₁-C₅-Alkyl,
m 1, 2 oder 3,
n 0, 1, 2 oder 3, und
p 0, 1 oder 2.

3. Verbindungen der Formel I gemäss Anspruch 1, worin bedeuten:
R₁ Wasserstoff, oder durch S(O)ₚ-C₁-C₃-Alkyl substituiertes C₁-C₅ Alkyl; C₁-C₅ Alkyl; C₁-C₃ Haloalkyl mit 1 bis 3 Halogenatomen; C₂-C₅ Alkoxyalkyl; C₂-C₅ Alkenyl; C₂-C₅ Haloalkenyl mit 1 bis 3 Halogenatomen; C₃-C₅ Cycloalkyl; Halogen oder C₂-C₄ Alkinyl;
R₂ Wasserstoff, C₁-C₅ Alkyl; C₁-C₃ Haloalkyl mit 1 bis 3 Halogenatomen; C₂-C₅ Alkoxyalkyl; Nitro, Cyano, S(O)ₚ-C₁-C₃-Alkyl, Amino, NHR₃, N(R₃)R₉, N=C(R₉)R₁₀ oder Halogen;
R₃ Wasserstoff; C₁-C₅ Alkyl; Benzyl; -CO-R₆ oder -S-R₇;
R₄ und R₈ unabhängig voneinander Wasserstoff; C₁-C₅ Alkyl; C₁-C₅ Haloalkyl mit 1 bis 3 Halogenatomen; C₂-C₅ Alkoxyalkyl oder C₃-C₆ Cycloalkyl;
R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, CN, NO₂ oder eine am Naphthylring auftretende X-Phenylgruppe, die unsubstituiert oder eine - bis dreifach gleich oder verschieden durch Halogen, C₁-C₃ Alkyl oder C₁-C₃ Alkoxy substituiert ist;
R₆ C₁-C₅ Alkyl oder Phenyl, das unsubstituiert oder durch Halogen und/oder C₁-C₃ substiutiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
X Sauerstoff oder Schwefel
R₉ C₁-C₅-Alkyl,
R₁₀ Wasserstoff oder C₁-C₅-Alkyl,
m 1, 2 oder 3;
n 0, 1,2 oder 3; und
p 0,1 oder 2.

4. Verbindung der Formel I' 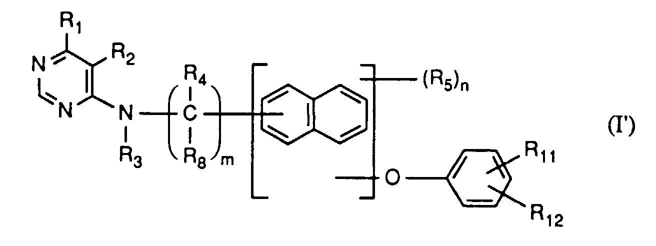 worin bedeuten:
R₁ Wasserstoff, C₁-C₅ Alkyl, oder durch S(O)ₚ-C₁-C₃-Alkyl substituiertes C₁-C₅-Alkyl; C₁-C₃ Haloalkyl mit 1 bis 3 Halogenatomen; C₂-C₅ Alkoxyalkyl; C₂-C₅ Alkenyl; C₂-C₅ Haloalkenyl mit 1 bis 3 Halogenatomen; C₃-C₆ Cycloalkyl; Halogen oder C₂-C₄ Alkinyl;
R₂ Wasserstoff, C₁-C₅ Alkyl; C₁-C₃ Haloalkyl mit 1 bis 3 Halogenatomen; C₃-C₅ Alkoxyalkyl; Nitro, Cyano, S(O)ₚ-C₁-C₃-Alkyl, Amino, NHR₃, N(R₃)R₉, N=C(R₉)R₁₀ oder Halogen;
R₃ Wasserstoff; C₁-C₅ Alkyl; Benzyl; -CO-R₆ oder -S-R₇;
R₄ und R₈ unabhängig voneinander Wasserstoff; C₁-C₅ Alkyl; C₁-C₅ Haloalkyl mit 1 bis 3 Halogenatomen; C₂-C₆ Alkoxyalkyl oder C₃-C₆ Cycloalkyl; C₂-C₆ Alkoxyalkyl oder C₃-C₆ Cycloalkyl;
R₅ Halogen; C₁-C₃ Alkyl; C₁-C₃ Alkoxy; C₁-C₃ Halogenalkyl, C₁-C₃ Halogenalkoxy, R₆ C₁-C₅ Alkyl; Phenyl, das unsubstituiert oder durch Halogen und/oder C₁-C₃ Alkyl substiutiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
R₉ C₁-C₅-Alkyl,
R₁₀ Wasserstoff oder C₁-C₅-Alkyl,
R₁₁ und R₁₂ unabhängig voneinander Wasserstoff, Halogen oder C₁-C₃-Alkyl,
m 1, 2 oder 3;
n 0, 1, 2; und
p 0, 1 oder 2.

5. Verbindungen der Formel I' gemäss Anspruch 4, worin
R₁ bis R₄ und R₈ die unter Formel I angegebenen Bedeutungen haben,
R₅ Halogen, C₁-C₃ Alkyl, CF₃, C₂-C₅ Alkoxyalkyl oder C₁-C₃Alkoxy;
R₁₁ und R₁₂ unabhängig voneinander Wasserstoff, Halogen oder C₁-C₃ Alkyl bedeuten;
m 1, 2 oder 3; und
n 0, 1 oder 2 darstellen.

6. Verbindungen der Formel I gemäss Anspruch 1, worin bedeuten:
R₁ Wasserstoff, C₁-C₅ Alkyl; C₁-C₅ Haloalkyl mit 1 bis 3 Halogenatomen; C₂-C₅ Alkoxyalkyl; C₂-C₅ Alkenyl; C₂-C₅ Halogenalkenyl oder Halogen;
R₂ Wasserstoff, C₁-C₅ Alkyl; C₁-C₃ Halogenalkyl mit 1 bis 3 Halogenatomen; C₃-C₅ Alkoxyalkyl; Nitro, Cyano, S(O)ₚ-C₁-C₄-Alkyl, Amino, NHR₃, N(R₃)R₉, N=C(R₉)R₁₀ oder Halogen;
R₃ Wasserstoff, C₁-C₃-Alkyl, Benzyl, -CO-R₆ oder SR₇;
R₄ und R₈ unabhängig voneinander Wasserstoff; C₁-C₅ Alkyl; C₁-C₅ Halogenalkyl mit 1 bis 3 Halogenatomen; C₂-C₆ Alkoxyalkyl oder C₃-C₆ Cycloalkyl;
R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, Cyano oder Nitro;
R₆ C₁-C₅ Alkyl, Phenyl, durch Halogen und/oder C₁-C₃ Alkyl substiutiertes Phenyl;
R₇ Phenyl, durch Halogen, Nitro oder Cyano ein- oder zweifach substituiertes Phenyl, Benzyl, durch Halogen, Nitro oder Cyano ein- oder zweifach im Ring substituiertes Benzyl, oder C₁-C₅ Alkyl oder durch Halogen oder Cyano substituiertes C₁-C₅ Alkyl;
R₉ C₁-C₅-Alkyl;
R₁₀ Wasserstoff oder C₁-C₃-Alkyl;
R₁₃ Wasserstoff oder C₁-C₄-Alkyl;
m 1, 2 oder 3;
n 0, 1 oder 2; und
p 0,1 oder 2.

7. Verbindungen gemäss Anspruch 6, worin bedeuten:
R₁ C₁-C₅ Alkyl; CF₃, C₂-C₅ Alkoxyalkyl; C₂-C₄ Alkenyl; C₂-C₄ Monohaloalkenyl; oder Halogen;
R₂ C₁-C₅ Alkyl oder Halogen;
R₃ Wasserstoff oder C₁-C₃ Alkyl;
R₄ Wasserstoff; C₁-C₃ Alkyl oder Cyclopropyl;
R₅ Halogen; C₁-C₂ Alkyl; C₁-C₃ Alkoxy;
R₈ Wasserstoff;
n 0, 1, 2 oder 3; und
m 1.

8. Verbindungen gemäss Anspruch 7, worin bedeuten:
R₁ C₁-C₄ Alkyl; CF₃; C₂-C₄ Alkoxyalkyl; C₂-C₄ Alkenyl; oder Halogen;
R₂ C₁-C₄ Alkyl oder Halogen;
R₃ Wasserstoff oder C₁-C₃ Alkyl;
R₄ Wasserstoff oder C₁-C₃ Alkyl;
R₅ Halogen; Methyl; Ethyl oder Methoxy;
R₈ Wasserstoff;
n 0, 1, 2 oder 3; und
m 1.

9. Verbindungen der Formel I'' 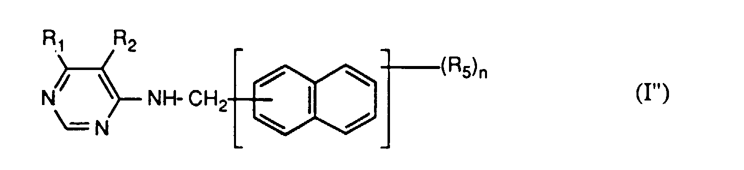 worin bedeuten:
R₁ C₁-C₄ Alkyl, CF₃, C₂-C₄ Alkoxyalkyl oder Halogen;
R₂ C₁-C₄ Alkyl oder Halogen;
R₅ Chlor, Brom, Methyl, Ethyl, Methoxy und
n 0, 1 oder 2.

10. Verbindungen der Formel I''' 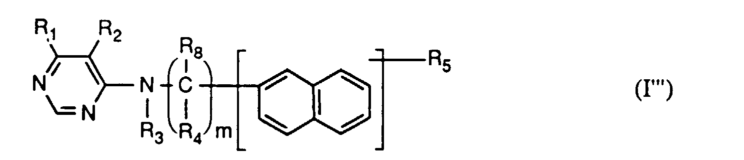 worin bedeuten:
R₁ Wasserstoff, C₁-C₃ Alkyl, CF₃ oder Halogen;
R₂ Wasserstoff, C₁-C₃ Alkyl, Halogen, NO₂ oder S(O)ₚ-C₁-C₃-Alkyl;
R₃ Wasserstoff;
R₄ Methyl, Ethyl, Isopropyl, n-Propyl oder Cyclopropyl;
R₈ Wasserstoff, Methyl oder Ethyl;
R₅ Wasserstoff, Halogen, C₁-C₃-Alkyl, OCHF₂, CF₃, NO₂ oder C₁-C₃-Alkoxy;
m 1 oder 2; und
p 0,1 oder 2.

11. Verbindungen gemäss Anspruch 3, worin bedeuten:
R₁ Wasserstoff, C₁-C₅ Alkyl; C₁-C₃ Halogenalkyl mit 1 bis 3 Halogenatomen; C₂-C₅ Alkoxyalkyl; C₂-C₅ Alkenyl; C₂-C₅ Halogenalkenyl mit 1 bis 3 Halogenatomen, C₃-C₆-Cycloalkyl oder Halogen;
R₂ Wasserstoff, C₁-C₅ Alkyl; C₁-C₃ Halogenalkyl mit 1 bis 3 Halogenatomen; C₃-C₅ Alkoxyalkyl; Nitro, Cyano, S(O)ₚ-C₁-C₄-Alkyl, Amino, NHR₃, N(R₃)R₉, N=C(R₉)R₁₀ oder Halogen;
R₃ Wasserstoff; C₁-C₅ Alkyl, Benzyl, COR₆ oder SR₇;
R₄ und R₈ unabhängig voneinander Wasserstoff; C₁-C₅ Alkyl; C₁-C₅ Halogenalkyl mit 1 bis 3 Halogenatomen; C₂-C₆ Alkoxyalkyl oder C₃-C₆ Cycloalkyl;
R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, Cyano oder Nitro oder eine am Naphthylring auftretende X-Phenylgruppe, die unsubstituiert oder ein- bis dreifach gleich oder verschieden durch Halogen, C₁-C₃-Alkyl oder C₁-C₃-Alkoxy substituiert ist;
R₆ C₁-C₅ Alkyl, Phenyl, durch Halogen und/oder C₁-C₃ Alkyl substituiertes Phenyl;
R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituierter Benzyl, oder C₁-C₅ Alkyl oder durch Halogen oder Cyano substituiertes C₁-C₅ Alkyl;
R₉ C₁-C₅ Alkyl;
R₁₀ Wasserstoff oder C₁-C₅ Alkyl;
R₁₃ Wasserstoff;
X Sauerstoff oder Schwefel
m 2 oder 3,
n 0, 1, 2 oder 3; und
p 0, 1 oder 2.

12. Verbindungen gemäss Anspruch 11, worin bedeuten:
R₁ Wasserstoff, C₁-C₃ Alkyl; C₁-C₃ Halogenalkyl mit 1 bis 3 Halogenatomen; C₂-C₅ Alkoxyalkyl; C₂-C₅ Alkenyl; C₃-C₆-Cycloalkyl oder Halogen;
R₂ Wasserstoff, C₁-C₅ Alkyl; C₁-C₃ Halogenalkyl mit 1 bis 3 Halogenatomen; C₃-C₅ Alkoxyalkyl; Nitro, Cyano, S(O)ₚ-C₁-C₃-Alkyl, Amino, NHR₃, N(R₃)R₉, N=C(R₉)R₁₀ oder Halogen;
R₃ Wasserstoff; C₁-C₃ Alkyl;
R₄ und R₈ unabhängig voneinander Wasserstoff; C₁-C₅ Alkyl; CF₃; C₂-C₆ Alkoxyalkyl oder C₃-C₆ Cycloalkyl;
R₅ Halogen; C₁-C₃ Alkyl; C₁-C₃ Alkoxy; C₂-C₆ Alkoxyalkyl, C₁-C₃ Halogenalkyl, C₁-C₃ Alkylthio, C₁-C₃ Halogenalkoxy, C₁-C₃ Halogenalkylthio, Cyano oder Nitro;
R₆ C₁-C₅ Alkyl, Phenyl, durch Halogen und/oder C₁-C₃ Alkyl substituiertes Phenyl;
R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituiertes Benzyl, oder C₁-C₅ Alkyl oder durch Halogen oder Cyano substituiertes C₁-C₅ Alkyl;
R₉ C₁-C₅ Alkyl;
R₁₀ Wasserstoff oder C₁-C₅ Alkyl;
R₁₃ Wasserstoff;
X Sauerstoff oder Schwefel
m 2 oder 3,
n 0, 1, 2 oder 3; und
p 0,1 oder 2.

13. Verbindungen gemäss Anspruch 11, worin bedeuten:
R₁ Wasserstoff, C₁-C₃ Alkyl; CF₃, C₂-C₅ Alkoxyalkyl; C₂-C₅ Alkenyl; C₃-C₆-Cycloalkyl oder Halogen;
R₂ Wasserstoff, C₁-C₅ Alkyl; CF₃, C₃-C₅ Alkoxyalkyl; Nitro, Cyano, S(O)ₚ-C₁-C₄-Alkyl, Amino, N(R₃)R₉, N=C(R₉)R₁₀ oder Halogen;
R₃ Wasserstoff;
R₄ und R₈ unabhängig voneinander Wasserstoff; C₁-C₅ Alkyl; C₂-C₆ Alkoxyalkyl oder C₃-C₆ Cycloalkyl;
R₅ Halogen; C₁-C₃ Alkyl; C₁-C₃ Alkoxy; C₂-C₆ Alkoxyalkyl, Halogenalkyl, C₁-C₃ Alkylthio, C₁-C₃ Halogenalkoxy, C₁-C₃ Halogenalkylthio, Nitro oder Cyano;
R₆ C₁-C₅ Alkyl, Phenyl oder durch Halogen und/oder C₁-C₃ Alkyl substituiert ist;
R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituiertes Benzyl, oder C₁-C₅ Alkyl oder durch Halogen oder Cyano substituiertes C₁-C₅ Alkyl;
R₉ C₁-C₃ Alkyl;
R₁₀ Wasserstoff oder C₁-C₃ Alkyl;
R₁₃ Wasserstoff;
X Sauerstoff oder Schwefel
m 2;
n 0, 1, 2 oder 3; und
p 0, 1 oder 2.

14. Verbindungen gemäss Anspruch 13, worin bedeuten:
R₁ Wasserstoff, C₁-C₃ Alkyl; CF₃, C₂-C₅ Alkoxyalkyl; C₂-C₅ Alkenyl; C₃-C₆-Cycloalkyl oder Halogen;
R₂ Wasserstoff, C₁-C₅ Alkyl; CF₃, C₃-C₅ Alkoxyalkyl; Nitro, Cyano, S(O)ₚ-C₁-C₃-Alkyl, Amino, N(R₃)R₉, N=C(R₉)R₁₀ oder Halogen;
R₃ Wasserstoff;
R₄ und R₈ unabhängig voneinander Wasserstoff; C₁-C₅ Alkyl; C₂-C₆ Alkoxyalkyl oder C₃-C₆ Cycloalkyl;
R₅ Halogen; C₁-C₃ Alkyl; C₁-C₃ Alkoxy; C₂-C₆ Alkoxyalkyl, C₁-C₃ Halogenalkyl, C₁-C₃ Alkylthio, C₁-C₃ Halogenalkoxy, C₁-C₃ Halogenalkylthio, Nitro oder Cyano;
R₆ C₁-C₅ Alkyl, Phenyl oder durch Halogen und/oder C₁-C₃ Alkyl substituiertes Phenyl;
R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituiertes Benzyl, oder C₁-C₅ Alkyl oder durch Halogen oder Cyano substituiertes C₁-C₅ Alkyl;
R₉ C₁-C₅ Alkyl;
R₁₀ Wasserstoff oder C₁-C₃ Alkyl;
R₁₃ Wasserstoff;
X Sauerstoff oder Schwefel
m 3,
n 0, 1, 2 oder 3; und
p 0, 1 oder 2.

15. Verbindungen der Formel Ia 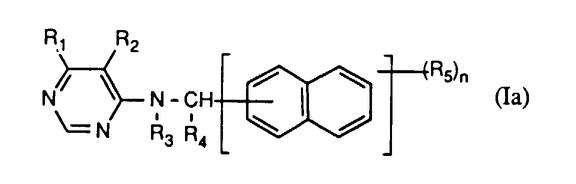 in welcher bedeuten:
R₁ C₁-C₅ Alkyl, das unsubstituiert oder durch Halogen oder C₁-C₃ Alkoxy substituiert ist; C₂-C₇ Alkenyl, das unsubstituiert oder durch Halogen substituiert ist; C₃-C₇ Cycloalkyl; oder Halogen;
R₂ C₁-C₅ Alkyl, das unsubstituiert oder durch Halogen oder C₁-C₃ Alkoxy substiutiert ist; Halogen; Nitro oder Cyano;
R₃ Wasserstoff; C₁-C₅ Alkyl; Benzyl; -CO-R₆ oder -S-R₇;
R₄ Wasserstoff; C₁-C₅ Alkyl, das unsubstituiert oder durch Halogen oder C₁-C₃ Alkoxy substituiert ist; oder C₃-C₇ Cycloalkyl;
R₅ Halogen; C₁-C₃ Alkyl; C₁-C₃ Alkoxy; C₁-C₃Alkylthio; oder eine einmal am Phenylring auftretende -X-Phenyl-Gruppe, die unsubstituiert oder ein- bis dreifach gleich oder verschieden durch Halogen, C₁-C₃ Alkyl oder C₁-C₃ Alkoxy substituiert ist;
R₆ C₁-C₅ Alkyl; Phenyl, das unsubstituiert oder durch Halogen und/oder C₁-C₃ Alkyl substituiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
X Sauerstoff oder Schwefel; und
n 0, 1, 2 oder 3.

16. Verbindungen der Formel I gemäß Anspruch 1 aus der Gruppe:
(d,l)-4-[1'-(β-Naphthyl)-ethylamino]-5-chlor-6-ethylpyrimidin (Verb. 1.2);
(-)-4-[1'(β-Naphthyl)-ethylamino]-5-chlor-6-ethylpyrimidin (Verb. 1.73);
(d,l)-4-[1'-(β-Naphthyl)-propylamino]-5-chlor-6-ethylpyrimidin (Verb. 1.3);
(d,l)-4-[1'-(β-Naphthyl)-ethylamino]-5-chlor-6-methylpyrimidin (Verb. 1.53);
(d,l)-4-[1'-(2-(6-Bromnaphthyl))-ethylamino]-5-chlor-6-ethylpyrimidin (Verb. 1.149);
(d,l)-4-[1'-(β-Naphthyl)-ethylamino]-5-brom-6-ethylpyrimidin (Verb. 1.84);
(d,l)-4-[1'-(β-Naphthyl)-ethylamino]-5-chlor-6-n-propylpyrimidin (Verb. 1.86);
(d,l)-4-[1'-(β-Naphthyl)-ethylamino]-5-jod-6-ethylpyrimidin (Verb. 1.100)

17. Verfahren zur Herstellung der Verbindungen der Formel I gemäß Anspruch 1, dadurch gekennzeichnet, dass man eine Verbindung der Formel II 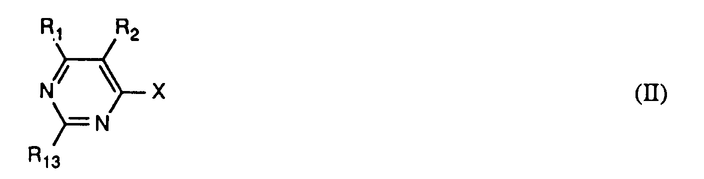 worin R₁, R₂ und R₁₃ die für Formel I angegebene Bedeutung haben und X einen leicht abspaltbaren Rest bedeutet, mit einer Verbindung der Formel III 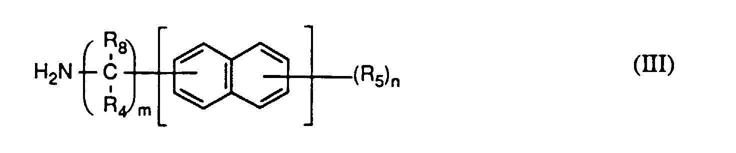 worin R₄, R₅, R₈, m und n die für Formel I gegebene Bedeutung haben, zur Reaktion bringt, vorzugsweise in Gegenwart einer Base, und sofern gewünscht, anschliessend den Substituenten R₃, sofern dieser Substituent Alkyl, Benzyl, Acyl oder -S-R₇ bedeutet, durch entsprechende N-Alkylierung, N-Benzylierung, N-Acylierung oder N-Thioalkylierung bzw. Thioarylierung einführt.

18. Mittel zum Schutz von Pflanzen gegen den Befall durch Schädlinge, dadurch gekennzeichnet, dass es als aktive Komponente mindestens eine Verbindung gemäss Anspruch 1 und einen Trägerstoff enthält.

19. Mittel gemäss Anspruch 18, dadurch gekennzeichnet, dass es als aktive Komponente mindestens eine Verbindung gemäss den Ansprüchen 2 bis 15 enthält.

20. Mittel gemäss Anspruch 18, dadurch gekennzeichnet, dass es als aktive Komponente eine Verbindung der Formel I gemäss Anspruch 16 enthält.

21. Verfahren zur Herstellung eines agrochemischen Mittels von Anspruch 18, dadurch gekennzeichnet, dass man mindestens eine gemäss Anspruch 1 definierte Verbindung mit geeigneten festen oder flüssigen Träger- und Hilfsstoffen innig vermischt.

22. Verwendung von Verbindungen gemäss Anspruch 1 zum Schutz von Pflanzen gegen den Befall durch phytopathogene Mikroorganismen.

23. Verwendung von Verbindungen gemäss einem der Ansprüche 2 bis 16 zum Schutz von Pflanzen gegen den Befall durch phytopathogene Mirkroorganismen.

24. Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanzen oder deren Standort appliziert.

25. Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 16 auf die Pflanzen odere deren Standort appliziert.

26. Verfahren gemäss Anspruch 25, dadurch gekennzeichnet, dass es sich bei den phytopathogenen Mikroorganismen um Pilzorganismen handelt.

## Patentansprüche (Patentansprüche für folgende(n) Vertragsstaat(en): ES)

1. Verfahren zum Schutz von Pflanzen gegen den Befall durch phytopathogene Microorganismen, dadurch gekennzeichnet, dass man als Wirkstoff eine Verbindung der Formel I auf die Pflanzen oder deren Standort appliziert, 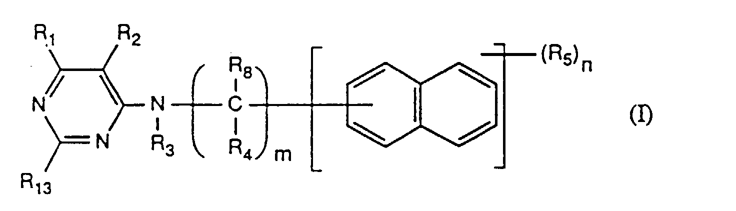 worin bedeuten:
R₁ Wasserstoff, C₁-C₅ Alkyl, das unsubstituiert oder durch Halogen oder C₁-C₃ Alkoxy oder S(O)_{P}-C₁-C₃-Alkyl substituiert ist; C₂-C₇ Alkenyl, das unsubstituiert oder durch Halogen substituiert ist; C₃-C₇ Cycloalkyl; Halogen; oder C₂-C₄-Alkinyl;
R₂ Wasserstoff, Hydroxy, C₁-C₅ Alkyl, das unsubstituiert oder durch Halogen oder C₁-C₃ Alkoxy substituiert ist, C₁-C₄-Alkoxy, S(O)ₚ-C₁-C₄-Alkyl, Halogen, Nitro , Cyano, NHR₃, N(R₃)R₉ oder N=C(R₉)R₁₀;
R₃ Wasserstoff; C₁-C₅ Alkyl; Benzyl; -CO-R₆ oder -S-R₇;
R₄ und R₈ unabhängig voneinander Wasserstoff; C₁-C₅ Alkyl, das unsubstituiert oder durch Halogen oder C₁-C₃ Alkoxy substituiert ist; oder C₃-C₇ Cycloalkyl;
R₅ Halogen, C₁-C₃ Alkyl, C₁-C₃ Alkoxy, C₂-C₆-Alkoxyalkyl, C₁-C₃-Halogenalkyl, C₁-C₃Alkylthio, C₁-C₃-Halogenalkoxy, C₁-C₃-Halogenalkylthio, CN, NO₂ oder eine einmal am Naphthylring auftretende -X- Phenylgruppe, die unsubstituiert oder ein- bis dreifach gleich oder verschieden durch Halogen, C₁-C₃ Alkyl oder C₁-C₃ Alkoxy substituiert ist;
R₆ C₁-C₅ Alkyl; Phenyl, das unsubstituiert oder durch Halogen und/oder C₁-C₃ Alkyl substituiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
R₉ C₁-C₅-Alkyl,
R₁₀ Wasserstoff oder C₁-C₅-Alkyl,
R₁₃ Wasserstoff; C₁-C₄ Alkyl, das unsubstituiert oder durch Halogen oder C₁-C₃ Alkoxy substituiert ist; Cyclopropyl; Halogen; C₁-C₃ Alkoxy; C₁-C₃ Alkylthio oder -N(C₁-C₃ Alkyl)₂;
X Sauerstoff oder Schwefel;
m 1, 2 oder 3;
n 0, 1, 2 oder 3 und
p 0, 1 oder 2.

2. Verfahren gemäss Anspruch 1, worin bedeuten:
R₁ Wasserstoff, C₁-C₅ Alkyl; oder durch S(O)ₚ-C₁-C₃-Alky substituiertes C₁-C₅-Alkyl; C₁-C₅ Haloalkyl mit 1 bis 5 Halogenatomen; C₂-C₆ Alkoxyalkyl; C₂-C₇ Alkenyl; C₂-C₇ Haloalkenyl mit 1 bis 2 Halogenatomen; C₃-C₆ Cycloalkyl; Halogen oder C₂-C₄-Alkinyl;
R₂ Wasserstoff, C₁-C₅ Alkyl; C₁-C₅ Haloalkyl mit 1 bis 5 Halogenatomen; C₂-C₆ Alkoxyalkyl; Nitro, Cyano, S(O)ₚ-C₁-C₃-Alkyl, Amino, NHR₃, N(R₃)R₉, N=C(R₉)R₁₀ oder Halogen;
R₃ Wasserstoff; C₁-C₅ Alkyl; Benzyl; -CO-R₆ oder -S-R₇;
R₄ und R₈ unabhängig voneinander Wasserstoff; C₁-C₅ Alkyl; C₁-C₅ Haloalkyl mit 1 bis 3 Halogenatomen; C₂-C₆ Alkoxyalkyl oder C₃-C₆ Cycloalkyl;
R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, CN, NO₂ oder eine am Naphthylring auftretende X-Phenylgruppe, die unsubstituiert oder eine - bis dreifach gleich oder verschieden durch Halogen, C₁-C₃ Alkyl oder C₁-C₃ Alkoxy substituiert ist;
R₆ C₁-C₅ Alkyl oder Phenyl, das unsubstituiert oder durch Halogen und/oder C₁-C₃ Alkyl substiutiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
X Sauerstoff oder Schwefel
R₉ C₁-C₅-Alkyl,
R₁₀ Wasserstoff oder C₁-C₅-Alkyl,
m 1, 2 oder 3,
n 0, 1, 2 oder 3, und
p 0, 1 oder 2.

3. Verfahren gemäss Anspruch 1, worin bedeuten:
R₁ Wasserstoff, oder durch S(O)ₚ-C₁-C₃-Alkyl substituiertes C₁-C₅ Alkyl; C₁-C₅ Alkyl; C₁-C₃ Haloalkyl mit 1 bis 3 Halogenatomen; C₂-C₅ Alkoxyalkyl; C₂-C₅ Alkenyl; C₂-C₅ Haloalkenyl mit 1 bis 3 Halogenatomen; C₃-C₅ Cycloalkyl; Halogen oder C₂-C₄ Alkinyl;
R₂ Wasserstoff, C₁-C₅ Alkyl; C₁-C₃ Haloalkyl mit 1 bis 3 Halogenatomen; C₂-C₅ Alkoxyalkyl; Nitro, Cyano, S(O)ₚ-C₁-C₃-Alky, Amino, NHR₃, N(R₃)R₉, N=C(R₉)R₁₀ oder Halogen;
R₃ Wasserstoff; C₁-C₅ Alkyl; Benzyl; -CO-R₆ oder -S-R₇;
R₄ und R₈ unabhängig voneinander Wasserstoff; C₁-C₅ Alkyl; C₁-C₅ Haloalkyl mit 1 bis 3 Halogenatomen; C₂-C₅ Alkoxyalkyl oder C₃-C₆ Cycloalkyl;
R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, CN, NO₂ oder eine am Naphthylring auftretende X-Phenylgruppe, die unsubstituiert oder eine - bis dreifach gleich oder verschieden durch Halogen, C₁-C₃ Alkyl oder C₁-C₃ Alkoxy substituiert ist;
R₆ C₁-C₅ Alkyl oder Phenyl, das unsubstituiert oder durch Halogen und/oder C₁-C₃ substiutiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
X Sauerstoff oder Schwefel
R₉ C₁-C₅-Alkyl,
R₁₀ Wasserstoff oder C₁-C₅-Alkyl,
m 1, 2 oder 3;
n 0, 1, 2 oder 3; und
p 0,1 1 oder 2.

4. Verfahren gemäss Anspruch 1, worin eine Verbindung der Formel I' appliziert wird, 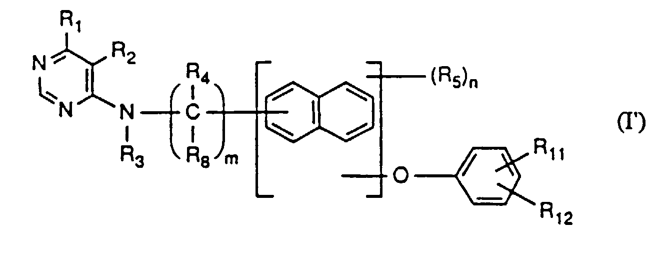 worin bedeuten:
R₁ Wasserstoff, C₁-C₅ Alkyl, oder durch S(O)ₚ-C₁-C₃-Alkyl substituiertes C₁-C₅-Alkyl; C₁-C₃ Haloalkyl mit 1 bis 3 Halogenatomen; C₂-C₅ Alkoxyalkyl; C₂-C₅ Alkenyl; C₂-C₅ Haloalkenyl mit 1 bis 3 Halogenatomen; C₃-C₆ Cycloalkyl; Halogen oder C₂-C₄ Alkinyl;
R₂ Wasserstoff, C₁-C₅ Alkyl; C₁-C₃ Haloalkyl mit 1 bis 3 Halogenatomen; C₃-C₅ Alkoxyalkyl; Nitro, Cyano, S(O)ₚ-C₁-C₃-Alkyl, Amino, NHR₃, N(R₃)R₉, N=C(R₉)R₁₀ oder Halogen;
R₃ Wasserstoff; C₁-C₅ Alkyl; Benzyl; -CO-R₆ oder -S-R₇;
R₄ und R₈ unabhängig voneinander Wasserstoff; C₁-C₅ Alkyl; C₁-C₅ Haloalkyl mit 1 bis 3 Halogenatomen; C₂-C₆ Alkoxyalkyl oder C₃-C₆ Cycloalkyl; C₂-C₆ Alkoxyalkyl oder C₃-C₆ Cycloalkyl;
R₅ Halogen; C₁-C₃ Alkyl; C₁-C₃ Alkoxy; C₁-C₃ Halogenalkyl, C₁-C₃ Halogenalkoxy,
R₆ C₁-C₅ Alkyl; Phenyl, das unsubstituiert oder durch Halogen und/oder C₁-C₃ Alkyl substiutiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
R₉ C₁-C₅-Alkyl,
R₁₀ Wasserstoff oder C₁-C₅-Alkyl,
R₁₁ und R₁₂ unabhängig voneinander Wasserstoff, Halogen oder C₁-C₃-Alkyl,
m 1, 2 oder 3;
n 0, 1, 2; und
p 0, 1 oder 2.

5. Verfahren gemäss Anspruch 4, worin
R₁ bis R₄ und R₈ die unter Formel I angegebenen Bedeutungen haben,
R₅ Halogen, C₁-C₃ Alkyl, CF₃, C₂-C₅ Alkoxyalkyl oder C₁-C₃Alkoxy;
R₁₁ und R₁₂ unabhängig voneinander Wasserstoff, Halogen oder C₁-C₃ Alkyl bedeuten;
m 1, 2 oder 3; und
n 0, 1 oder 2 darstellen.

6. Verfahren gemäss Anspruch 1, worin bedeuten:
R₁ Wasserstoff, C₁-C₅ Alkyl; C₁-C₅ Haloalkyl mit 1 bis 3 Halogenatomen; C₂-C₅ Alkoxyalkyl; C₂-C₅ Alkenyl; C₂-C₅ Halogenalkenyl oder Halogen;
R₂ Wasserstoff, C₁-C₅ Alkyl; C₁-C₃ Halogenalkyl mit 1 bis 3 Halogenatomen; C₃-C₅ Alkoxyalkyl; Nitro, Cyano, S(O)ₚ-C₁-C₄-Alkyl, Amino, NHR₃, N(R₃)R₉, N=C(R₉)R₁₀ oder Halogen;
R₃ Wasserstoff, C₁-C₃-Alkyl, Benzyl, -CO-R₆ oder SR₇;
R₄ und R₈ unabhängig voneinander Wasserstoff; C₁-C₅ Alkyl; C₁-C₅ Halogenalkyl mit 1 bis 3 Halogenatomen; C₂-C₆ Alkoxyalkyl oder C₃-C₆ Cycloalkyl;
R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, Cyano oder Nitro;
R₆ C₁-C₅ Alkyl, Phenyl, durch Halogen und/oder C₁-C₃ Alkyl substiutiertes Phenyl;
R₇ Phenyl, durch Halogen, Nitro oder Cyano ein- oder zweifach substituiertes Phenyl, Benzyl, durch Halogen, Nitro oder Cyano ein- oder zweifach im Ring substituiertes Benzyl, oder C₁-C₅ Alkyl oder durch Halogen oder Cyano substituiertes C₁-C₅ Alkyl;
R₉ C₁-C₅-Alkyl;
R₁₀ Wasserstoff oder C₁-C₃-Alkyl;
R₁₃ Wasserstoff oder C₁-C₄-Alkyl;
m 1, 2 oder 3;
n 0, 1 oder 2; und
p 0, 1 oder 2.

7. Verfahren gemäss Anspruch 6, worin bedeuten:
R₁ C₁-C₅ Alkyl; CF₃, C₂-C₅ Alkoxyalkyl; C₂-C₄ Alkenyl; C₂-C₄ Monohaloalkenyl; oder Halogen;
R₂ C₁-C₅ Alkyl oder Halogen;
R₃ Wasserstoff oder C₁-C₃ Alkyl;
R₄ Wasserstoff; C₁-C₃ Alkyl oder Cyclopropyl;
R₅ Halogen; C₁-C₂ Alkyl; C₁-C₃ Alkoxy;
R₈ Wasserstoff;
n 0, 1, 2 oder 3; und
m 1.

8. Verfahren gemäss Anspruch 7, worin bedeuten:
R₁ C₁-C₄ Alkyl; CF₃; C₂-C₄ Alkoxyalkyl; C₂-C₄ Alkenyl; oder Halogen;
R₂ C₁-C₄ Alkyl oder Halogen;
R₃ Wasserstoff oder C₁-C₃ Alkyl;
R₄ Wasserstoff oder C₁-C₃ Alkyl;
R₅ Halogen; Methyl; Ethyl oder Methoxy;
R₈ Wasserstoff;
n 0, 1, 2 oder 3;und
m 1.

9. Verfahren gemäss Anspruch 1, worin eine Verbindung der Formel I" appliziert wird, 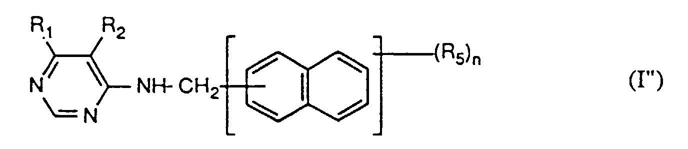 worin bedeuten:
R₁ C₁-C₄ Alkyl, CF₃, C₂-C₄ Alkoxyalkyl oder Halogen;
R₂ C₁-C₄ Alkyl oder Halogen;
R₅ Chlor, Brom, Methyl, Ethyl, Methoxy und
n 0, 1 oder 2.

10. Verfahren gemäss Anspruch 1, worin eine Verbindung der Formel I‴ appliziert wird, 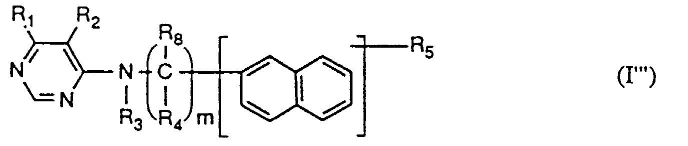 worin bedeuten:
R₁ Wasserstoff, C₁-C₃ Alkyl, CF₃ oder Halogen;
R₂ Wasserstoff, C₁-C₃ Alkyl, Halogen, NO₂ oder S(O)ₚ-C₁-C₃-Alkyl;
R₃ Wasserstoff;
R₄ Methyl, Ethyl, Isopropyl, n-Propyl oder Cyclopropyl;
R₈ Wasserstoff, Methyl oder Ethyl;
R₅ Wasserstoff, Halogen, C₁-C₃-Alkyl, OCHF₂, CF₃, NO₂ oder C₁-C₃-Alkoxy;
m 1 oder 2; und
p 0, 1 oder 2.

11. Verfahren gemäss Anspruch 3, worin bedeuten:
R₁ Wasserstoff, C₁-C₅ Alkyl; C₁-C₃ Halogenalkyl mit 1 bis 3 Halogenatomen; C₂-C₅ Alkoxyalkyl; C₂-C₅ Alkenyl; C₂-C₅ Halogenalkenyl mit 1 bis 3 Halogenatomen, C₃-C₆-Cycloalkyl oder Halogen;
R₂ Wasserstoff, C₁-C₅ Alkyl; C₁-C₃ Halogenalkyl mit 1 bis 3 Halogenatomen; C₃-C₅ Alkoxyalkyl; Nitro, Cyano, S(O)ₚ-C₁-C₄-Alkyl, Amino, NHR₃, N(R₃)R₉, N=C(R₉)R₁₀ oder Halogen;
R₃ Wasserstoff; C₁-C₅ Alkyl, Benzyl, COR₆ oder SR₇;
R₄ und R₈ unabhängig voneinander Wasserstoff; C₁-C₅ Alkyl; C₁-C₅ Halogenalkyl mit 1 bis 3 Halogenatomen; C₂-C₆ Alkoxyalkyl oder C₃-C₆ Cycloalkyl;
R₅ Halogen; C₁-C₃ Alkyl; C₁-C₃ Alkoxy; C₁-C₆ Alkoxyalkyl, C₁-C₃ Halogenalkyl, C₁-C₃ Alkylthio, C₁-C₃ Halogenalkoxy, C₁-C₃ Halogenalkylthio, Cyano oder Nitro oder eine am Naphthylring auftretende X-Phenylgruppe, die unsubstituiert oder ein- bis dreifach gleich oder verschieden durch Halogen, C₁-C₃-Alkyl oder C₁-C₃-Alkoxy substituiert ist;
R₆ C₁-C₅ Alkyl, Phenyl, durch Halogen und/oder C₁-C₃ Alkyl substituiertes Phenyl;
R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituierter Benzyl, oder C₁-C₅ Alkyl oder durch Halogen oder Cyano substituiertes C₁-C₅ Alkyl;
R₉ C₁-C₅ Alkyl;
R₁₀ Wasserstoff oder C₁-C₅ Alkyl;
R₁₃ Wasserstoff;
X Sauerstoff oder Schwefel
m 2 oder 3,
n 0, 1, 2 oder 3; und
p 0, 1 oder 2.

12. Verfahren gemäss Anspruch 11, worin bedeuten:
R₁ Wasserstoff, C₁-C₃ Alkyl; C₁-C₃ Halogenalkyl mit 1 bis 3 Halogenatomen; C₂-C₅ Alkoxyalkyl; C₂-C₅ Alkenyl; C₃-C₆-Cycloalkyl oder Halogen;
R₂ Wasserstoff, C₁-C₅ Alkyl; C₁-C₃ Halogenalkyl mit 1 bis 3 Halogenatomen; C₃-C₅ Alkoxyalkyl; Nitro, Cyano, S(O)ₚ-C₁-C₃-Alkyl, Amino, NHR₃, N(R₃)R₉, N=C(R₉)R₁₀ oder Halogen;
R₃ Wasserstoff; C₁-C₃ Alkyl;
R₄ und R₈ unabhängig voneinander Wasserstoff; C₁-C₅ Alkyl; CF₃; C₂-C₆ Alkoxyalkyl oder C₃-C₆ Cycloalkyl;
R₅ Halogen; C₁-C₃ Alkyl; C₁-C₃ Alkoxy; C₂-C₆ Alkoxyalkyl, C₁-C₃ Halogenalkyl, C₁-C₃ Alkylthio, C₁-C₃ Halogenalkoxy, C₁-C₃ Halogenalkylthio, Cyano oder Nitro; R₆ C₁-C₅ Alkyl, Phenyl, durch Halogen und/oder C₁-C₃ Alkyl substituiertes Phenyl;
R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituiertes Benzyl, oder C₁-C₅ Alkyl oder durch Halogen oder Cyano substituiertes C₁-C₅ Alkyl;
R₉ C₁-C₅ Alkyl;
R₁₀ Wasserstoff oder C₁-C₅ Alkyl;
R₁₃ Wasserstoff;
X Sauerstoff oder Schwefel
m 2 oder 3,
n 0, 1, 2 oder 3; und
p 0, 1 oder 2.

13. Verfahren gemäss Anspruch 11, worin bedeuten:
R₁ Wasserstoff, C₁-C₃ Alkyl; CF₃, C₂-C₅ Alkoxyalkyl; C₂-C₅ Alkenyl; C₃-C₆-Cycloalkyl oder Halogen;
R₂ Wasserstoff, C₁-C₅ Alkyl; CF₃, C₃-C₅ Alkoxyalkyl; Nitro, Cyano, S(O)ₚ-C₁-C₄-Alkyl, Amino, N(R₃)R₉, N=C(R)R_{R0} oder Halogen;
R₃ Wasserstoff;
R₄ und R₈ unabhängig voneinander Wasserstoff; C₁-C₅ Alkyl; C₂-C₆ Alkoxyalkyl oder C₃-C₆ Cycloalkyl;
R₅ Halogen; C₁-C₃ Alkyl; C₁-C₃ Alkoxy; C₂-C₆ Alkoxyalkyl, Halogenalkyl, C₁-C₃ Alkylthio, C₁-C₃ Halogenalkoxy, C₁-C₃ Halogenalkylthio, Nitro oder Cyano;
R₆ C₁-C₅ Alkyl, Phenyl oder durch Halogen und/oder C₁-C₃ Alkyl substituiert ist;
R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituiertes Benzyl, oder C₁-C₅ Alkyl oder durch Halogen oder Cyano substituiertes C₁-C₅ Alkyl;
R₉ C₁-C₃ Alkyl;
R₁₀ Wasserstoff oder C₁-C₃ Alkyl;
R₁₃ Wasserstoff;
X Sauerstoff oder Schwefel
m 2;
n 0, 1, 2 oder 3; und
p 0, 1 oder 2.

14. Verfahren gemäss Anspruch 13, worin bedeuten:
R₁ Wasserstoff, C₁-C₃ Alkyl; CF₃, C₂-C₅ Alkoxyalkyl; C₂-C₅ Alkenyl; C₃-C₆-Cycloalkyl oder Halogen;
R₂ Wasserstoff, C₁-C₅ Alkyl; CF₃, C₃-C₅ Alkoxyalkyl; Nitro, Cyano, S(O)ₚ-C₁-C₃-Alkyl, Amino, N(R₃)R₉, N=C(R₉)R₁₀ oder Halogen;
R₃ Wasserstoff;
R₄ und R₈ unabhängig voneinander Wasserstoff; C₁-C₅ Alkyl; C₂-C₆ Alkoxyalkyl oder C₃-C₆ Cycloalkyl;
R₅ Halogen; C₁-C₃ Alkyl; C₁-C₃ Alkoxy; C₂-C₆ Alkoxyalkyl, C₁-C₃ Halogenalkyl, C₁-C₃ Alkylthio, C₁-C₃ Halogenalkoxy, C₁-C₃ Halogenalkylthio, Nitro oder Cyano;
R₆ C₁-C₅ Alkyl, Phenyl oder durch Halogen und/oder C₁-C₃ Alkyl substituiertes Phenyl;
R₇ Phenyl, durch Halogen, Nitro oder Cyano ein- oder zweifach gleich oder verschieden substituiertes Phenyl, Benzyl, durch Halogen, Nitro oder Cyano ein- oder zweifach gleich oder verschieden im Ring substituiertes Benzyl, oder C₁-C₅ Alkyl oder durch Halogen oder Cyano substituiertes C₁-C₅ Alkyl;
R₉ C₁-C₅ Alkyl;
R₁₀ Wasserstoff oder C₁-C₃ Alkyl;
R₁₃ Wasserstoff;
X Sauerstoff oder Schwefel m 3,
n 0, 1 , 2 oder 3; und
p 0, 1 oder 2.

15. Verfahren gemäss Anspruch 1, worin eine Verbindung der Formel Ia appliziert wird, 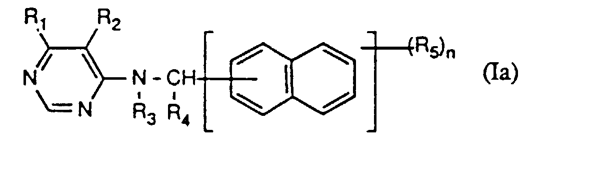 in welcher bedeuten:
R₁ C₁-C₅ Alkyl, das unsubstituiert oder durch Halogen oder C₁-C₃ Alkoxy substituiert ist; C₂-C₇ Alkenyl, das unsubstituiert oder durch Halogen substituiert ist; C₃-C₇ Cycloalkyl; oder Halogen;
R₂ C₁-C₅ Alkyl, das unsubstituiert oder durch Halogen oder C₁-C₃ Alkoxy substiutiert ist; Halogen; Nitro oder Cyano;
R₃ Wasserstoff; C₁-C₅ Alkyl; Benzyl; -CO-R₆ oder -S-R₇;
R₄ Wasserstoff; C₁-C₅ Alkyl, das unsubstituiert oder durch Halogen oder C₁-C₃ Alkoxy substituiert ist; oder C₃-C₇ Cycloalkyl;
R₅ Halogen; C₁-C₃ Alkyl; C₁-C₃ Alkoxy; C₁-C₃Alkylthio; oder eine einmal am Phenylring auftretende -X-Phenyl-Gruppe, die unsubstituiert oder ein- bis dreifach gleich oder verschieden durch Halogen, C₁-C₃ Alkyl oder C₁-C₃ Alkoxy substituiert ist;
R₆ C₁-C₅ Alkyl; Phenyl, das unsubstituiert oder durch Halogen und/oder C₁-C₃ Alkyl substituiert ist;
R₇ Phenyl oder Benzyl, die unsubstituiert oder im Ring durch Halogen, Nitro, Cyano ein- bis zweifach gleich oder verschieden substituiert sind; oder C₁-C₅ Alkyl, das unsubstituiert oder durch Halogen oder Cyano substituiert ist;
X Sauerstoff oder Schwefel; und
n 0, 1, 2 oder 3.

16. Verfahren gemäss Anspruch 1, worin eine Verbindung der Formel I aus der Gruppe:
(d,l)-4-[1'-(β-Naphthyl)-ethylamino]-5-chlor-6-ethylpyrimidin (Verb. 1.2);
(-)-4-[1'-(β-Naphthyl)-ethylamino]-5-chlor-6-ethylpyrimidin (Verb. 1.73);
(d,l)-4-[1'-(β-Naphthyl)-propylamino]-5-chlor-6-ethylpyrimidin (Verb. 1.3);
(d,l)-4-[1'-(β-Naphthyl)-ethylamino]-5-chlor-6-methylpyrimidin (Verb. 1.53);
(d,l)-4-[1'-(2-(6-Bromnaphthyl))-ethylamino]-5chlor-6-ethylpyrimldin (Verb. 1.149);
(d,l)-4-[1'-(β-Naphthyl)-ethylamino]-5-brom-6-ethylpyrimidin (Verb. 1.84);
(d,l)-4-[1'-(β-Naphthyl)-ethylamino]-5-chlor-6-n-propylpyrimidin (Verb. 1.86);
(d,l)-4-[1'-(β-Naphthyl)-ethylamino]-5-jod-6-ethylpyrimidin (Verb. 1.100) appliziert wird.

17. Verfahren zur Herstellung der Verbindungen der Formel I gemäß Anspruch 1, dadurch gekennzeichnet, dass man eine Verbindung der Formel II 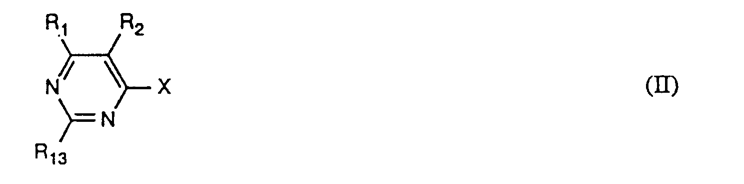 worin R₁, R₂ und R₁₃ die für Formel I angegebene Bedeutung haben und X einen leicht abspaltbaren Rest bedeutet, mit einer Verbindung der Formel III 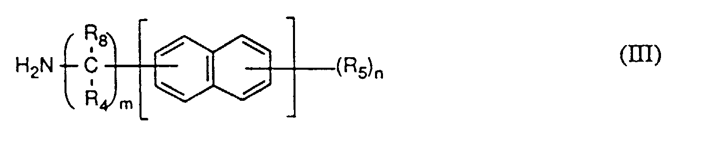 worin R₄, R₅, R₈, m und n die für Formel I gegebene Bedeutung haben, zur Reaktion bringt, vorzugsweise in Gegenwart einer Base, und sofern gewünscht, anschliessend den Substituenten R₃, sofern dieser Substituent Alkyl, Benzyl, Acyl oder -S-R₇ bedeutet, durch entsprechende N-Alkylierung, N-Benzylierung, N-Acylierung oder N-Thioalkylierung bzw. Thioarylierung einführt.

## Claims (Claims for the following Contracting State(s): CH, DE, DK, FR, GB, IT, LI, NL)

1. A compound of formula I 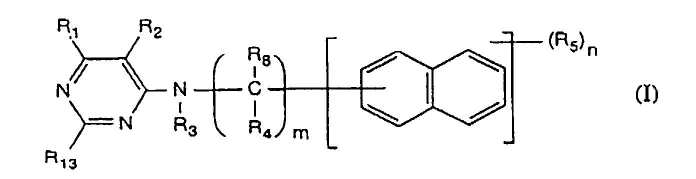 wherein:
R₁ is hydrogen; C₁-C₅alkyl that is unsubstituted or substituted by halogen or by C₁-C₃alkoxy or by S(O)ₚ-C₁-C₃alkyl; C₂-C₇alkenyl that is unsubstituted or substituted by halogen; C₃-C₇cycloalkyl; halogen: or C₂-C₄alkynyl;
R₂ is hydrogen; hydroxy; C₁-C₅alkyl that is unsubstituted or substituted by halogen or by C₁-C₃alkoxy; C₁-C₄alkoxy; S(O)ₚ-C₁-C₄alkyl; halogen; nitro; cyano; NHR₃; N(R₃)R₉; or N=C(R₉)R₁₀;
R₃ is hydrogen; C₁-C₅alkyl; benzyl; -CO-R₆; or -S-R₇;
R₄ and R₈ are each independently of the other hydrogen; C₁-C₅alkyl that is unsubstituted or substituted by halogen or by C₁-C₃alkoxy; or C₃-C₇cycloalkyl;
R₅ is halogen; C₁-C₃alkyl; C₁-C₃alkoxy; C₂-C₆alkoxyalkyl; C₁-C₃haloalkyl; C₁-C₃alkylthio; C₁-C₃haloalkoxy; C₁-C₃haloalkylthio; CN; NO₂; or an -X-phenyl group that occurs once at the naphthyl ring and that is unsubstituted or mono- to tri-substituted by identical or different substituents selected from halogen, C₁-C₃alkyl and C₁-C₃alkoxy;
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
R₉ is C₁-C₅alkyl;
R₁₀ is hydrogen or C₁-C₅alkyl;
R₁₃ is hydrogen; C₁-C₄alkyl that is unsubstituted or substituted by halogen or by C₁-C₃alkoxy; cyclopropyl; halogen; C₁-C₃alkoxy; C₁-C₃alkylthio; or -N(C₁-C₃alkyl)₂;
X is oxygen or sulfur;
m is 1, 2 or 3;
n is 0, 1, 2 or 3; and
p is 0, 1 or 2.

2. A compound of formula I according to claim 1 wherein:
R₁ is hydrogen; C₁-C₅alkyl; C₁-C₅alkyl substituted by S(O)ₚ-C₁-C₃alkyl; C₁-C₅haloalkyl having from 1 to 5 halogen atoms; C₂-C₆alkoxyalkyl; C₂-C₇alkenyl; C₂-C₇haloalkenyl having 1 or 2 halogen atoms; C₃-C₆cycloalkyl; halogen; or C₂-C₄alkynyl;
R₂ is hydrogen; C₁-C₅alkyl; C₁-C₅haloalkyl having from 1 to 5 halogen atoms; C₂-C₆alkoxyalkyl; nitro; cyano; S(O)ₚ-C₁-C₃alkyl; amino; NHR₃; N(R₃)R₉; N=C(R₉)R₁₀; or halogen;
R₃ is hydrogen; C₁-C₅alkyl; benzyl; -CO-R₆; or -S-R₇;
R₄ and R₈ are each independently of the other hydrogen; C₁-C₅alkyl; C₁-C₅haloalkyl having from 1 to 3 halogen atoms; C₂-C₆alkoxyalkyl; or C₃-C₆cycloalkyl;
R₅ is halogen; C₁-C₃alkyl; C₁-C₃alkoxy; C₁-C₆alkoxyalkyl; C₁-C₃haloalkyl; C₁-C₃alkylthio; C₁-C₃haloalkoxy; C₁-C₃haloalkylthio; CN; NO₂; or an X-phenyl group that occurs at the naphthyl ring and that is unsubstituted or mono- to tri-substituted by identical or different substituents selected from halogen, C₁-C₃alkyl and C₁-C₃alkoxy;
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
X is oxygen or sulfur;
R₉ is C₁-C₅alkyl;
R₁₀ is hydrogen or C₁-C₅alkyl;
m is 1, 2 or 3;
n is 0, 1, 2 or 3; and
p is 0, 1 or 2.

3. A compound of formula I according to claim 1 wherein:
R₁ is hydrogen; C₁-C₅alkyl substituted by S(O)ₚ-C₁-C₃alkyl; C₁-C₅alkyl; C₁-C₃haloalkyl having from 1 to 3 halogen atoms; C₂-C₅alkoxyalkyl; C₂-C₅alkenyl; C₂-C₅haloalkenyl having from 1 to 3 halogen atoms; C₃-C₅cycloalkyl; halogen; or C₂-C₄alkynyl;
R₂ is hydrogen; C₁-C₅alkyl; C₁-C₃haloalkyl having from 1 to 3 halogen atoms; C₂-C₅alkoxyalkyl; nitro; cyano; S(O)ₚ-C₁-C₃alkyl; amino; NHR₃; N(R₃)R₉; N=C(R₉)R₁₀; or halogen;
R₃ is hydrogen; C₁-C₅alkyl; benzyl; -CO-R₆; or -S-R₇;
R₄ and R₈ are each independently of the other hydrogen; C₁-C₅alkyl; C₁-C₅haloalkyl having from 1 to 3 halogen atoms; C₂-C₅alkoxyalkyl; or C₃-C₆cycloalkyl;
R₅ is halogen; C₁-C₃alkyl; C₁-C₃alkoxy; C₁-C₆alkoxyalkyl; C₁-C₃haloalkyl; C₁-C₃alkylthio; C₁-C₃haloalkoxy; C₁-C₃haloalkylthio; CN; NO₂; or an X-phenyl group that occurs at the naphthyl ring and that is unsubstituted or mono- to tri-substituted by identical or different substituents selected from halogen, C₁-C₃alkyl and C₁-C₃alkoxy;
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
X is oxygen or sulfur;
R₉ is C₁-C₅alkyl;
R₁₀ is hydrogen or C₁-C₅alkyl;
m is 1, 2 or 3;
n is 0, 1, 2 or 3; and
p is 0, 1 or 2.

4. A compound of formula I' 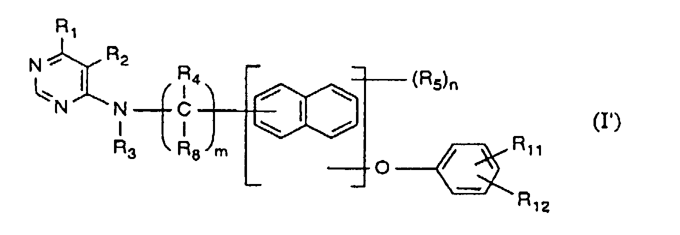 wherein:
R₁ is hydrogen; C₁-C₅alkyl; C₁-C₅alkyl substituted by S(O)ₚ-C₁-C₃alkyl; C₁-C₃haloalkyl having from 1 to 3 halogen atoms; C₂-C₅alkoxyalkyl; C₂-C₅alkenyl; C₂-C₅haloalkenyl having from 1 to 3 halogen atoms; C₃-C₆cycloalkyl; halogen; or C₂-C₄alkynyl;
R₂ is hydrogen; C₁-C₅alkyl; C₁-C₃haloalkyl having from 1 to 3 halogen atoms; C₃-C₅alkoxyalkyl; nitro; cyano; S(O)ₚ-C₁-C₃alkyl; amino; NHR₃; N(R₃)R₉; N=C(R₉)R₁₀; or halogen;
R₃ is hydrogen; C₁-C₅alkyl; benzyl; -CO-R₆; or -S-R₇;
R₄ and R₈ are each independently of the other hydrogen; C₁-C₅alkyl; C₁-C₅haloalkyl having from 1 to 3 halogen atoms; C₂-C₆alkoxyalkyl; or C₃-C₆cycloalkyl; C₂-C₆alkoxyalkyl; or C₃-C₆cycloalkyl;
R₅ is halogen; C₁-C₃alkyl; C₁-C₃alkoxy; C₁-C₃haloalkyl; or C₁-C₃haloalkoxy;
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
R₉ is C₁-C₅alkyl;
R₁₀ is hydrogen or C₁-C₅alkyl;
R₁₁ and R₁₂ are each independently of the other hydrogen; halogen; or C₁-C₃alkyl; m is 1, 2 or 3;
n is 0, 1 or 2; and
p is 0, 1 or 2.

5. A compound of formula I' according to claim 4 wherein:
R₁ to R₄ and R₈ are as defined under formula I;
R₅ is halogen, C₁-C₃alkyl, CF₃, C₂-C₅alkoxyalkyl or C₁-C₃alkoxy;
R₁₁ and R₁₂ are each independently of the other hydrogen, halogen or C₁-C₃alkyl ;
m is 1, 2 or 3; and
n is 0, 1 or 2.

6. A compound of formula I according to claim 1 wherein:
R₁ is hydrogen; C₁-C₅alkyl; C₁-C₅haloalkyl having from 1 to 3 halogen atoms; C₂-C₅alkoxyalkyl; C₂-C₅alkenyl; C₂-C₅haloalkenyl; or halogen;
R₂ is hydrogen; C₁-C₅alkyl; C₁-C₃haloalkyl having from 1 to 3 halogen atoms; C₃-C₅alkoxyalkyl; nitro; cyano; S(O)ₚ-C₁-C₄alkyl; amino; NHR₃; N(R₃)R₉; N=C(R₉)R₁₀; or halogen;
R₃ is hydrogen; C₁-C₃alkyl; benzyl; -CO-R₆; or SR₇;
R₄ and R₈ are each independently of the other hydrogen; C₁-C₅alkyl; C₁-C₅haloalkyl having from 1 to 3 halogen atoms; C₂-C₆alkoxyalkyl; or C₃-C₆cycloalkyl;
R₅ is halogen; C₁-C₃alkyl; C₁-C₃alkoxy; C₁-C₆alkoxyalkyl; C₁-C₃haloahkyl; C₁-C₃alkylthio; C₁-C₃haloalkoxy; C₁-C₃haloalkylthio; cyano; or nitro;
R₆ is C₁-C₅alkyl; phenyl; or phenyl substituted by halogen and/or by C₁-C₃alkyl; R₇ is phenyl; phenyl mono- or di-substitutcd by halogen, nitro or by cyano; benzyl; benzyl mono- or di-substituted in the ring by halogen, nitro or by cyano; C₁-C₅alkyl; or C₁-C₅-alkyl substituted by halogen or by cyano;
R₉ is C₁-C₅alkyl;
R₁₀ is hydrogen or C₁-C₃alkyl;
R₁₃ is hydrogen or C₁-C₄alkyl;
m is 1, 2 or 3;
n is 0, 1 or 2; and
p is 0,1 or 2.

7. A compound according to claim 6 wherein:
R₁ is C₁-C₅alkyl; CF₃; C₂-C₅alkoxyalkyl; C₂-C₄alkenyl; C₂-C₄monohaloalkenyl; or halogen;
R₂ is C₁-C₅alkyl or halogen;
R₃ is hydrogen or C₁-C₃alkyl;
R₄ is hydrogen; C₁-C₃alkyl; or cyclopropyl;
R₅ is halogen; C₁-C₂alkyl; or C₁-C₃alkoxy;
R₈ is hydrogen;
n is 0, 1, 2 or 3; and
m is 1.

8. A compound according to claim 7 wherein:
R₁ is C₁-C₄alkyl; CF₃; C₂-C₄alkoxyalkyl; C₂-C₄alkenyl; or halogen;
R₂ is C₁-C₄alkyl or halogen;
R₃ is hydrogen or C₁-C₃alkyl;
R₄ is hydrogen or C₁-C₃alkyl;
R₅ is halogen; methyl; ethyl; or methoxy;
R₈ is hydrogen;
n is 0, 1, 2 or 3; and
m is 1.

9. A compound of formula I" 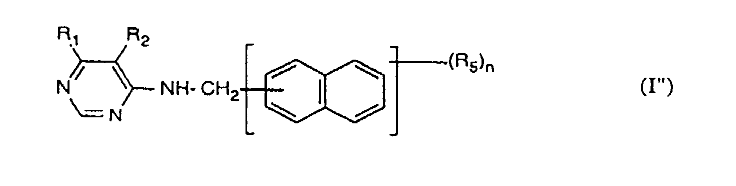 wherein:
R₁ is C₁-C4alkyl, CF₃, C₂-C₄alkoxyalkyl or halogen;
R₂ is C₁-C₄alkyl or halogen;
R₅ is chlorine, bromine, methyl, ethyl or methoxy; and
n is 0, 1 or 2.

10. A compound of formula I"' 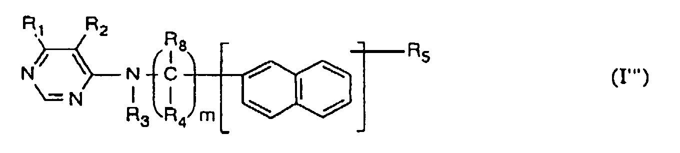 wherein:
R₁ is hydrogen, C₁-C₃alkyl, CF₃ or halogen;
R₂ is hydrogen, C₁-C₃alkyl, halogen, NO₂ or S(O)ₚ-C₁-C₃alkyl;
R₃ is hydrogen;
R₄ is methyl, ethyl, isopropyl, n-propyl or cyclopropyl;
R₈ is hydrogen, methyl or ethyl;
R₅ is hydrogen, halogen, C₁-C₃alkyl, OCHF₂, CF₃, NO₂ or C₁-C₃alkoxy;
m is 1 or 2; and
p is 0, 1 or 2.

11. A compound according to claim 3 wherein:
R₁ is hydrogen; C₁-C₅alkyl; C₁-C₃haloalkyl having from 1 to 3 halogen atoms; C₂-C₅alkoxyalkyl; C₂-C₅alkenyl; C₂-C₅haloalkenyl having from 1 to 3 halogen atoms; C₃-C₆cycloalkyI; or halogen;
R₂ is hydrogen; C₁-C₅alkyl; C₁-C₃haloalkyl having from 1 to 3 halogen atoms; C₃-C₅alkoxyalkyl; nitro; cyano; s(O)ₚ-C₁-C₄alkyl; amino; NHR₃; N(R₃)R₉; N=C(R₉)R₁₀; or halogen;
R₃ is hydrogen; C₁-C₅alkyl; benzyl; COR₆; or SR₇;
R₄ and R₈ are each independently of the other hydrogen; C₁-C₅alkyl; C₁-C₅haloalkyl having from 1 to 3 halogen atoms; C₂-C₆alkoxyalkyl; or C₃-C₆cycloalkyl;
R₅ is halogen; C₁-C₃alkyl; C₁-C₃alkoxy; C₁-C₆alkoxyalkyl; C₁-C₃haloalkyl; C₁-C₃alkylthio; C₁-C₃haloalkoxy; C₁-C₃haloalkylthio; cyano; nitro; or an X-phenyl group that occurs at the naphthyl ring and that is unsubstituted or mono- to tri-substituted by identical or different substituents selected from halogen, C₁-C₃alkyl and C₁-C₃alkoxy;
R₆ is C₁-C₅alkyl; phenyl; or phenyl substituted by halogen and/or by C₁-C₃alkyl;
R₇ is phenyl; phenyl mono- or di-substituted by identical or different substituents selected from halogen, nitro and cyano; benzyl; benzyl mono- or di-substituted in the ring by identical or different substituents selected from halogen, nitro and cyano: C₁-C₅alkyl; or C₁-C₅alkyl substituted by halogen or by cyano:
R₉ is C₁-C₅alkyl;
R₁₀ is hydrogen or C₁-C₅alkyl;
R₁₃ is hydrogen;
X is oxygen or sulfur;
m is 2 or 3;
n is 0, 1, 2 or 3; and
p is 0, 1 or 2.

12. A compound according to claim 11 wherein:
R₁ is hydrogen; C₁-C₃alkyl; C₁-C₃haloalkyl having from 1 to 3 halogen atoms: C₂-C₅alkoxyalkyl; C₂-C₅alkenyl; C₃-C₆cycloalkyl; or halogen;
R₂ is hydrogen; C₁-C₅alkyl; C₁-C₃haloalkyi having from 1 to 3 halogen atoms; C₃-C₅alkoxyalkyl; nitro; cyano; S(O)ₚ-C₁-C₃alkyl; amino; NHR₃; N(R₃)R₉; N=C(R₉)R₁₀; or halogen;
R₃ is hydrogen or C₁-C₃alkyl;
R₄ and R₈ are each independently of the other hydrogen; C₁-C₅alkyl; CF₃; C₂-C₆alkoxyalkyl; or C₃-C₆cycloalkyl;
R₅ is halogen; C₁-C₃alkyl; C₁-C₃alkoxy; C₂-C₆alkoxyalkyl; C₁-C₃haloalkyl; C₁-C₃alkylthio; C₁-C₃haloalkoxy; C₁-C₃haloalkylthio; cyano; or nitro;
R₆ is C₁-C₅alkyl; phenyl; or phenyl substituted by halogen and/or by C₁-C₃alkyl;
R₇ is phenyl; phenyl mono- or di-substituted by identical or different substituents selected from halogen, nitro and cyano; benzyl; benzyl mono- or di-substituted in the ring by identical or different substituents selected from halogen, nitro and cyano; C₁-C₅alkyl; or C₁-C₅alkyl substituted by halogen or by cyano;
R₉ is C₁-C₅alkyl;
R₁₀ is hydrogen or C₁-C₅alkyl;
R₁₃ is hydrogen;
X is oxygen or sulfur;
m is 2 or 3;
n is 0, 1, 2 or 3; and
p is 0, 1 or 2.

13. A compound according to claim 11 wherein:
R₁ is hydrogen; C₁-C₃alkyl; CF₃; C₂-C₅alkoxyalkyl; C₂-C₅alkenyl; C₁-C₆cycloalkl; or halogen;
R₂ is hydrogen; C₁-C₅alkyl; CF₃; C₃-C₅alkoxyalkyl; nitro; cyano; S(O)ₚC₁-C₄alkyl; amino; N(R₃)R₉; N=C(R₉)R₁₀; or halogen;
R₃ is hydrogen;
R₄ and R₈ are each independently of the other hydrogen; C₁-C₅alkyl; C₂-C₆alkoxyalkyl; or C₃-C₆cycloalkyl;
R₅ is halogen; C₁-C₃alkyl; C₁-C₃alkoxy; C₂-C₆alkoxyalkyl; haloalkyl; C₁-C₃alkylthio; C₁-C₃haloalkoxy; C₁-C₃haloalkylthio; nitro; or cyano;
R₆ is C₁-C₅alkyl; phenyl; or substituted by halogen and/or by C₁-C₃alkyl;
R₇ is phenyl; phenyl mono- or di-substituted by identical or different substituents selected from halogen, nitro and cyano; benzyl; benzyl mono- or di-substituted in the ring by identical or different substituents selected from halogen, nitro and cyano; C₁-C₅alkyl; or C₁-C₅alkyl substituted by halogen or by cyano;
R₉ is C₁-C₃alkyl;
R₁₀ is hydrogen or C₁-C₃alkyl;
R₁₃ is hydrogen;
X is oxygen or sulfur;
m is 2;
n is 0, 1, 2 or 3; and
p is 0, 1 or 2.

14. A compound according to claim 13 wherein:
R₁ is hydrogen; C₁-C₃alkyl; CF₃; C₂-C₅alkoxyalkyl; C₂-C₅alkenyl; C₃-C₆cycloalkyl; or halogen;
R₂ is hydrogen; C₁-C₅alkyl; CF₃; C₃-C₅alkoxyalkyl; nitro; cyano; S(O)ₚ-C₁-C₃alkyl; amino; N(R₃)R₉; N=C(R₉)R₁₀; or halogen;
R₃ is hydrogen;
R₄ and R₈ are each independently of the other hydrogen; C₁-C₅alkyl; C₂-C₆alkoxyalkyl; or C₃-C₆cycloalkyl;
R₅ is halogen; C₁-C₃alkyl; C₁-C₃alkoxy; C₂-C₆alkoxyalkyl; C₁-C₃haloalkyl; C₁-C₃alkylthio; C₁-C₃haloalkoxy; C₁-C₃haloalkylthio; nitro; or cyano;
R₆ is C₁-C₅alkyl; phenyl; or phenyl substituted by halogen and/or by C₁-C₃alkyl;
R₇ is phenyl; phenyl mono- or di-substituted by identical or different substituents selected from halogen, nitro and cyano; benzyl; benzyl mono- or di-substituted in the ring by identical or different substituents selected from halogen, nitro and cyano; C₁-C₅alkyl; or C₁-C₅alkyl substituted by halogen or by cyano;
R₉ is C₁-C₅alkyl;
R₁₀ is hydrogen or C₁-C₃alkyl;
R₁₃ is hydrogen;
X is oxygen or sulfur;
m is 3;
n is 0, 1, 2 or 3; and
p is 0, 1 or 2.

15. A compound of formula Ia 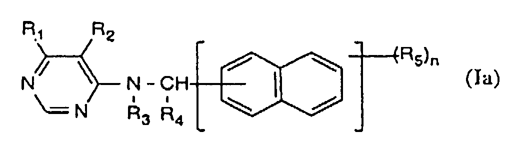 wherein:
R₁ is C₁-C₃alkyl that is unsubstituted or substituted by halogen or by C₁-C₃alkoxy; C₂-C₇alkenyl that is unsubstituted or substituted by halogen; C₃-C₇cycloalkyl; or halogen; R₂ is C₁-C₅alkyl that is unsubstituted or substituted by halogen or by C₁-C₃alkoxy; halogen; nitro; or cyano;
R₃ is hydrogen; C₁-C₅alkyl; benzyl; -CO-R₆; or -S-R₇;
R₄ is hydrogen; C₁-C₅alkyl that is unsubstituted or substituted by halogen or by C₁-C₃alkoxy; or C₃-C₇cycloalkyl;
R₅ is halogen; C₁-C₃alkyl; C₁-C₃alkoxy; C₁-C₃alkylthio; or an -X-phenyl group that occurs once at the phenyl ring and that is unsubstituted or mono- to tri-substituted by identical or different substituents selected from halogen, C₁-C₃alkyl and C₁-C₃alkoxy;
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
X is oxygen or sulfur; and
n is 0, 1, 2 or 3.

16. A compound of formula I according to claim 1 from the group:
(d,l)-4-[1'-(β-naphthyl)-ethylamino]-5-chloro-6-ethylpyrimidine (comp. 1.2);
(-)-4-[1"(β-naphthyl)-ethylamino]-5-chloro-6-ethylpyrimidine (comp. 1.73);
(d,l)-4-[1'-(β-naphthyl)-propylamino]-6-chloro-6-ethylpyrimidine (comp. 1.3);
(d,l)-4-[1'-(β-naphthyl)-ethylamino)-5-chloro-6-methylpydinidine (comp. 1.53);
(d,l)-4-[1'-(2-(6-bromonaphthyl))-ethylamino]-5-chloro-6-ethylpyrimidine (comp. 1.149);
(d,l)-4-[1'-(β-naphthyl)-ethylamino]-5-bromo-6-ethylpyrimidine (comp. 1 .84);
(d,l)-4-[1'-(β-naphthyl)-ethylamino]-5-chloro-6-n-propylpyrimidine (comp. 1.86);
(d,l)-4-[1'-((3-naphthyl)-ethylamino]-5-iodo-6-ethylpyrimidine (comp. 1.100).

17. A process for the preparation of a compound of formula I according to claim 1, which comprises reacting a compound of formula II 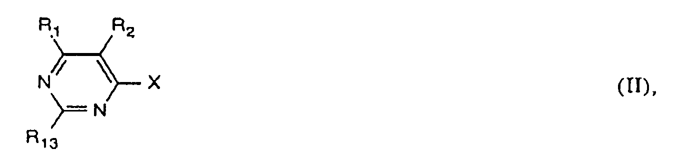 wherein R₁, R₂ and R₁₃ are as defined for formula I and X is a readily removable radical, with a compound of formula III 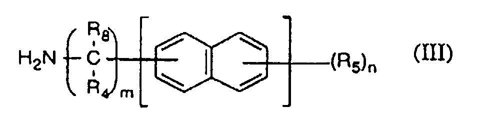 wherein R₄, R₅, R₈, m and n arc as defined for formula I, preferably in the presence of a base, and, if desired, then introducing the substituent R₃ (where that substituent is alkyl, benzyl, acyl or -S-R₇) by corresponding N-alkylation, N-benzylation, N-acylation or N-thioalkylation or N-thioarylation, respectively.

18. A composition for protecting plants against attack by pests, which comprises as active ingredient at least one compound according to claim 1 and a carrier.

19. A composition according to claim 18 that comprises as active ingredient at least one compound according to claims 2 to 15.

20. A composition according to claim 18 that comprises as active ingredient a compound of formula I according to claim 16.

21. A process for the preparation of an agrochemical composition according to claim 18, which comprises homogeneously mixing at least one compound defined according to claim 1 with suitable solid or liquid carriers and adjuvants.

22. The use of a compound according to claim 1 for protecting plants against attack by phytopathogenic microorganisms.

23. The use of a compound according to any one of claims 2 to 16 for protecting plants against attack by phytopathogenic microorganisms.

24. A method of protecting plants against attack by phytopathogenic microorganisms, which comprises applying a compound according to claim 1 as active ingredient to the plants or to the locus thereof.

25. A method of protecting plants against attack by phytopathogenic microorganisms, which comprises applying a compound according to any one of claims 2 to 16 as active ingredient to the plants or to the locus thereof.

26. A method according to claim 25 wherein the phytopathogenic microorganisms are fungus organisms.

## Claims (Claims for the following Contracting State(s): ES)

1. A method of protecting plants against attack by phytopathogenic microorganisms which comprises applying as active ingredient to the plants or to the locus thereof a compound of formula I 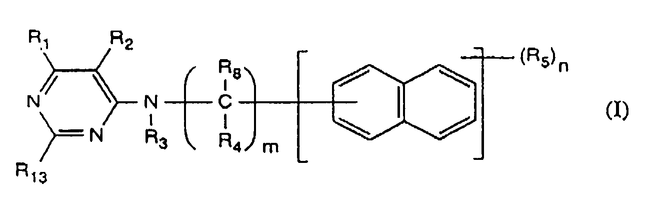 wherein:
R₁ is hydrogen; C₁-C₅alkyl that is unsubstituted or substituted by halogen or by C₁-C₃alkoxy or by S(O)ₚ-C₁-C₃alkyl; C₂-C₇alkenyl that is unsubstituted or substituted by halogen; C₃-C₇cycloalkyl; halogen; or c₂-c₄alkynyl;
R₂ is hydrogen; hydroxyl C₁-C₅alkyl that is unsubstituted or substituted by halogen or by C₁-C₃alkoxy; C₁-C₄alkoxy; S(O)ₚ-C₁-C₄alkyl; halogen; nitro; cyano; NHR₃; N(R₃)R₉; or N=C(R₉)R₁₀;
R₃ is hydrogen; C₁-C₅alkyl; benzyl; -CO-R₆; or -S-R₇;
R₄ and R₈ are each independently of the other hydrogen; C₁-C₅alkyl that is unsubstituted or substituted by halogen or by C₁-C₃alkoxy; or C₃-C₇cycloalkyl;
R₅ is halogen; C₁-C₃alkyl; C₁-C₃alkoxy; C₂-C₆alkoxyalkyl; C₁-C₃haloalkyl; C₁-C₃alkylthio; C₁-C₃haloalkoxy; C₁-C₃haloalkylthio; CN; NO₂; or an -X-phenyl group that occurs once at the naphthyl ring and that is unsubstituted or mono- to tri-substituted by identical or different substituents selected from halogen, C₁-C₃alkyl and C₁-C₃alkoxyl
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
R₉ is C₁-C₅alkyl;
R₁₀ is hydrogen or C₁-C₅alkyl;
R₁₃ is hydrogen; C₁-C₄alkyl that is unsubstituted or substituted by halogen or by
C₁-C₃alkoxy; cyclopropyl; halogen; C₁-C₃alkoxy; C₁-C₃alkylthio; or -N(C₁-C₃alkyl)₂; X is oxygen or sulfur;
m is 1, 2 or 3;
n is 0, 1, 2 or 3; and
p is 0,1or 2.

2. A method according to claim 1 wherein:
R₁ is hydrogen; C₁-C₅alkyl; C₁-C₅alkyl substituted by S(O)ₚ-C₁-C₃alkyl; C₁-C₅haloalkyl having from 1 to 5 halogen atoms; C₂-C₆alkoxyalkyl; C₂-C₇alkenyl; C₂-C₇haloalkenyl having 1 or 2 halogen atoms; C₃-C₆cycloalkyl; halogen; or C₂-C₄alkynyl;
R₂ is hydrogen; C₁-C₅alkyl; C₁-C₅haloalkyl having from 1 to 5 halogen atoms; C₂-C₆alkoxyalkyl; nitro; cyano; S(O)ₚ-C₁-C₃alkyl; amino; NHR₃; N(R₃)R₉; N=C(R₉)R₁₀: or halogen;
R₃ is hydrogen; C₁-C₅alkyl; benzyl; -CO-R₆; or -S-R₇;
R₄ and R₈ are each independently of the other hydrogen; C₁-C₅alkyl; C₁-C₅haloalkyl having from 1 to 3 halogen atoms: C₂-C₆alkoxyalkyl; or C₃-C₆cycloalkyl;
R₅ is halogen; C₁-C₃alkyl; C₁-C₃alkoxy; C₁-C₆alkoxyalkyl; C₁-C₃haloalkyl; C₁-C₃alkylthio; C₁-C₃haloalkoxy; C₁-C₃haloahkylthio; CN; NO₂; or an X-phenyl group that occurs at the naphthyl ring and that is unsubstituted or mono- to tri-substituted by identical or different substituents selected from halogen, C₁-C₃alkyl and C₁-C₃alkoxy;
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
X is oxygen or sulfur;
R₉ is C₁-C₅alkyl;
R₁₀ is hydrogen or C₁-C₅alkyl;
m is 1, 2 or 3;
n is 0, 1, 2 or 3; and
p is 0, 1 or 2.

3. A method according to claim 1 wherein:
R₁ is hydrogen; C₁-C₅alkyl substituted by S(O)ₚ-C₁-C₃alkyl; C₁-C₅alkyl; C₁-C₃haloalkyl having from 1 to 3 halogen atoms; C₂-C₅alkoxyalkyl; C₂-C₅alkenyl; C₂-C₅haloalkenyl having from 1 to 3 halogen atoms; C₃-C₅cycloalkyl; halogen; or C₂-C₄alkynyl;
R₂ is hydrogen; C₁-C₅alkyl; C₁-C₃haloalkyl having from 1 to 3 halogen atoms; C₂-C₅alkoxyalkyl; nitro; cyano; S(O)ₚ-C₁-C₃alkyl; amino; NHR₃; N(R₃)R₉; N=C(R₉)R₁₀; or halogen;
R₃ is hydrogen; C₁-₅alkyl; benzyl; -CO-R₆; or -S-R₇;
R₄ and R₈ are each independently of the other hydrogen; C₁-C₅alkyl; C₁-C₅haloalkyl having from 1 to 3 halogen atoms; C₂-C₅alkoxyalkyl; or C₃-C₆cycloalkyl ;
R₅ is halogen; C₁-C₃alkyl; C₁-C₃alkoxy; C₁-C₆alkoxyalkyl; C₁-C₃haloalkyl; C₁-C₃alkylthio; C₁-C₃haloalkoxy; C₁-C₃haloalkylthio; CN; NO₂; or an X-phenyl group that occurs at the naphthyl ring and that is unsubstituted or mono- to tri-substituted by identical or different substituents selected from halogen, C₁-C₃alkyl and C₁-C₃alkoxy;
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
X is oxygen or sulfur;
R₉ is C₁-C₅alkyl;
R₁₀ is hydrogen or C₁-C₅alkyl;
m is 1, 2 or 3;
n is 0, 1, 2 or 3; and
p is 0, 1 or 2.

4. A method according to claim 1 which comprises applying a compound of formula I' 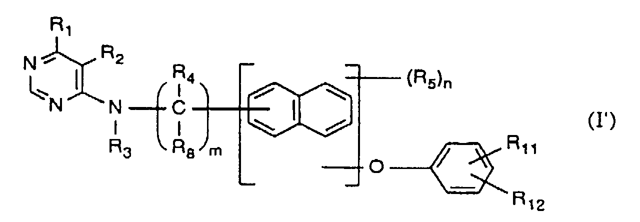 wherein:
R₁ is hydrogen; C₁-C₅alkyl; C₁-C₅alkyl substituted by S(O)ₚ-C₁-C₃alkyl; C₁-C₃haloalkyl having from 1 to 3 halogen atoms; C₂-C₅alkoxyalkyl; C₂-C₅alkenyl; C₂-C₃haloalkenyl having from 1 to 3 halogen atoms; C₃-C₆cycloalkyl; halogen; or C₂-C₄alkynyl;
R₂ is hydrogen; C₁-C₅alkyl; C₁-C₃haloalkyl having from 1 to 3 halogen atoms; C₃-C₅alkoxyalkyl; nitro; cyano; S(O)ₚ-C₁-C₃alkyl; amino; NHR₃; N(R₃)R₉; N=C(R₉)R₁₀; or halogen;
R₃ is hydrogen; C₁-C₅alkyl; benzyl; -CO-R₆; or -S-R₇;
R₄ and R₈ are each independently of the other hydrogen; C₁-C₅alkyl; C₁-C₅haloalkyl having from 1 to 3 halogen atoms; C₂-C₆alkoxyalkyl; or C₃-C₆cycloalkyl; C₂-C₆alkoxyalkyl; or C₃-C₆cycloalkyl;
R₅ is halogen; C₁-C₃alkyl; C₁-C₃alkoxy; C₁-C₃haloalkyl; or C₁-C₃haloalkoxy;
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
R₉ is C₁-C₅alkyl;
R₁₀ is hydrogen or C₁-C₅alkyl;
R₁₁ and R₁₂ are each independently of the other hydrogen; halogen ; or C₁-C₃alkyl;
m is 1, 2 or 3;
n is 0, 1 or 2; and
p is 0, 1 or 2.

5. A method according to claim 4 wherein:
R₁ to R₄ and R₈ are as defined under formula I;
R₅ is halogen, C₁-C₃alkyl, CF₃, C₂-C₅alkoxyalkyl or C₁-C₃alkoxy;
R₁₁ and R₁₂ are each independently of the other hydrogen, halogen or C₁-C₃alkyl;
m is 1, 2 or 3; and
n is 0, 1 or 2.

6. A method according to claim 1 wherein:
R₁ is hydrogen; C₁-C₅alkyl; C₁-C₅haloalkyl having from 1 to 3 halogen atoms; C₂-C₅alkoxyalkyl; C₂-C₅alkenyl; C₂-C₅haloalkenyl; or halogen;
R₂ is hydrogen; C₁-C₅alkyl; C₁-C₃haoalkyl having from 1 to 3 halogen atoms: C₃-C₅alkoxyalkyl; nitro; cyano; S(O)ₚ-C₁-C₄alkyl; amino; NHR₃; N(R₃)R₉; N=C(R₉)R₁₀; or halogen;
R₃ is hydrogen; C₁-C₃alkyl; benzyl; -CO-R₆; or SR₇;
R₄ and R₈ are each independently of the other hydrogen; C₁-C₅alkyl; C₁-C₅haloalkyl having from 1 to 3 halogen atoms; C₂-C₆alkoxyalkyl; or C₃-C₆cycloalkyl;
R₅ is halogen; C₁-C₃alkyl; C₁-C₃alkoxy; C₁-C₆alkoxyalkyl; C₁-C₃haloalkyl; C₁-C₃alkylthio; C₁-C₃haloalkoxy; C₁-C₃haloalkylthio; cyano; or nitro;
R₆ is C₁-C₅alkyl; phenyl; or phenyl substituted by halogen and/or by C₁-C₃alkyl;
R₇ is phenyl; phenyl mono- or di-substituted by halogen, nitro or by cyano; benzyl; benzyl mono- or di-substituted in the ring by halogen, nitro or by cyano; C₁-C₅alkyl; or C₁-C₅-alkyl substituted by halogen or by cyano;
R₉ is C₁-C₅alkyl;
R₁₀ is hydrogen or C₁-C₃alkyl;
R₁₃ is hydrogen or C₁-C₄alkyl;
m is 1, 2 or 3;
n is 0, 1 or 2; and
p is 0, 1 or 2.

7. A method according to claim 6 wherein:
R₁ is C₁-C₅alkyl: CF₃; C₂-C₅alkoxyalkyl; C₂-C₄alkenyl; C₂-C₄monohaloalkenyl; or halogen;
R₂ is C₁-C₅alkyl or halogen;
R₃ is hydrogen or C₁-C₃alkyl;
R₄ is hydrogen; C₁-C₃alkyl; or cyclopropyl;
R₅ is halogen; C₁-C₂alkyl; or C₁-C₃alkoxy;
R₈ is hydrogen;
n is 0, 1, 2 or 3; and
mis 1.

8. A method according to claim 7 wherein:
R₁ is C₁-C₄alkyl; CF₃; C₂-C₄alkoxyalkyl; C₂-C₄alkenyl; or halogen;
R₂ is C₁-C₄alkyl or halogen;
R₃ is hydrogen or C₁-C₃alkyl;
R₄ is hydrogen or C₁-C₃alkyl;
R₅ is halogen; methyl; ethyl; or methoxy;
R₈ is hydrogen;
n is 0, 1,2 or 3; and
mis 1.

9. A method according to claim I which comprises applying a compound of formula I" 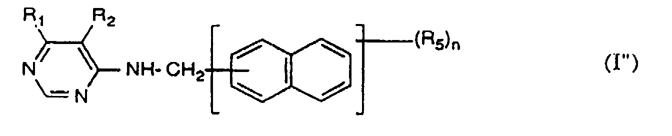 wherein:
R₁ is C₁-C₄alkyl, CF₃, C₂-C₄alkoxyalkyl or halogen;
R₂ is C₁-C₄alkyl or halogen;
R₅ is chlorine, bromine, methyl, ethyl or methoxy; and
n is 0, 1 or 2.

10. A method according to claim 1 which comprises applying a compound of formula I"' 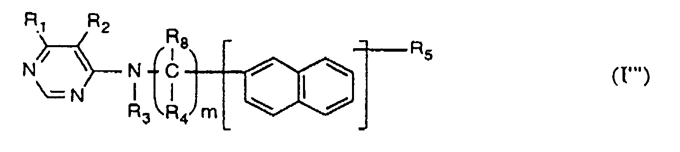 wherein:
R₁ is hydrogen, C₁-C₃alkyl, CF₃ or halogen;
R₂ is hydrogen, C₁-C₃alkyl, halogen, NO₂ or S(O)ₚ-C₁-C₃alkyl;
R₃ is hydrogen;
R₄ is methyl, ethyl, isopropyl, n-propyl or cyclopropyl;
R₈ is hydrogen, methyl or ethyl;
R₅ is hydrogen, halogen, C₁-C₃alkyl, OCH₂, CF₃*,* NO₂ or C₁-C₃alkoxy;
m is 1 or 2; and
p is 0,1 or 2.

11. A method according to claim 3 wherein:
R₁ is hydrogen; C₁-C₅alkyl; C₁-C₃haloalkyl having from 1 to 3 halogen atoms; C₂-C₅alkoxyalkyl; C₂-C₅alkenyl; C₂-C₅haloalkenyl having from I to 3 halogen atoms; C₃-C₆cycloalkyl; or halogen;
R₂ is hydrogen; C₁-C₅alkyl; C₁-C₃haloalkyl having from 1 to 3 halogen atoms; C₃-C₅alkoxyalkyl; nitro; cyano; S(O)ₚ-C₁-C₄alkyl; amino; NHR₃; N(R₃)R₉; N=C(R₉)R₁₀; or halogen;
R₃ is hydrogen; C₁-C₅alkyl; benzyl; COR₆; or SR₇;
R₄ and R₈ are each independently of the other hydrogen; C₁-C₅alkyl; C₁-C₅haloalkyl having from 1 to 3 halogen atoms; C₂-C₆alkoxyalkyl; or C₃-C₆cycloalkyl;
R₅ is halogen; C₁-C₃alkyl; C₁-C₃alkoxy; C₁-C₆alkoxyalkyl; C₁-C₃haloalkyl; C₁-C₃alkylthio; C₁-C₃haloalkoxy; C₁-C₃haloalkylthio; cyano; nitro; or an X-phenyl group that occurs at the naphthyl ring and that is unsubstituted or mono- to tri-substituted by identical or different substituents selected from halogen, C₁-C₃alkyl and C₁-C₃alkoxy;
R₆ is C₁-C₅alkyl; phenyl; or phenyl substituted by halogen and/or by C₁-C₃alkyl;
R₇ is phenyl; phenyl mono- or di-substituted by identical or different substituents selected from halogen, nitro and cyano; benzyl; benzyl mono- or di-substituted in the ring by identical or different substituents selected from halogen, nitro and cyano; C₁-C₅alkyl; or C₁-C₅alkyl substituted by halogen or by cyano;
R₉ is C₁-C₅alkyl;
R₁₀ is hydrogen or C₁-C₅alkyl;
R₁₃ is hydrogen;
X is oxygen or sulfur;
m is 2 or 3;
n is 0, 1, 2 or 3; and
p is 0, 1 or 2.

12. A method according to claim 11 wherein:
R₁ is hydrogen; C₁-C₃alkyl; C₁-C₃haloalkyl having from 1 to 3 halogen atoms; C₂-C₅alkoxyalkyl; C₂-C₅alkenyl; C₃-C₆cycloalkyl; or halogen;
R₂ is hydrogen; C₁-C₅alkyl; C₁-C₃haloalkyl having from 1 to 3 halogen atoms; C₃-C₅alkoxyalkyl; nitro; cyano; S(O)ₚ-C₁-C₃alkyl; amino; NHR₃; N(R₃)R₉; N=C(R₉)R₁₀; or halogen;
R₃ is hydrogen or C₁-C₃alkyl;
R₄ and R₈ are each independently of the other hydrogen; C₁-C₅alkyl; CF₃; C₂-C₆alkoxyalkyl; or C₃-C₆cycloalkyl;
R₅ is halogen; C₁-C₃alkyl; C₁-C₃alkoxy; C₂-C₆alkoxyalkyl; C₁-C₃haloalkyl; C₁-C₃alkylthio; C₁-C₃haloalkoxy; C₁-C₃haloalkylthio; cyano; or nitro;
R₆ is C₁-C₅alkyl; phenyl; or phenyl substituted by halogen and/or by C₁-C₃alkyl;
R₇ is phenyl; phenyl mono- or di-substituted by identical or different substituents selected from halogen, nitro and cyano; benzyl; benzyl mono- or di-substituted in the ring by identical or different substituents selected from halogen, nitro and cyano; C₁-C₅alkyl; or C₁-C₅alkyl substituted by halogen or by cyano;
R₉ is C₁-C₅alkyl;
R₁₀ is hydrogen or C₁-C₅alkyl;
R₁₃ is hydrogen;
X is oxygen or sulfur;
m is 2 or 3;
n is 0, 1, 2 or 3; and
p is 0, 1 or 2.

13. A method according to claim 11 wherein:
R₁ is hydrogen; C₁-C₃alkyl; CF₃; C₂-C₅alkoxyalkyl; C₂-C₅alkenyl; C₁-C₆cycloalkyl; or halogen;
R₂ is hydrogen; C₁-C₅alkyl; CF₃; C₃-C₅alkoxyalkyl; nitro; cyano; S(O)ₚ-C₁-C₄alkyl; amino; N(R₃)R₉; N=C(R₉)R₁₀; or halogen;
R₃ is hydrogen;
R₄ and R₈ are each independently of the other hydrogen; C₁-C₅alkyl; C₂-C₆alkoxyalkyl; or C₃-C₆cycloalkyl;
R₅ is halogen; C₁-C₃alkyl; C₁-C₃alkoxy; C₂-C₆alkoxyalkyl; haloalkyl; C₁-C₃alkylthio; C₁-C₃haloalkoxy; C₁-C₃haloalkylthio; nitro; or cyano;
R₆ is C₁-C₅alkyl; phenyl; or substituted by halogen and/or by C₁-C₃alkyl;
R₇ is phenyl; phenyl mono- or di-substituted by identical or different substituents selected from halogen, nitro and cyano; benzyl; benzyl mono- or di-substituted in the ring by identical or different substituents selected from halogen, nitro and cyano; C₁-C₅alkyl; or C₁-C₅alkyl substituted by halogen or by cyano;
R₉ is C₁-C₃alkyl;
R₁₀ is hydrogen or C₁-C₃alkyl;
R₁₃ is hydrogen;
X is oxygen or sulfur;
m is 2;
n is 0, 1, 2 or 3; and
p is 0, 1 or 2.

14. A method according to claim 13 wherein:
R₁ is hydrogen; C₁-C₃alkyl; CF₃; C₂-C₅alkoxyalkyl; C₂-C₅alkenyl; C₃-C₆cycloalkyl; or halogen;
R₂ is hydrogen; C₁-C₅alkyl; CF₃; C₃-C₅alkoxyalkyl; nitro; cyano; S(O)ₚ-C₁-C₃alkyl; amino; N(R₃)R₉; N=C(R₉)R₁₀; or halogen;
R₃ is hydrogen;
R₄ and R₈ are each independently of the other hydrogen; C₁-C₅alkyl; C₂-C₆alkoxyalkyl; or C₃-C₆cycloalkyl;
R₅ is halogen; C₁-C₃alkyl; C₁-C₃alkoxy; C₂-C₆alkoxyalkyl; C₁-C₃haloalkyl; C₁-C₃alkylthio; C₁-C₃haloalkoxy; C₁-C₃haloalkylthio; nitro; or cyano;
R₆ is C₁-C₅alkyl; phenyl; or phenyl substituted by halogen and/or by C₁-C₃alkyl;
R₇ is phenyl; phenyl mono- or di-substituted by identical or different substituents selected from halogen, nitro and cyano; benzyl; benzyl mono- or di-substituted in the ring by identical or different substituents selected from halogen, nitro and cyano; C₁-C₅alkyl; or C₁-C₅alkyl substituted by halogen or by cyano;
R₉ is C₁-C₅alkyl;
R₁₀ is hydrogen or C₁-C₃alkyl;
R₁₃ is hydrogen;
X is oxygen or sulfur;
m is 3;
n is 0, 1, 2 or 3; and
p is 0, 1 or 2.

15. A method according to claim 1 which comprises applying a compound of formula Ia 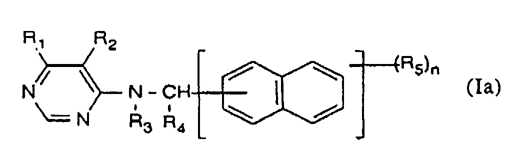 wherein:
R₁ is C₁-C₅alkyl that is unsubstituted or substituted by halogen or by C₁-C₃alkoxy; C₂-C₇alkenyl that is unsubstituted or substituted by halogen; C₃-C₇cycloalkyl: or halogen;
R₂ is C₁-C₅alkyl that is unsubstituted or substituted by halogen or by C₁-C₃alkoxy; halogen; nitro; or cyano;
R₃ is hydrogen; C₁-C₅alkyl; benzyl; -CO-R₆; or -S-R₇;
R₄ is hydrogen; C₁-C₅alkyl that is unsubstituted or substituted by halogen or by C₁-C₃alkoxy; or C₁-C₇cycloalkyl;
R₅ is halogen; C₁-C₃alkyl; C₁-C₃alkoxy; C₁-C₃alkylthio; or an -X-phenyl group that occurs once at the phenyl ring and that is unsubstituted or mono- to tri-substituted by identical or different substituents selected from halogen, C₁-C₃alkyl and C₁-C₃alkoxy;
R₆ is C₁-C₅alkyl; or phenyl that is unsubstituted or substituted by halogen and/or by C₁-C₃alkyl;
R₇ is phenyl or benzyl each of which is unsubstituted or mono- or di-substituted in the ring by identical or different substituents selected from halogen, nitro and cyano; or C₁-C₅alkyl that is unsubstituted or substituted by halogen or by cyano;
X is oxygen or sulfur; and
n is 0, 1, 2 or 3.

16. A method according to claim 1 which comprises applying a compound of formula I from the group:
(d,l)-4-[1'-(β-naphthyl)-ethylamino]-5-chloro-6-ethylpyrimidine (comp. 1.2);
(-)-4-[1'-(β-naphthyl)-ethylamino]-5-chloro-6-ethylpyrimidine (comp. 1.73);
(d,l)-4-[1'-(β-naphthyl)-propylamino]-5-chloro-6-ethylpyrimidine (comp. 1.3);
(d,l)-4-[1'-(β-naphthyl)-ethylamino]-5-chloro-6-methylpyrimidine (comp. 1.53);
(d,l)-4-[1'-(2-(6-bromonaphthyl))-ethylamino]-5-chloro-6-ethylpyrimidine (comp. 1. 149) ;
(d,l)-4-[1'-(β-naphthyl)-ethylamino]-5-bromo-6-ethylpyrimidine (comp. 1.84);
(d,l)-4-[1'-(β-naphthyl)-ethylamino]-5-chloro-6-n-propylpyrimidine (comp. 1.86);
(d,l)-4-[1'-((β-naphthyl)-ethylamino]-5-iodo-6-ethylpyrimidine (comp. 1.100).

17. A process for the preparation of a compound of formula I according to claim 1, which comprises reacting a compound of formula II 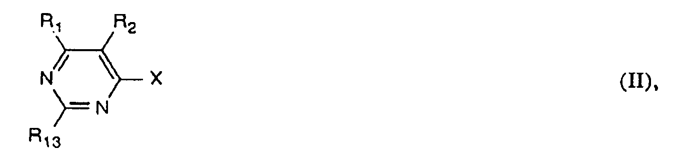 wherein R₁, R₂ and R₁₃ are as defined for formula I and X is a readily removable radical, with a compound of formula III 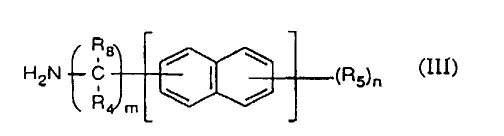 wherein R₄, R₅, R₈, m and n are as defined for formula I, preferably in the presence of a base, and, if desired, then introducing the substituent R₃ (where that substituent is alkyl, benzyl, acyl or -S-R₇) by corresponding N-alkylation, N-benzylation, N-acylation or N-thioalkylation or N-thioarylation, respectively.

## Revendications (Revendications pour l'(les) Etat(s) contractant(s) suivant(s): CH, DE, DK, FR, GB, IT, LI, NL)

1. Composé de formule I 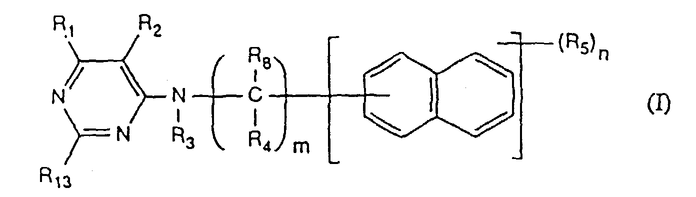 dans laquelle ;
R₁ est un hydrogène, un groupe alkyle en C₁-C₅, qui est non substitué ou est substitué par des substituants halogéno ou alcoxy en C₁-C₃ ou S(O)p-(alkyle en C₁-C₃) ; alcényle en C₂-C₇, qui est non substitué ou substitué par des halogènes ; cycloalkyle en C₃-C₇ ; halogéno ; ou alcynyle en C₂-C₄ ;
R₂ est un hydrogène, le groupe hydroxy, un groupe alkyle en C₁-C₅, qui est non substitué ou est substitué par des substituants halogéno ou alcoxy en C₁-C₃, alcoxy en C₁-C₄, S(O)ₚ(alkyle en C₁-C₄), halogéno, nitro, cyano, amino, NHR₃, N(R₃)R₉ ou N=C(R₉)R₁₀ ;
R₃ est un hydrogène ; un groupe alkyle en C₁-C₅ ; benzyle ; -CO-R₆ ou -S-R₇ ;
R₄ et R₈ sont, indépendamment l'un de l'autre, chacun un hydrogène ; un groupe alkyle en C₁-C₅, qui est non substitué ou substitué par des substituants halogéno ou alcoxy en C₁-C₃ ; ou cycloalkyle en C₃-C₇ ;
R₅ est un halogène ou un groupe alkyle en C₁-C₃, alcoxy en C₁-C₃, alcoxyalkyle en C₂-C₆; halogénalkyle en C₁-C₃, alkylthio en C₁-C₃, halogénalcoxy en C₁-C₃, halogénalkylthio en C₁-C₃, CN, NO₂, ou encore un groupe -X-phényle apparaissant une fois sur le noyau naphtyle, et qui est non substitué ou est une à trois fois substitué par des substituants, identiques ou différents, halogéno, alkyle en C₁-C₃ ou alcoxy en C₁-C₃ ;
R₆ est un groupe alkyle en C₁-C₅ ; phényle qui est non substitué ou est substitué par des substituants halogéno et/ou alkyle en C₁-C₃ ;
R₇ est le radical phényle ou benzyle, qui est non substitué ou encore est, sur le noyau, une à deux fois substitué par des substituants, identiques ou différents, halogéno, nitro, cyano ; ou un groupe alkyle en C₁-C₅, qui est non substitué ou est substitué par des substituants halogéno ou cyano ;
R₉ est un groupe alkyle en C₁-C₅ ;
R₁₀ est un hydrogène ou un groupe alkyle en C₁-C₅ ;
R₁₃ est un hydrogène ; un groupe alkyle en C₁-C₄ qui est non substitué ou est substitué par des substituants halogéno ou alcoxy en C₁-C₃ ; cyclopropyle, halogéno ; alcoxy en C₁-C₃ ; alkylthio en C₁-C₃ ou - N(alkyle en C₁-C₃)₂ ;
X est l'oxygène ou le soufre ;
m vaut 1, 2 ou 3 ;
n vaut 0, 1, 2 ou 3, et
p vaut 0, 1 ou 2.

2. Composé de formule I selon la revendication 1, dans lesquels :
R₁ est un hydrogène ou un groupe alkyle en C₁-C₅ ; ou alkyle en C₁-C₅ substitué par des substituants S(O)p- (alkyle en C₁-C₃) ; halogénalkyle en C₁-C₅ ayant de 1 à 5 atomes d'halogène ; alcoxyalkyle en C₂-C₆ ; alcényle en C₂-C₇; halogénalcényle en C₂-C₇ ayant de 1 à 2 atomes d'halogène ; cycloalkyle en C₃-C₆ ; halogéno ou alcynyle en C₂-C₄ ;
R₂ est un hydrogène ou un groupe alkyle en C₁-C₅ ; halogénalkyle en C₁-C₅ ayant de 1 à 5 atomes d'halogène ; alcoxyalkyle en C₂-C₆ ; nitro, cyano, S(O)p-(alkyle en C₁-C₃), amino, NHR₃, N(R₃)R₉, N=C(R₉)R₁₀ ou halogéno ;
R₃ est un hydrogène ou un groupe alkyle en C₁-C₅ ; benzyle ; -CO-R₆ ou -S-R₇ ;
R₄ et R₈, indépendamment l'un de l'autre, sont chacun un hydrogène ; un groupe alkyle en C₁-C₅ ; halogénalkyle en C₁-C₅ ayant de 1 à 3 atomes d'halogène ; alcoxyalkyle en C₂-C₆ ou cycloalkyle en C₃-C₆ ;
R₅ est un halogène ; un groupe alkyle en C₁-C₃ ; alcoxy en C₁-C₃ ; alcoxyalkyle en C₁-C₆, halogénalkyle en C₁-C₃, alkylthio en C₁-C₃, halogénalcoxy en C₁-C₃, halogénalkylthio en C₁-C₃, CN, NO₂, ou un groupe X-phényle apparaissant sur le noyau naphtyle, et qui est non substitué ou est une à trois fois substitué par des substituants identiques ou différents, halogéno, alkyle en C₁-C₃ ou alcoxy en C₁-C₃ ;
R₆ est un groupe alkyle en C₁-C₅ ou phényle, qui est non substitué ou est substitué par des substituants halogéno et/ou alkyle en C₁-C₃ ;
R₇ est le radical phényle ou benzyle, qui est non substitué, ou est substitué sur le noyau, une à deux fois par des substituants identiques ou différents halogéno, nitro, cyano ; ou encore un groupe alkyle en C₁-C₅, qui est non substitué ou substitué par des substituants halogéno ou cyano ;
X est un oxygène ou un soufre ;
R₉ est un groupe alkyle en C₁-C₅,
R₁₀ est un hydrogène ou un groupe alkyle en C₁-C₅,
m vaut 1, 2 ou 3,
n vaut 0, 1, 2 ou 3, et p vaut 0, 1 ou 2.

3. Composé de formule I selon la revendication 1, dans lesquels :
R₁ est un hydrogène ou un groupe alkyle en C₁-C₅ substitué par des substituants S(O)ₚ-(alkyle en C₁-C₃) ; alkyle en C₁-C₅ ; halogénalkyle en C₁-C₃ ayant de 1 à 3 atomes d'halogène ; alcoxyalkyle en C₂-C₅ ; alcényle en C₂-C₅ ; halogénalcényle en C₂-C₅ ayant de 1 à 3 atomes d'halogène ; cycloalkyle en C₃-C₅ ; halogéno ou alcynyle en C₂-C₄ ;
R₂ est un hydrogène ou un groupe alkyle en C₁-C₅ ; halogénalkyle en C₁-C₃ ayant de 1 à 3 atomes d'halogène ; alcoxyalkyle en C₂-C₅ ; nitro, cyano, S(O)ₚ-(alkyle en C₁-C₃), amino, NHR₃, N(R₃)R₉, N=C(R₉)R₁₀ ou halogéno ;
R₃ est un hydrogène ou un groupe alkyle en C₁-C₅ ; benzyle ; -CO-R₆ ou -S-R₇ ;
R₄ et R₈, indépendamment de l'autre, sont chacun un hydrogène ; un groupe alkyle en C₁-C₅ , halogénalkyle en C₁-C₅ ayant de 1 à 3 atomes d'halogène ; alcoxyalkyle en C₂-C₅ ou cycloalkyle en C₃-C₆ ;
R₅ est un halogène ; un groupe alkyle en C₁-C₃ ; alcoxy en C₁-C₃ ; alcoxyalkyle en C₁-C₆, halogénalkyle en C₁-C₃, alkylthio en C₁-C₃, halogénalcoxy en C₁-C₃, halogénalkylthio en C₁-C₃, CN, NO₂ ou un groupe X-phényle apparaissant sur le noyau naphtyle et qui est non substitué ou est une à trois fois substitué par des substituants identiques ou différents halogéno, alkyle en C₁-C₃ ou alcoxy en C₁-C₃ ;
R₆ est un groupe alkyle en C₁-C₅ ou phényle, qui est non substitué ou est substitué par des substituants halogéno ou alkyle en C₁-C₃ ;
R₇ est un radical phényle ou benzyle, qui est non substitué ou qui est substitué sur le noyau, une à deux fois, par des substituants identiques ou différents halogéno, nitro, cyano ; ou alkyle en C₁-C₅, qui est non substitué ou est substitué par des substituants halogéno ou cyano ;
X est un oxygène ou un soufre,
R₉ est un groupe alkyle en C₁-C₅ ;
R₁₀ est un hydrogène ou un groupe alkyle en C₁-C₅,
m vaut 1, 2 ou 3 ;
n vaut 0, 1, 2 ou 3 ; et
p vaut 0, 1 ou 2.

4. Composé de formule I' 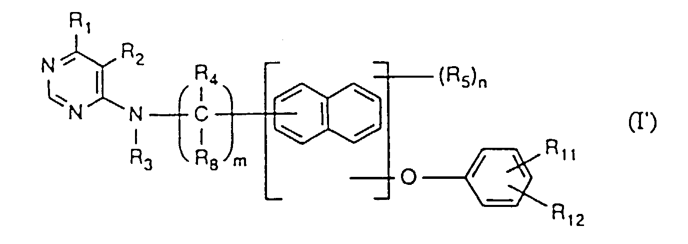 dans laquelle :
R₁ est un hydrogène, un groupe alkyle en C₁-C₅ ou alkyle en C₁-C₅ substitué par des substituants S(O)ₚ-(alkyle en C₁-C₃) ; halogénalkyle en C₁-C₃ ayant de 1 à 3 atomes d'halogène ; alcoxyalkyle en C₂-C₅ ; alcényle en C₂-C₅ ; halogénalcényle en C₂-C₅ ayant de 1 à 3 atomes d'halogène ; cycloalkyle en C₃-C₆ ; halogéno ou alcynyle en C₂-C₄ ;
R₂ est un hydrogène ou un groupe alkyle en C₁-C₅ ; halogénalkyle en C₁-C₃ ayant de 1 à 3 atomes d'halogène ; alcoxyalkyle en C₃-C₅ ; nitro, cyano, S(O)ₚ-(alkyle en C₁-C₃), amino, NHR₃, N(R₃)R₉, N=C(R₉)R₁₀ ou halogéno ;
R₃ est un hydrogène ou un groupe alkyle en C₁-C₅ ; benzyle ; -CO-R₆ ou S-R₇ ;
R₄ et R₈, indépendamment l'un de l'autre, sont chacun un hydrogène ou un groupe alkyle en C₁-C₅ ; halogénalkyle en C₁-C₅ ayant de 1 à 3 atomes d'halogène ; alcoxyalkyle en C₂-C₆ ou cycloalkyle en C₃-C₆ ;
R₅ est un halogène ou un groupe alkyle en C₁-C₃, alcoxy en C₁-C₃ ; halogénalkyle en C₁-C₃, halogénalcoxy en C₁-C₃,
R₆ est un groupe alkyle en C₁-C₅ ; phényle qui est non substitué, ou est substitué par des substituants halogéno et/ou alkyle en C₁-C₃ ;
R₇ est un radical phényle ou benzyle, qui est non substitué ou est une à deux fois substitué sur le noyau par des substituants identiques ou différents halogéno, nitro, cyano ; ou encore alkyle en C₁-C₅, qui est non substitué ou est substitué par des substituants halogéno ou cyano ;
R₉ est un groupe alkyle en C₁-C₅,
R₁₀ est un hydrogène ou un groupe alkyle en C₁-C₅,
R₁₁ et R₁₂, indépendamment l'un de l'autre, sont chacun un hydrogène, un halogène ou un groupe alkyle en C₁-C₃,
m vaut 1, 2 ou 3 ;
n vaut 0, 1, 2 ; et
p vaut 0, 1 ou 2.

5. Composés de formule I' selon la revendication 4, dans lesquels :
R₁ à R₄ et R₈ ont les significations données à propos de la formule (I),
R₅ est un halogène ou un groupe alkyle en C₁-C₃, CF₃, alcoxyalkyle en C₂-C₅ ou alcoxy en C₁-C₃ ;
R₁₁ et R₁₂, indépendamment l'un de l'autre, sont chacun un hydrogène, un halogène ou un groupe alkyle en C₁-C₃ ;
m vaut 1, 2 ou 3 ; et
n vaut 0, 1 ou 2.

6. Composé de formule I selon la revendication 1, dans lesquels :
R₁ est un hydrogène ou un groupe alkyle en C₁-C₅ ; halogénalkyle en C₁-C₅ ayant de 1 à 3 atomes d'halogène ; alcoxyalkyle en C₂-C₅ ; alcényle en C₂-C₅ halogénalcényle en C₂-C₅ ou halogéno ;
R₂ est un hydrogène ou un groupe alkyle en C₁-C₅ ; halogénalkyle en C₁-C₃ ayant de 1 à 3 atomes d'halogène ; alcoxyalkyle en C₃-C₅ ; nitro, cyano, S(O)ₚ-(alkyle en C₁-C₄), amino, NHR₃, N(R₃)R₉, N=C(R₉)R₁₀ ou halogéno ;
R₃ est un hydrogène ou un groupe alkyle en C₁-C₃, benzyle, -CO-R₆ ou SR₇ ;
R₄ et R₈, indépendamment l'un de l'autre, sont chacun un hydrogène ou un groupe alkyle en C₁-C₅ ; halogénalkyle en C₁-C₅ ayant de 1 à 3 atomes d'halogène ; alcoxyalkyle en C₂-C₆ ou cycloalkyle en C₃-C₆ ;
R₅ est un halogène ou un groupe alkyle en C₁-C₃ ; alcoxy en C₁-C₃ ; alcoxyalkyle en C₁-C₆, halogénalkyle en C₁-C₃, alkylthio en C₁-C₃, halogénalcoxy en C₁-C₃, halogénalkylthio en C₁-C₃, cyano ou nitro ;
R₆ est un groupe alkyle en C₁-C₅, phényle, phényle substitué par des substituants halogéno et/ou alkyle en C₁-C₃ ;
R₇ est le radical phényle, un radical phényle substitué une ou deux fois par des substituants halogéno, nitro ou cyano, le radical benzyle, un radical benzyle une ou deux fois substitué sur le noyau par des substituants halogéno, nitro ou cyano, ou encore un groupe alkyle en C₁-C₅ ou un groupe alkyle en C₁-C₅ substitué par des substituants halogéno ou cyano ;
R₉ est un groupe alkyle en C₁-C₅ ;
R₁₀ est un hydrogène ou un groupe alkyle en C₁-C₃ ;
R₁₃ est un hydrogène ou un groupe alkyle en C₁-C₄ ;
m vaut 1, 2 ou 3 ;
n vaut 0, 1 ou 2 ; et
p vaut 0, 1 ou 2.

7. Composés selon la revendication 6, dans lesquels :
R₁ est un groupe alkyle en C₁-C₅ ; CF₃ ; alcoxyalkyle en C₂-C₅ ; alcényle en C₂-C₄ ; monohalogénalcényle en C₂-C₄ ; ou halogéno ;
R₂ est un groupe alkyle en C₁-C₅ ou halogéno ;
R₃ est un hydrogène ou un groupe alkyle en C₁-C₃ ;
R₄ est un hydrogène ou un groupe alkyle en C₁-C₃ ou cyclopropyle ;
R₅ est un halogène ; un groupe alkyle en C₁-C₂, alcoxy en C₁-C₃ ;
R₈ est un hydrogène ;
n vaut 0, 1, 2 ou 3 ; et
m vaut 1.

8. Composés selon la revendication 7, dans lesquels :
R₁ est un groupe alkyle en C₁-C₄ ; CF₃ ; alcoxyalkyle en C₂-C₄ ; alcényle en C₂-C₄ ; ou halogéno ;
R₂ est un groupe alkyle en C₁-C₄ ou halogéno ;
R₃ est un hydrogène ou un groupe alkyle en C₁-C₃ ;
R₄ est un hydrogène ou un groupe alkyle en C₁-C₃ ;
R₅ est un halogène ou le groupe méthyle ; éthyle ou méthoxy ;
R₈ est un hydrogène ;
n vaut 0, 1, 2 ou 3 ; et
m vaut 1.

9. Composés de formule I" 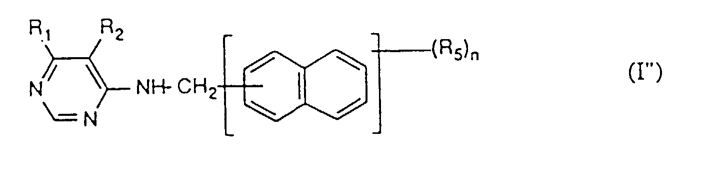 dans laquelle :
R₁ est un groupe alkyle en C₁-C₄, CF₃, alcoxyalkyle en C₂-C₄ ou halogéno ;
R₂ est un groupe alkyle en C₁-C₄ ou halogéno ;
R₅ est le groupe chloro, bromo, méthyle, éthyle, méthoxy, et
n vaut 0, 1 ou 2.

10. Composé de formule I"' 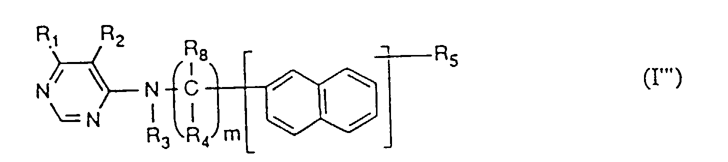 dans laquelle :
R₁ est un hydrogène ou un groupe alkyle en C₁-C₃, CF₃ ou halogéno ;
R₂ est un hydrogène ou un groupe alkyle en C₁-C₃, halogéno, NO₂ ou S(O)ₚ-(alkyle en C₁-C₃) ;
R₃ est un hydrogène ;
R₄ est le radical méthyle, éthyle, isopropyle, n-propyle ou cyclopropyle ;
R₈ est un hydrogène ou le radical méthyle ou éthyle ;
R₅ est un hydrogène ou un groupe halogéno, alkyle en C₁-C₃, OCHF₂, CF₃, NO₂ ou alcoxy en C₁-C₃ ;
n vaut 1 ou 2 ; et
p vaut 0, 1 ou 2.

11. Composés selon la revendication 3, dans lesquels :
R₁ est un hydrogène ou un groupe alkyle en C₁-C₅ ; halogénalkyle en C₁-C₃ ayant de 1 à 3 atomes d'halogène ; alcoxyalkyle en C₂-C₅ ; alcényle en C₂-C₅ ; halogénalcényle en C₂-C₅ ayant de 1 à 3 atomes d'halogène, cycloalkyle en C₃-C₆ ou halogéno ;
R₂ est un hydrogène ou un groupe alkyle en C₁-C₅ ; halogénalkyle en C₁-C₃ ayant de 1 à 3 atomes d'halogène ; alcoxyalkyle en C₃-C₅ ; nitro, cyano, S(O)ₚ-(alkyle en C₁-C₄), amino, NHR₃, N(R₃)R₉, N=C(R₉)R₁₀ ou halogéno ;
R₃ est un hydrogène ou un groupe alkyle en C₁-C₅, benzyle, COR₆ ou SR₇ ;
R₄ et R₈, indépendamment l'un de l'autre, sont chacun un hydrogène ou un groupe alkyle en C₁-C₅ ; halogénalkyle en C₁-C₅ ayant de 1 à 3 atomes d'halogène ; alcoxyalkyle en C₂-C₆ ou cycloalkyle en C₃-C₆ ;
R₅ est un halogène ou un groupe alkyle en C₁-C₃ ; alcoxy en C₁-C₃ ; alcoxyalkyle en C₁-C₆, halogénalkyle en C₁-C₃, alkylthio en C₁-C₃, halogénalcoxy en C₁-C₃, halogénalkylthio en C₁-C₃, cyano ou nitro, ou un groupe X-phényle apparaissant sur le noyau naphtyle, qui est non substitué ou est une à trois fois substitué par des substituants identiques ou différents halogéno, alkyle en C₁-C₃ ou alcoxy en C₁-C₃ ;
R₆ est un groupe alkyle en C₁-C₅, phényle, ou phényle substitué par des substituants halogéno et/ou alkyle en C₁-C₃ ;
R₇ est le radical phényle, un radical phényle une ou deux fois substitué par des substituants identiques ou différents halogéno, nitro ou cyano, le radical benzyle, un radical benzyle une ou deux substitué sur le noyau par des substituants identiques ou différents halogéno, nitro ou cyano, ou encore un groupe alkyle en C₁-C₅ ou alkyle en C₁-C₅ substitué par des substituants halogéno ou cyano ;
R₉ est un groupe alkyle en C₁-C₅ ;
R₁₀ est un hydrogène ou un groupe alkyle en C₁-C₅ ;
R₁₃ est un hydrogène ;
X est un oxygène ou un soufre,
m vaut 2 ou 3,
n vaut 0, 1, 2 ou 3, et
p vaut 0, 1 ou 2.

12. Composés selon la revendication 11, dans lesquels :
R₁ est un hydrogène ou un groupe alkyle en C₁-C₃ ; halogénalkyle en C₁-C₃ ayant de 1 à 3 atomes d'halogène ; alcoxyalkyle en C₂-C₅ ; alcényle en C₂-C₅ ; cycloalkyle en C₃-C₆ ou halogéno ;
R₂ est un hydrogène ou un groupe alkyle en C₁-C₅ ; halogénalkyle en C₁-C₃ ayant de 1 à 3 atomes d'halogène ; alcoxyalkyle en C₃-C₅ ; nitro, cyano, S(O)ₚ-(alkyle en C₁-C₃ ), amino, NHR₃, N(R₃)R₉, N=C(R₉)R₁₀ ou halogéno ;
R₃ est un hydrogène ou un groupe alkyle en C₁-C₃ ;
R₄ et R₈, indépendamment l'un de l'autre, sont chacun un hydrogène ou un groupe alkyle en C₁-C₅ ; CF₃ ; alcoxyalkyle en C₂-C₆ ou cycloalkyle en C₃-C₆ ;
R₅ est un halogène ou un groupe alkyle en C₁-C₃ ; alcoxy en C₁-C₃ ; alcoxyalkyle en C₂-C₆, halogénalkyle en C₁-C₃, alkylthio en C₁-C₃, halogénalcoxy en C₁-C₃, halogénalkylthio en C₁-C₃, cyano ou nitro ;
R₆ est un groupe alkyle en C₁-C₅, phényle, phényle substitué par des substituants halogéno et/ou alkyle en C₁-C₃ ;
R₇ est le radical phényle, phényle une ou deux fois substitué par des substituants identiques ou différents halogéno, nitro ou cyano, benzyle, benzyle une ou deux fois substitué sur le noyau par des substituants identiques ou différents halogéno, nitro ou cyano, ou encore un groupe alkyle en C₁-C₅ ou alkyle en C₁-C₅ substitué par des substituants halogéno ou cyano ;
R₉ est un groupe alkyle en C₁-C₅ ;
R₁₀ est un hydrogène ou un groupe alkyle en C₁-C₅ ;
R₁₃ est un hydrogène,
X est un oxygène ou un soufre,
m vaut 2 ou 3,
n vaut 0, 1, 2 ou 3 ; et
p vaut 0, 1 ou 2.

13. Composés selon la revendication 11, dans lesquels :
R₁ est un hydrogène ou un groupe alkyle en C₁-C₃ ; CF₃, alcoxyalkyle en C₂-C₅ ; alcényle en C₂-C₅ ; cycloalkyle en C₃-C₆ ou halogéno ;
R₂ est un hydrogène ou un groupe alkyle en C₁-C₅ ; CF₃, alcoxyalkyle en C₃-C₅ ; nitro, cyano, S(O)ₚ-(alkyle en C₁-C₄), amino, N(R₃)R₉, N=C(R₉)R₁₀ ou halogéno ;
R₃ est un hydrogène ;
R₄ et R₈, indépendamment l'un de l'autre, sont chacun un hydrogène ou un groupe alkyle en C₁-C₅ ; alcoxyalkyle en C₂-C₆ ou cycloalkyle en C₃-C₆ ;
R₅ est un halogène ou un groupe alkyle en C₁-C₃ ; alcoxy en C₁-C₃ ; alcoxyalkyle en C₂-C₆, halogénalkyle en C₁-C₃, alkylthio en C₁-C₃, halogénalcoxy en C₁-C₃, halogénalkylthio en C₁-C₃, nitro ou cyano,
R₆ est un groupe alkyle en C₁-C₅, phényle ou phényle substitué par des substituants halogéno et/ou alkyle en C₁-C₃ ;
R₇ est le radical phényle, phényle une ou deux fois substitué par des substituants identiques ou différents halogéno, nitro ou cyano, benzyle, benzyle une ou deux fois substitué sur le noyau par des substituants identiques ou différents halogéno, nitro ou cyano, ou bien alkyle en C₁-C₅, ou alkyle en C₁-C₅ substitué par des substituants halogéno ou cyano ;
R₉ est un groupe alkyle en C₁-C₃ ;
R₁₀ est un hydrogène ou un groupe alkyle en C₁-C₃ ;
R₁₃ est un hydrogène ;
X est un oxygène ou un soufre,
m vaut 2 ;
n vaut 0, 1, 2 ou 3 ; et
p vaut 0, 1 ou 2.

14. Composés selon la revendication 13, dans lesquels :
R₁ est un hydrogène ou un groupe alkyle en C₁-C₃ ; CF₃ ; alcoxyalkyle en C₂-C₅ alcényle en C₂-C₅ ; cycloalkyle en C₃-C₆ ou halogéno ;
R₂ est un hydrogène ou un groupe alkyle en C₁-C₅ ; CF₃, alcoxyalkyle en C₃-C₅ ; nitro, cyano, S(O)ₚ-(alkyle en C₁-C₃), amino, N(R₃)R₉, N=C(R₉)R₁₀ ou halogéno ;
R₃ est un hydrogène ;
R₄ et R₈, indépendamment l'un de l'autre, sont chacun un hydrogène ou un groupe alkyle en C₁-C₅ ; alcoxyalkyle en C₂-C₆ ou cycloalkyle en C₃-C₆ ;
R₅ est un halogène ou un groupe alkyle en C₁-C₃ ; alcoxy en C₁-C₃ ; alcoxyalkyle en C₂-C₆, halogénalkyle en C₁-C₃, alkylthio en C₁-C₃, halogénalcoxy en C₁-C₃, halogénalkylthio en C₁-C₃, nitro ou cyano ;
R₆ est un groupe alkyle en C₁-C₅, phényle ou phényle substitué par des substituants halogéno et/ou alkyle en C₁-C₃ ;
R₇ est le radical phényle, phényle une ou deux fois substitué par des substituants identiques ou différents halogéno, nitro ou cyano, benzyle, benzyle une ou deux fois substitué sur le noyau par des substituants identiques ou différents halogéno, nitro ou cyano, ou encore alkyle en C₁-C₅ ou alkyle en C₁-C₅ substitué par des substituants halogéno ou cyano ;
R₉ est un groupe alkyle en C₁-C₅ ;
R₁₀ est un hydrogène ou un groupe alkyle en C₁-C₃ ;
R₁₃ est un hydrogène ;
X est un oxygène ou un soufre,
m vaut 3 ; n vaut 0, 1, 2 ou 3 ; et
p vaut 0, 1 ou 2.

15. Composés de formule Ia 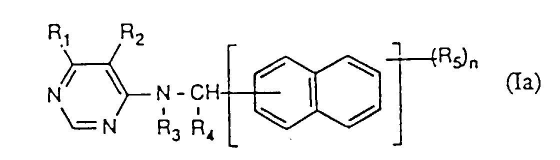 dans laquelle :
R₁ est un groupe alkyle en C₁-C₅, qui est non substitué ou est substitué par des substituants halogéno ou alcoxy en C₁-C₃ ; alcényle en C₂-C₇, qui est non substitué ou est substitué par des halogènes ; cycloalkyle en C₃-C₇ ; ou halogéno ;
R₂ est un groupe alkyle en C₁-C₅, qui est non substitué ou est substitué par des substituants halogéno ou alcoxy en C₁-C₃ ; halogéno ; nitro ou cyano ;
R₃ est un hydrogène ou un groupe alkyle en C₁-C₅ ; benzyle ; CO-R₆ ou -S-R₇ ;
R₄ est un hydrogène ou un groupe alkyle en C₁-C₅, qui est non substitué ou est substitué par des substituants halogéno ou alcoxy en C₁-C₃ ; ou encore cycloalkyle en C₃-C₇ ;
R₅ est un halogène ou un groupe alkyle en C₁-C₃ ; alcoxy en C₁-C₃ ; alkylthio en C₁-C₃ ; ou un groupe -X-phényle apparaissant une fois sur le noyau naphtyle, qui est non substitué ou est une à trois fois substitué par des substituants identiques ou différents halogéno, alkyle en C₁-C₃ ou alcoxy en C₁-C₃ ;
R₆ est un groupe alkyle en C₁-C₅ ; phényle, qui est non substitué ou est substitué par des substituants halogéno et/ou alkyle en C₁-C₃ ;
R₇ est le radical phényle ou benzyle, qui est non substitué ou qui est une à deux fois substitué sur le noyau par des substituants identiques ou différents halogéno, nitro, cyano ; ou encore alkyle en C₁-C₅, qui est non substitué ou est substitué par des substituants halogéno ou cyano ;
X est un oxygène ou un soufre ; et
n vaut 0, 1, 2 ou 3.

16. Composés de formule I selon la revendication 1, appartenant au groupe suivant :
(d,l)-4-[1'-(β-naphtyl)éthylamino]-5-chloro-6-éthylpyrimidine (Composé 1.2) ;
(-)-4-[1'-(β-naphtyl)éthylamino]-5-chloro-6-éthylpyrimidine (Composé 1.73) ;
(d,l)-4-[1'-(β-naphtyl)propylamino]-5-chloro-6-éthylpyrimidine (Composé 1.3) ;
(d,l)-4-[1'-(β-naphtyl)éthylamino]-5-chloro-6-méthylpyrimidine (Composé 1.53) ;
(d,l)-4-[1'-(2-(6-bromonaphtyl)éthylamino]-5-chloro-6-éthylpyrimidine (Composé 1.149) ;
(d,l)-4-[1'-(β-naphtyl)éthylamino]-5-bromo-6-éthylpyrimidine (Composé 1.84) ;
(d,l)-4-[1'-(β-naphtyl)éthylamino]-5-chloro-6-n-propylpyrimidine (Composé 1.86) ;
(d,l)-4-[1'-(β-naphtyl)éthylamino]-5-iodo-6-éthylpyrimidine (Composé 1.100).

17. Procédé de préparation des composé de formule I selon la revendication 1, caractérisé en ce qu'on fait réagir un composé de formule II 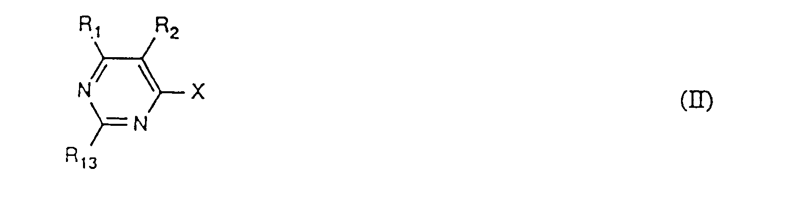 dans laquelle R₁, R₂ et R₁₃ ont les significations données pour la formule I et X est un résidu facilement éliminable, avec un composé de formule III 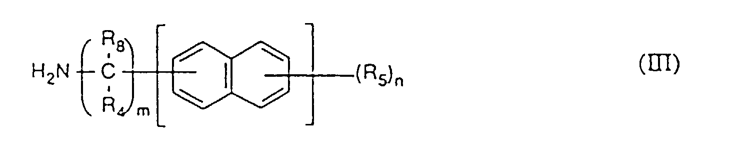 dans laquelle R₄, R₅, R₈, m et n ont les significations données pour la formule I, de préférence en présence d'une base, puis, si on le souhaite, on introduit le substituant R₃, dans la mesure où ce substituant est un groupe alkyle, benzyle, acyle ou -S-R₇, par des opérations correspondantes de N-alkylation, de N-benzylation, de N-acylation ou de N-thioalkylation ou thioarylation.

18. Produit pour la protection de végétaux contre une infestation par des ravageurs, caractérisé en ce qu'il contient en tant que matière active au moins un composé selon la revendication 1 et un support.

19. Produit selon la revendication 18, caractérisé en ce qu'il contient en tant que matière active au moins un composé selon les revendications 2 à 15.

20. Produit selon la revendication 18, caractérisé en ce qu'il contient en tant que matière active un composé de formule I selon la revendication 16.

21. Procédé pour préparer un produit à usage agrochimique selon la revendication 18, caractérisé en ce qu'on mélange des supports ou adjuvants appropriés, solides ou liquides, à au moins un composé selon la revendication 1.

22. Utilisation de composés selon la revendication 1 pour protéger des végétaux contre une infestation par des microorganismes phytopathogènes.

23. Utilisation de composés selon l'une des revendications 2 à 16 pour protéger des végétaux contre une infestation par des microorganismes phytopathogènes.

24. Procédé pour protéger des végétaux contre une infestation par des microorganismes phytopathogènes, caractérisé en ce que l'on applique en tant que matière active un composé selon la revendication 1 sur les végétaux ou sur leur site.

25. Procédé pour protéger des végétaux contre une infestation par des microorganismes phytopathogènes, caractérisé en ce que l'on applique en tant que matière active un composé selon l'une des revendications 2 à 16 sur les végétaux ou sur leur site.

26. Procédé selon la revendication 25, caractérisé en ce que, pour ce qui est des microorganismes phytopathogènes, il s'agit d'organismes fongiques.

## Revendications (Revendications pour l'(les) Etat(s) contractant(s) suivant(s): ES)

1. Procédé pour protéger des végétaux contre une infestation par des microorganismes phytopathogènes, caractérisé en ce que l'on applique sur les végétaux ou sur leur site, en tant que matière active, un composé de formule I 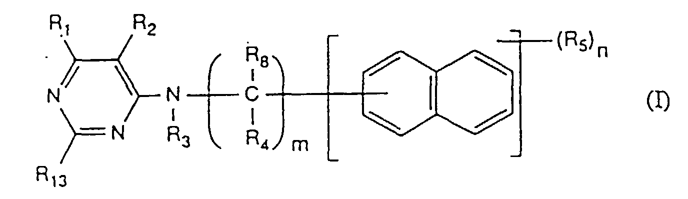 dans laquelle :
R₁ est un hydrogène, un groupe alkyle en C₁-C₅, qui est non substitué ou est substitué par des substituants halogéno ou alcoxy en C₁-C₃ ou S(O)ₚ-(alkyle en C₁-C₃) ; alcényle en C₂-C₇, qui est non substitué ou substitué par des halogènes ; cycloalkyle en C₃-C₇ halogéno ; ou alcynyle en C₂-C₄ ;
R₂ est un hydrogène, le groupe hydroxy, un groupe alkyle en C₁-C₅, qui est non substitué ou est substitué par des substituants halogéno ou alcoxy en C₁-C₃, alcoxy en C₁-C₄, S(O)ₚ(alkyle en C₁-C₄), halogéno, nitro, cyano, amino, NHR₃, N(R₃)R₉ ou N=C(R₉)R₁₀ ;
R₃ est un hydrogène ; un groupe alkyle en C₁-C₅ ; benzyle ; -CO-R₆ ou -S-R₇ ;
R₄ et R₈ sont, indépendamment l'un de l'autre, chacun un hydrogène ; un groupe alkyle en C₁-C₅, qui est non substitué ou substitué par des substituants halogéno ou alcoxy en C₁-C₃ ; ou cycloalkyle en C₃-C₇ ;
R₅ est un halogène ou un groupe alkyle en C₁-C₃, alcoxy en C₁-C₃, alcoxyalkyle en C₂-C₆; halogénalkyle en C₁-C₃, alkylthio en C₁-C₃, halogénalcoxy en C₁-C₃, halogénalkylthio en C₁-C₃, CN, NO₂, ou encore un groupe -X-phényle apparaissant une fois sur le noyau naphtyle, et qui est non substitué ou est une à trois fois substitué par des substituants, identiques ou différents, halogéno, alkyle en C₁-C₃ ou alcoxy en C₁-C₃ ;
R6 est un groupe alkyle en C₁-C₅ ; phényle qui est non substitué ou est substitué par des substituants halogéno et/ou alkyle en C₁-C₃ ;
R₇ est le radical phényle ou benzyle, qui est non substitué ou encore est, sur le noyau, une à deux fois substitué par des substituants, identiques ou différents, halogéno, nitro, cyano ; ou un groupe alkyle en C₁-C₅, qui est non substitué ou est substitué par des substituants halogéno ou cyano ;
R₉ est un groupe alkyle en C₁-C₅ ;
R₁₀ est un hydrogène ou un groupe alkyle en C₁-C₅ ;
R₁₃ est un hydrogène ; un groupe alkyle en C₁-C₄ qui est non substitué ou est substitué par des substituants halogéno ou alcoxy en C₁-C₃ cyclopropyle, halogéno ; alcoxy en C₁-C₃ ; alkylthio en C₁-C₃ ou-N(alkyle en C₁-C₃)₂ ;
X est l'oxygène ou le soufre ;
m vaut 1, 2 ou 3 ;
n vaut 0, 1, 2 ou 3, et
p vaut 0, 1 ou 2.

2. Procédé selon la revendication 1, dans lequel :
R₁ est un hydrogène ou un groupe alkyle en C₁-C₅ ; ou alkyle en C₁-C₅ substitué par des substituants S(O)ₚ-(alkyle en C₁-C₃) ; halogénalkyle en C₁-C₅ ayant de 1 à 5 atomes d'halogène ; alcoxyalkyle en C₂-C₆ alcényle en C₂-C₇ ; halogénalcényle en C₂-C₇ ayant de 1 à 2 atomes d'halogène ; cycloalkyle en C₃-C₆ ; halogéno ou alcynyle en C₂-C₄ ;
R₂ est un hydrogène ou un groupe alkyle en C₁-C₅ ; halogénalkyle en C₁-C₅ ayant de 1 à 5 atomes d'halogène ; alcoxyalkyle en C₂-C₆ ; nitro, cyano, S(O)ₚ-(alkyle en C₁-C₃), amino, NHR₃, N(R₃)R₉, N=C(R₉)R₁₀ ou halogéno ;
R₃ est un hydrogène ou un groupe alkyle en C₁-C₅ ; benzyle ; -CO-R₆ ou -S-R7 ;
R₄ et R₈, indépendamment l'un de l'autre, sont chacun un hydrogène ; un groupe alkyle en C₁-C₅ ; halogénalkyle en C₁-C₅ ayant de 1 à 3 atomes d'halogène ; alcoxyalkyle en C₂-C₆ ou cycloalkyle en C₃-C₆ ;
R₅ est un halogène ; un groupe alkyle en C₁-C₃ ; alcoxy en C₁-C₃ ; alcoxyalkyle en C₁-C₆, halogénalkyle en C₁-C₃, alkylthio en C₁-C₃, halogénalcoxy en C₁-C₃, halogénalkylthio en C₁-C₃, CN, NO₂, ou un groupe X-phényle apparaissant sur le noyau naphtyle, et qui est non substitué ou est une à trois fois substitué par des substituants identiques ou différents, halogéno, alkyle en C₁-C₃ ou alcoxy en C₁-C₃ ;
R₆ est un groupe alkyle en C₁-C₅ ou phényle, qui est non substitué ou est substitué par des substituants halogéno et/ou alkyle en C₁-C₃ ;
R₇ est le radical phényle ou benzyle, qui est non substitué, ou est substitué sur le noyau, une à deux fois par des substituants identiques ou différents halogéno, nitro, cyano ; ou encore un groupe alkyle en C₁-C₅, qui est non substitué ou substitué par des substituants halogéno ou cyano ;
X est un oxygène ou un soufre ;
R₉ est un groupe alkyle en C₁-C₅,
R₁₀ est un hydrogène ou un groupe alkyle en C₁-C₅,
m vaut 1, 2 ou 3,
n vaut 0, 1, 2 ou 3, et
p vaut 0, 1 ou 2.

3. Procédé selon la revendication 1, dans lequel
R₁ est un hydrogène ou un groupe alkyle en C₁-C₅ substitué par des substituants S(O)ₚ-(alkyle en C₁-C₃) ; alkyle en C₁-C₅ ; halogénalkyle en C₁-C₃ ayant de 1 à 3 atomes d'halogène ; alcoxyalkyle en C₂-C₅ ; alcényle en C₂-C₅ ; halogénalcényle en C₂-C₅ ayant de 1 à 3 atomes d'halogène ; cycloalkyle en C₃-C₅ ; halogéno ou alcynyle en C₂-C₄ ;
R₂ est un hydrogène ou un groupe alkyle en C₁-C₅ ; halogénalkyle en C₁-C₃ ayant de 1 à 3 atomes d'halogène ; alcoxyalkyle en C₂-C₅ ; nitro, cyano, S(O)ₚ-(alkyle en C₁-C₃), amino, NHR₃, N(R₃)R₉, N=C(R₉)R₁₀ ou halogéno ;
R₃ est un hydrogène ou un groupe alkyle en C₁-C₅ ; benzyle ; -CO-R₆ ou -S-R₇ ;
R₄ et R₈, indépendamment de l'autre, sont chacun un hydrogène ; un groupe alkyle en C₁-C₅, halogénalkyle en C₁-C₅ ayant de 1 à 3 atomes d'halogène ; alcoxyalkyle en C₂-C₅ ou cycloalkyle en C₃-C₆ ;
R₅ est un halogène ; un groupe alkyle en C₁-C₃ ; alcoxy en C₁-C₃ ; alcoxyalkyle en C₁-C₆, halogénalkyle en C₁-C₃, alkylthio en C₁-C₃, halogénalcoxy en C₁-C₃, halogénalkylthio en C₁-C₃, CN, NO₂ ou un groupe X-phényle apparaissant sur le noyau naphtyle et qui est non substitué ou est une à trois fois substitué par des substituants identiques ou différents halogéno, alkyle en C₁-C₃ ou alcoxy en C₁-C₃ ;
R₆ est un groupe alkyle en C₁-C₅ ou phényle, qui est non substitué ou est substitué par des substituants halogéno ou alkyle en C₁-C₃ ;
R₇ est un radical phényle ou benzyle, qui est non substitué ou qui est substitué sur le noyau, une à deux fois, par des substituants identiques ou différents halogéno, nitro, cyano ; ou alkyle en C₁-C₅, qui est non substitué ou est substitué par des substituants halogéno ou cyano ;
X est un oxygène ou un soufre,
R₉ est un groupe alkyle en C₁-C₅ ;
R₁₀ est un hydrogène ou un groupe alkyle en C₁-C₅,
m vaut 1, 2 ou 3 ;
n vaut 0, 1, 2 ou 3 ; et
p vaut 0, 1 ou 2.

4. Procédé selon la revendication 1, dans lequel on applique un composé de formule I' 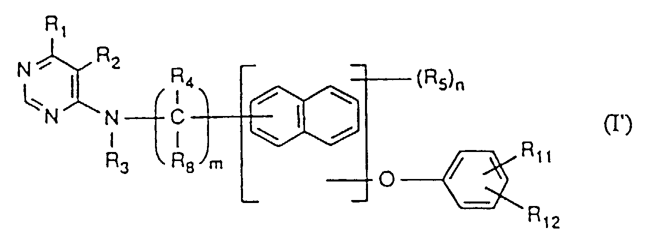 dans laquelle
R₁ est un hydrogène, un groupe alkyle en C₁-C₅ ou alkyle en C₁-C₅ substitué par des substituants S(O)ₚ-(alkyle en C₁-C₃) ; halogénalkyle en C₁-C₃ ayant de 1 à 3 atomes d'halogène ; alcoxyalkyle en C₂-C₅ ; alcényle en C₂-C₅ ; halogénalcényle en C₂-C₅ ayant de 1 à 3 atomes d'halogène ; cycloalkyle en C₃-C₆ ; halogéno ou alcynyle en C₂-C₄ ;
R₂ est un hydrogène ou un groupe alkyle en C₁-C₅ ; halogénalkyle en C₁-C₃ ayant de 1 à 3 atomes d'halogène ; alcoxyalkyle en C₃-C₅ ; nitro, cyano, S(O)ₚ-(alkyle en C₁-C₃), amino, NHR₃, N(R₃)R₉, N=C(R₉)R₁₀ ou halogéno ;
R₃ est un hydrogène ou un groupe alkyle en C₁-C₅ ; benzyle ; -CO-R₆ ou S-R₇ ;
R₄ et R₈, indépendamment l'un de l'autre, sont chacun un hydrogène ou un groupe alkyle en C₁-C₅ ; halogénalkyle en C₁-C₅ ayant de 1 à 3 atomes d'halogène ; alcoxyalkyle en C₂-C₆ ou cycloalkyle en C₃-C₆ ;
R₅ est un halogène ou un groupe alkyle en C₁-C₃, alcoxy en C₁-C₃ ; halogénalkyle en C₁-C₃, halogénalcoxy en C₁-C₃,
R₆ est un groupe alkyle en C₁-C₅ ; phényle qui est non substitué, ou est substitué par des substituants halogéno et/ou alkyle en C₁-C₃ ;
R₇ est un radical phényle ou benzyle, qui est non substitué ou est une à deux fois substitué sur le noyau par des substituants identiques ou différents halogéno, nitro, cyano ; ou encore alkyle en C₁-C₅, qui est non substitué ou est substitué par des substituants halogéno ou cyano ;
R₉ est un groupe alkyle en C₁-C₅,
R₁₀ est un hydrogène ou un groupe alkyle en C₁-C₅,
R₁₁ et R₁₂, indépendamment l'un de l'autre, sont chacun un hydrogène, un halogène ou un groupe alkyle en C₁-C₃,
m vaut 1, 2 ou 3 ;
n vaut 0, 1, 2 ; et
p vaut 0, 1 ou 2.

5. Procédé selon la revendication 4, dans lequel
R₁ à R₄ et R₈ ont les significations données à propos de la formule (I),
R₅ est un halogène ou un groupe alkyle en C₁-C₃, CF₃, alcoxyalkyle en C₂-C₅ ou alcoxy en C₁-C₃ ;
R₁₁ et R₁₂, indépendamment l'un de l'autre, sont chacun un hydrogène, un halogène ou un groupe alkyle en C₁-C₃ ;
m vaut 1, 2 ou 3 ; et
n vaut 0, 1 ou 2.

6. Procédé selon la revendication 1, dans lequel
R₁ est un hydrogène ou un groupe alkyle en C₁-C₅ ; halogénaîkyle en C₁-C₅ ayant de 1 à 3 atomes d'halogène alcoxyalkyle en C₂-C₅ ; alcényle en C₂-C₅ ; halogénalcényle en C₂-C₅ ou halogéno ;
R₂ est un hydrogène ou un groupe alkyle en C₁-C₅ ; halogénalkyle en C₁-C₃ ayant de 1 à 3 atomes d'halogène ; alcoxyalkyle en C₃-C₅ ; nitro, cyano, S(O)ₚ-(alkyle en C₁-C₄), amino, NHR₃, N(R₃)R₉, N=C(R₉)R₁₀ ou halogéno ;
R₃ est un hydrogène ou un groupe alkyle en C₁-C₃, benzyle, -CO-R₆ ou SR₇ ;
R₄ et R₈, indépendamment l'un de l'autre, sont chacun un hydrogène ou un groupe alkyle en C₁-C₅ ; halogénalkyle en C₁-C₅ ayant de 1 à 3 atomes d'halogène ; alcoxyalkyle en C₂-C₆ ou cycloalkyle en C₃-C₆ ;
R₅ est un halogène ou un groupe alkyle en C₁-C₃ ; alcoxy en C₁-C₃ ; alcoxyalkyle en C₁-C₆, halogénalkyle en C₁-C₃, alkylthio en C₁-C₃, halogénalcoxy en C₁-C₃, halogénalkylthio en C₁-C₃, cyano ou nitro ;
R₆ est un groupe alkyle en C₁-C₅, phényle, phényle substitué par des substituants halogéno et/ou alkyle en C₁-C₃ ;
R₇ est le radical phényle, un radical phényle substitué une ou deux fois par des substituants halogéno, nitro ou cyano, le radical benzyle, un radical benzyle une ou deux fois substitué sur le noyau par des substituants halogéno, nitro ou cyano, ou encore un groupe alkyle en C₁-C₅ ou un groupe alkyle en C₁-C₅ substitué par des substituants halogéno ou cyano ;
R₉ est un groupe alkyle en C₁-C₅ ;
R₁₀ est un hydrogène ou un groupe alkyle en C₁-C₃ ;
R₁₃ est un hydrogène ou un groupe alkyle en C₁-C₄ ;
m vaut 1, 2 ou 3 ;
n vaut 0, 1 ou 2 ; et
p vaut 0, 1 ou 2.

7. Procédé selon la revendication 6, dans lequel
R₁ est un groupe alkyle en C₁-C₅ ; CF₃ alcoxyalkyle en C₂-C₅ ; alcényle en C₂-C₄ ; monohalogénalcényle en C₂-C₄ ou halogéno ;
R₂ est un groupe alkyle en C₁-C₅ ou halogéno ;
R₃ est un hydrogène ou un groupe alkyle en C₁-C₃ ;
R₄ est un hydrogène ou un groupe alkyle en C₁-C₃ ou cyclopropyle ;
R₅ est un halogène ; un groupe alkyle en C₁-C₂, alcoxy en C₁-C₃ ;
R₈ est un hydrogène ;
n vaut 0, 1, 2 ou 3 ; et
m vaut 1.

8. Procédé selon la revendication 7, dans lequel ;
R₁ est un groupe alkyle en C₁-C₄ ; CF₃ ; alcoxyalkyle en C₂-C₄ alcényle en C₂-C₄ ; ou halogéno ;
R₂ est un groupe alkyle en C₁-C₄ ou halogéno ;
R₃ est un hydrogène ou un groupe alkyle en C₁-C₃ ;
R₄ est un hydrogène ou un groupe alkyle en C₁-C₃ ;
R₅ est un halogène ou le groupe méthyle ; éthyle ou méthoxy ;
R₈ est un hydrogène ;
n vaut 0, 1, 2 ou 3 ; et
m vaut 1.

9. Procédé selon la revendication 1, dans lequel on applique un composé de formule I" 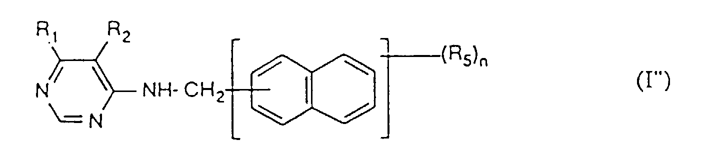 dans laquelle :
R₁ est un groupe alkyle en C₁-C₄, CF₃, alcoxyalkyle en C₂-C₄ ou halogéno ;
R₂ est un groupe alkyle en C₁-C₄ ou halogéno ;
R₅ est le groupe chloro, bromo, méthyle, éthyle, méthoxy, et
n vaut 0, 1 ou 2.

10. Procédé selon la revendication 1, dans lequel on applique un composé de formule I"' 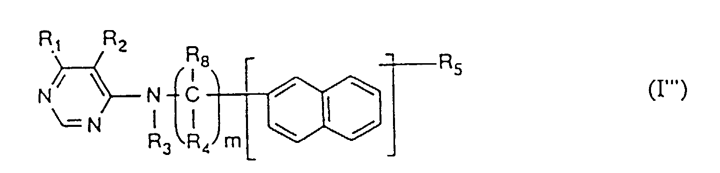 dans laquelle
R₁ est un hydrogène ou un groupe alkyle en C₁-C₃, CF₃ ou halogéno ;
R₂ est un hydrogène ou un groupe alkyle en C₁-C₃, halogéno, NO₂ ou S(O)ₚ-(alkyle en C₁-C₃) ;
R₃ est un hydrogène ;
R₄ est le radical méthyle, éthyle, isopropyle, n-propyle ou cyclopropyle ;
R₈ est un hydrogène ou le radical méthyle ou éthyle ;
R₅ est un hydrogène ou un groupe halogéno, alkyle en C₁-C₃, OCHF₂, CF₃, NO₂ ou alcoxy en C₁-C₃ ;
n vaut 1 ou 2 ; et
p vaut 0, 1 ou 2.

11. Procédé selon la revendication 3, dans lequel :
R₁ est un hydrogène ou un groupe alkyle en C₁-C₅ ; halogénalkyle en C₁-C₃ ayant de 1 à 3 atomes d'halogène ; alcoxyalkyle en C₂-C₅ ; alcényle en C₂-C₅ halogénalcényle en C₂-C₅ ayant de 1 à 3 atomes d'halogène, cycloalkyle en C₃-C₆ ou halogéno ;
R₂ est un hydrogène ou un groupe alkyle en C₁-C₅ ; halogénalkyle en C₁-C₃ ayant de 1 à 3 atomes d'halogène ; alcoxyalkyle en C₃-C₅ ; nitro, cyano, S(O)ₚ-(alkyle en C₁-C₄), amino, NHR₃, N(R₃)R₉, N=C(R₉)R₁₀ ou halogéno ;
R₃ est un hydrogène ou un groupe alkyle en C₁-C₅, benzyle, COR₆ ou SR₇ ;
R₄ et R₈, indépendamment l'un de l'autre, sont chacun un hydrogène ou un groupe alkyle en C₁-C₅ ; halogénalkyle en C₁-C₅ ayant de 1 à 3 atomes d'halogène ; alcoxyalkyle en C₂-C₆ ou cycloalkyle en C₃-C₆ ;
R₅ est un halogène ou un groupe alkyle en C₁-C₃ ; alcoxy en C₁-C₃ ; alcoxyalkyle en C₁-C₆, halogénalkyle en C₁-C₃, alkylthio en C₁-C₃, halogénalcoxy en C₁-C₃, halogénalkylthio en C₁-C₃, cyano ou nitro, ou un groupe X-phényle apparaissant sur le noyau naphtyle, qui est non substitué ou est une à trois fois substitué par des substituants identiques ou différents halogéno, alkyle en C₁-C₃ ou alcoxy en C₁-C₃ ;
R₆ est un groupe alkyle en C₁-C₅, phényle, ou phényle substitué par des substituants halogéno et/ou alkyle en C₁-C₃ ;
R₇ est le radical phényle, un radical phényle une ou deux fois substitué par des substituants identiques ou différents halogéno, nitro ou cyano, le radical benzyle, un radical benzyle une ou deux substitué sur le noyau par des substituants identiques ou différents halogéno, nitro ou cyano, ou encore un groupe alkyle en C₁-C₅ ou alkyle en C₁-C₅ substitué par des substituants halogéno ou cyano ;
R₉ est un groupe alkyle en C₁-C₅ ;
R₁₀ est un hydrogène ou un groupe alkyle en C₁-C₅ ;
R₁₃ est un hydrogène ;
X est un oxygène ou un soufre,
m vaut 2 ou 3,
n vaut 0, 1, 2 ou 3, et
p vaut 0, 1 ou 2.

12. Procédé selon la revendication 11, dans lequel
R₁ est un hydrogène ou un groupe alkyle en C₁-C₃ ; halogenalkyle en C₁-C₃ ayant de 1 à 3 atomes d'halogène ; alcoxyalkyle en C₂-C₅ ; alcényle en C₂-C₅ ; cycloalkyle en C₃-C₆ ou halogéno ;
R₂ est un hydrogène ou un groupe alkyle en C₁-C₅ ; halogénalkyle en C₁-C₃ ayant de 1 à 3 atomes d'halogène ; alcoxyalkyle en C₃-C₅ ; nitro, cyano, S(O)ₚ-(alkyle en C₁-C₃), amino, NHR₃, N(R₃)R₉, N=C(R₉)R₁₀ ou halogéno ;
R₃ est un hydrogène ou un groupe alkyle en C₁-C₃ ;
R₄ et R₈, indépendamment l'un de l'autre, sont chacun un hydrogène ou un groupe alkyle en C₁C₅ ; CF₃ ; alcoxyalkyle en C₂-C₆ ou cycloalkyle en C₃-C₆ ;
R₅ est un halogène ou un groupe alkyle en C₁-C₃ ; alcoxy en C₁-C₃ ; alcoxyalkyle en C₂-C₆, halogénalkyle en C₁-C₃, alkylthio en C₁-C₃, halogénalcoxy en C₁-C₃, halogénalkylthio en C₁-C₃, cyano ou nitro ;
R₆ est un groupe alkyle en C₁-C₅, phényle, phényle substitué par des substituants halogéno et/ou alkyle en C₁-C₃ ;
R₇ est le radical phényle, phényle une ou deux fois substitué par des substituants identiques ou différents halogéno, nitro ou cyano, benzyle, benzyle une ou deux fois substitué sur le noyau par des substituants identiques ou différents halogéno, nitro ou cyano, ou encore un groupe alkyle en C₁-C₅ ou alkyle en C₁-C₅ substitué par des substituants halogéno ou cyano ;
R₉ est un groupe alkyle en C₁-C₅ ;
R₁₀ est un hydrogène ou un groupe alkyle en C₁-C₅ ;
R₁₃ est un hydrogène,
X est un oxygène ou un soufre,
m vaut 2 ou 3,
n vaut 0, 1, 2 ou 3 ; et
p vaut 0, 1 ou 2.

13. Procédé selon la revendication 11, dans lequel
R₁ est un hydrogène ou un groupe alkyle en C₁-C₃ ; CF₃, alcoxyalkyle en C₂-C₅ alcényle en C₂-C₅ cycloalkyle en C₃-C₆ ou halogéno ;
R₂ est un hydrogène ou un groupe alkyle en C₁-C₅ ; CF₃, alcoxyalkyle en C₃-C₅ ; nitro, cyano, S(O)ₚ-(alkyle en C₁-C₄ ), amino, N(R₃)R₉, N=C(R₉)R₁₀ ou halogéno ;
R3 est un hydrogène ;
R₄ et R₈, indépendamment l'un de l'autre, sont chacun un hydrogène ou un groupe alkyle en C₁-C₅ ; alcoxyalkyle en C₂-C₆ ou cycloalkyle en C₃-C₆ ;
R₅ est un halogène ou un groupe alkyle en C₁-C₃ ; alcoxy en C₁-C₃ ; alcoxyalkyle en C₂-C₆, halogénalkyle en C₁-C₃, alkylthio en C₁-C₃, halogénalcoxy en C₁-C₃, halogénalkylthio en C₁-C₃, nitro ou cyano,
R₆ est un groupe alkyle en C₁-C₅, phényle ou phényle substitué par des substituants halogéno et/ou alkyle en C₁-C₃ ;
R₇ est le radical phényle, phényle une ou deux fois substitué par des substituants identiques ou différents halogéno, nitro ou cyano, benzyle, benzyle une ou deux fois substitué sur le noyau par des substituants identiques ou différents halogéno, nitro ou cyano, ou bien alkyle en C₁-C₅, ou alkyle en C₁-C₅ substitué par des substituants halogéno ou cyano ;
R₉ est un groupe alkyle en C₁-C₃ ;
R₁₀ est un hydrogène ou un groupe alkyle en C₁-C₃ ;
R₁₃ est un hydrogène ;
X est un oxygène ou un soufre,
m vaut 2 ;
n vaut 0, 1, 2 ou 3 ; et
p vaut 0, 1 ou 2.

14. Procédé selon la revendication 13, dans lequel
R₁ est un hydrogène ou un groupe alkyle en C₁-C₃ ; CF₃ ; alcoxyalkyle en C₂-C₅ ; alcényle en C₂-C₅ ; cycloalkyle en C₃-C₆ ou halogéno ;
R₂ est un hydrogène ou un groupe alkyle en C₁-C₅ ; CF₃, alcoxyalkyle en C₃-C₅ ; nitro, cyano, S(O)ₚ-(alkyle en C₁-C₃), amino, N(R₃)R₉, N=C(R₉)R₁₀ ou halogéno ;
R₃ est un hydrogène ;
R₄ et R₈, indépendamment l'un de l'autre, sont chacun un hydrogène ou un groupe alkyle en C₁-C₅ ; alcoxyalkyle en C₂-C₆ ou cycloalkyle en C₃-C₆ ;
R₅ est un halogène ou un groupe alkyle en C₁-C₃ ; alcoxy en C₁-C₃ alcoxyalkyle en C₂-C₆, halogénalkyle en C₁-C₃, alkylthio en C₁-C₃, halogénalcoxy en C₁-C₃, halogénalkylthio en C₁-C₃, nitro ou cyano ;
R₆ est un groupe alkyle en C₁-C₅, phényle ou phényle substitué par des substituants halogéno et/ou alkyle en C₁-C₃ ;
R₇ est le radical phényle, phényle une ou deux fois substitué par des substituants identiques ou différents halogéno, nitro ou cyano, benzyle, benzyle une ou deux fois substitué sur le noyau par des substituants identiques ou différents halogéno, nitro ou cyano, ou encore alkyle en C₁-C₅ ou alkyle en C₁-C₅ substitué par des substituants halogéno ou cyano ;
R₉ est un groupe alkyle en C₁-C₅ ;
R₁₀ est un hydrogène ou un groupe alkyle en C₁-C₃ ;
R₁₃ est un hydrogène ;
X est un oxygène ou un soufre,
m vaut 3,
n vaut 0, 1, 2 ou 3 ; et
p vaut 0, 1 ou 2.

15. Composé selon la revendication 1, dans lequel on applique un composé de formule Ia 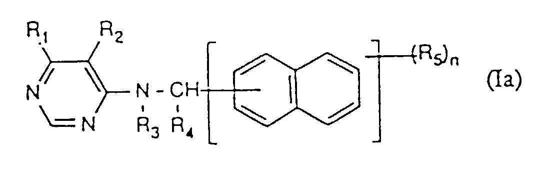 dans laquelle
R₁ est un groupe alkyle en C₁-C₅, qui est non substitué ou est substitué par des substituants halogéno ou alcoxy en C₁-C₃ ; alcényle en C₂-C₇, qui est non substitué ou est substitué par des halogènes ; cycloalkyle en C₃-C₇ ; ou halogéno ;
R₂ est un groupe alkyle en C₁-C₅, qui est non substitué ou est substitué par des substituants halogéno ou alcoxy en C₁-C₃ ; halogéno ; nitro ou cyano ;
R₃ est un hydrogène ou un groupe alkyle en C₁-C₅ ; benzyle ; CO-R₆ ou -S-R₇ ;
R₄ est un hydrogène ou un groupe alkyle en C₁-C₅, qui est non substitué ou est substitué par des substituants halogéno ou alcoxy en C₁-C₃ ; ou encore cycloalkyle en C₃-C₇ ;
R₅ est un halogène ou un groupe alkyle en C₁-C₃ ; alcoxy en C₁-C₃ ; alkylthio en C₁-C₃ ; ou un groupe -X-phényle apparaissant une fois sur le noyau naphtyle, qui est non substitué ou est une à trois fois substitué par des substituants identiques ou différents halogéno, alkyle en C₁-C₃ ou alcoxy en C₁-C₃ ;
R₆ est un groupe alkyle en C₁-C₅ phényle, qui est non substitué ou est substitué par des substituants halogéno et/ou alkyle en C₁-C₃ ;
R₇ est le radical phényle ou benzyle, qui est non substitué ou qui est une à deux fois substitué sur le noyau par des substituants identiques ou différents halogéno, nitro, cyano ; ou encore alkyle en C₁-C₅, qui est non substitué ou est substitué par des substituants halogéno ou cyano ;
X est un oxygène ou un soufre ; et
n vaut 0, 1, 2 ou 3.

16. Procédé selon la revendication 1, dans lequel on applique un composé de formule 1 appartenant au groupe suivant :
(d,l)-4-[1'-(β-naphtyl)éthylamino]-5-chloro-6-éthylpyrimidine (Composé 1.2) ;
(-)-4-[1'-(β-naphtyl)éthylamino]-5-chloro-6-éthylpyrimidine (Composé 1.73) ;
(d,l)-4-[1'-(β-naphtyl)propylamino]-5-chloro-6-éthylpyrimidine (Composé 1.3) ;
(d,l)-4-[1'-(β-naphtyl)éthylamino]-5-chloro-6-méthylpyrimidine (Composé 1.53) ;
(d,l)-4-[1'-(2-(6-bromonaphtyl)éthylamino]-5-chloro-6-éthylpyrimidine (Composé 1.149) ;
(d,l)-4-[1'-(β-naphtyl)éthylamino]-5-bromo-6-éthylpyrimidine (Composé 1.84) ;
(d,l)-4-[1'-(β-naphtyl)éthylamino]-5-chloro-6-n-propylpyrimidine (Composé 1.86) ;
(d,l)-4-[1'-(β-naphtyl)éthylamino]-5-iodo-6-éthylpyrimidine (Composé 1.100).

17. Procédé de préparation des composé de formule I selon la revendication 1, caractérisé en ce qu'on fait réagir un composé de formule II 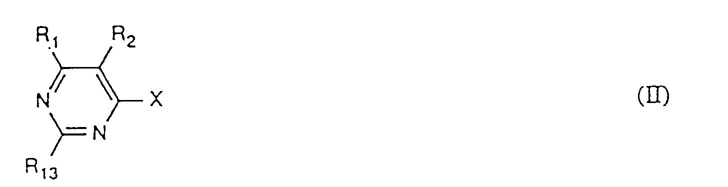 dans laquelle R₁, R₂ et R₁₃ ont les significations données pour la formule I et X est un résidu facilement éliminable, avec un composé de formule III 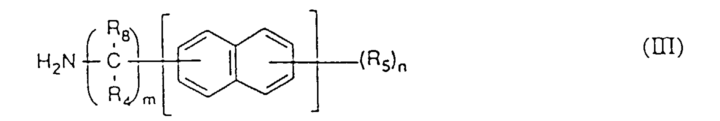 dans laquelle R₄, R₅, R₈, m et n ont les significations données pour la formule I, de préférence en présence d'une base, puis, si on le souhaite, on introduit le substituant R₃, dans la mesure où ce substituant est un groupe alkyle, benzyle, acyle ou -S-R₇, par des opérations correspondantes de N-alkylation, de N-benzylation, de N-acylation ou de N-thioalkylation ou thioarylation.
